(19) Europäisches Patentamt
European Patent Office
Office européen des brevets

(11) EP 2 488 020 B1

# (12) EUROPEAN PATENT SPECIFICATION

(45) Date of publication and mention of the grant of the patent:
**14.12.2016 Bulletin 2016/50**

(21) Application number: **10823855.1**

(22) Date of filing: **07.10.2010**

(51) Int Cl.:
*A01N 31/00* (2006.01) *A61K 31/05* (2006.01)
*A61K 31/045* (2006.01) *A61K 31/00* (2006.01)
*A61K 45/06* (2006.01) *C07C 59/66* (2006.01)
*C07C 59/68* (2006.01) *C07D 257/04* (2006.01)

(86) International application number:
**PCT/US2010/051781**

(87) International publication number:
**WO 2011/046800 (21.04.2011 Gazette 2011/16)**

## (54) 3-SUBSTITUTED COMPOUNDS FOR REDUCING URIC ACID

3-SUBSTITUIERTE VERBINDUNGEN ZUR SENKUNG VON HARNSÄURE

COMPOSÉS 3-SUBSTITUÉS POUR RÉDUIRE L'ACIDE URIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2009 US 251098 P**

(43) Date of publication of application:
**22.08.2012 Bulletin 2012/34**

(73) Proprietor: **Wellstat Therapeutics Corporation**
**Gaithersburg, MD 20878 (US)**

(72) Inventors:
- **SHARMA, Shalini**
  **Gaithersburg**
  **MD 20877 (US)**
- **O'NEIL, James, Dennen**
  **Frederick**
  **MD 21701 (US)**
- **VON BORSTEL, Reid, W.**
  **Potomac**
  **MD 20854 (US)**
- **ARUDCHANDRAN, Ramchandran**
  **Germantown**
  **MD 20876 (US)**

(74) Representative: **Titmus, Craig Edward**
**Mathys & Squire LLP**
**The Shard**
**32 London Bridge Street**
**London SE1 9SG (GB)**

(56) References cited:
**WO-A1-2009/151695**
**WO-A2-2009/134995**
**US-A- 4 024 253**
**US-A1- 2004 019 208**
**US-A1- 2008 015 209**
**US-A1- 2008 015 254**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

BACKGROUND OF THE INVENTION

Diseases caused by elevated levels of uric acid fall into two major categories: disorders caused by precipitation of uric acid crystals and diseases related to pathological effects of soluble uric acid. Gouty arthritis is the classic example of the former. Deposition of urate crystals in the kidney is also a common cause of renal dysfunction. Elevated levels of soluble uric acid are associated with a variety of disorders, including cardiovascular and renal diseases.

There is a significant medical need for new medications that can safely, conveniently and effectively treat and prevent disorders related to elevation of blood uric acid, whether such diseases are due to crystallization of uric acid or to effects of supranormal (whether by an individual or a population-based standard) levels of soluble uric acid.

Certain benzyloxyphenyl compounds and their uric acid-lowering activity are disclosed in International Patent Application Nos. PCT/US2009/37128 and PCT/US2009/42298, both assigned to Wellstat Therapeutics Corporation. They do not specifically disclose substitution at the 3-position of the benzyloxy moiety. PCT/US2009/37128 describes [2-(3-(2, 6-Dimethylbenzyloxy)-4-methylphenyl acetic acid], and PCT/US2009/42298 describes [5-(3-(2, 6-Dimethylbenzyloxy)phenyl)-1H-tetrazole].

SUMMARY OF THE INVENTION

The present invention is defined according to the claims.

This invention provides a compound represented by Formula I

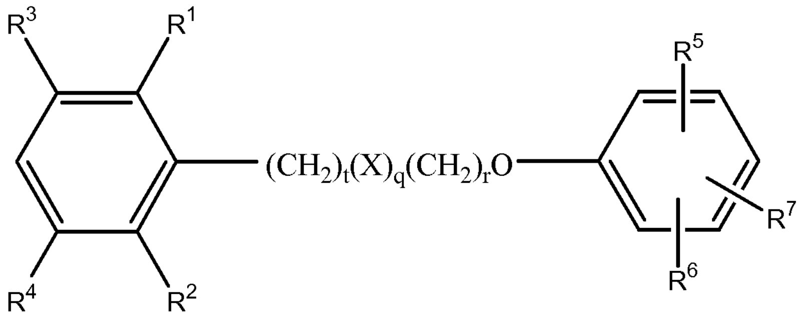

I

wherein $R^1$ is methyl; $R^2$ is methyl; one of $R^3$ and $R^4$ is hydrogen, and the other is selected from the group consisting of hydroxy, alkoxy having 1 or 2 carbon atoms, and nitro; t is 0; q is 0; r is 1; $R^5$ is hydrogen or methyl; $R^6$ is hydrogen; $R^7$ is:

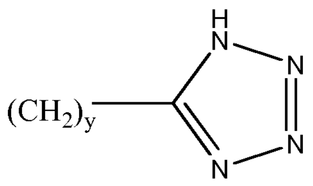

or

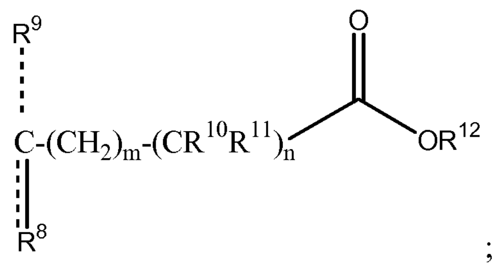

;

y is 0, 1, 2, or 3; m is 0, 1, 2, 3, or 4; n is 0; $R^8$ is hydrogen and $R^9$ is hydrogen; $R^{12}$ is hydrogen or alkyl having from 1 to 3 carbon atoms, or when $R^{12}$ is hydrogen, a pharmaceutically acceptable salt of the compound.

The disclosure provides a compound represented by Formula I, wherein $R^1$ and $R^2$ are independently selected from the group consisting of hydrogen, halo, alkyl having 1 or 2 carbon atoms, perfluoromethyl, alkoxy having 1 or 2 carbon atoms, perfluoromethoxy, hydroxy, and amino. One of $R^3$ and $R^4$ is hydrogen, and the other is selected from the group consisting of hydroxy, alkoxy having 1 or 2 carbon atoms, nitro, alkyl having 1 or 2 carbon atoms, amino, halo, and cyano. t is 0 or 1; q is 0 or 1; and r is 0, 1, or 2. X is C(O) and r is 0 and t is 0, or X is $N(R^{13})$ wherein $R^{13}$ is hydrogen or alkyl having from 1 to 3 carbon atoms. One of $R^5$ and $R^6$ is hydrogen, and the other is selected from the group consisting of hydrogen, alkyl having 1 or 2 carbon atoms, hydroxy, alkoxy having 1 or 2 carbon atoms, fluoro, chloro, bromo, amino.

$R^7$ is

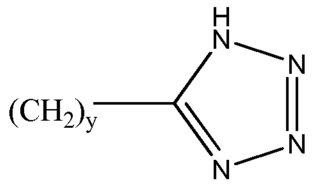

or

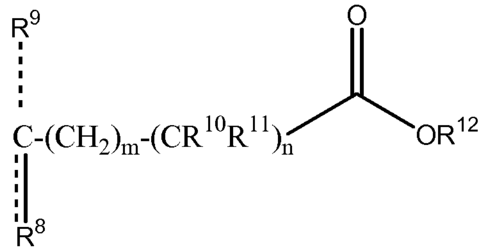

y is 0, 1, 2, or 3; m is 0, 1, 2, 3, or 4; n is 0 or 1. $R^8$ is hydrogen, methyl or ethyl and $R^9$ is hydrogen or methyl, or $R^8$ is hydroxy and $R^9$ is hydrogen, or $R^8$ is O and $R^9$ is absent, or $R^8$ and $R^9$ together are $CH_2CH_2$. One of $R^{10}$ and $R^{11}$ is alkyl having from 1 to 3 carbon atoms, and the other is hydrogen or alkyl having from 1 to 3 carbon atoms. $R^{12}$ is hydrogen or alkyl having from 1 to 3 carbon atoms. Alternatively, when $R^{12}$ is hydrogen, this invention also provides a pharmaceutically acceptable salt of the compound. The invention also provides a pharmaceutical composition comprising a compound or salt of this invention and a pharmaceutically acceptable carrier.

This disclosure provides a method of reducing the uric acid concentration in blood of, or increasing uric acid excretion from, a mammalian subject, comprising administering to the subject a compound or salt of this invention in an amount effective to reduce the uric acid concentration in blood of, or increase uric acid excretion from, the subject. This invention provides a compound or salt of this invention or a pharmaceutical composition of this invention for use in reducing the uric acid concentration in blood of, or increasing uric acid excretion from, a mammalian subject. This invention provides the use of a compound or salt of this invention or a pharmaceutical composition of this invention in the manufacture of a medicament for reducing the uric acid concentration in blood of, or increasing uric acid excretion from, a mammalian subject. This invention provides a pharmaceutical composition for use in reducing the uric acid concentration in blood of, or increasing uric acid excretion from, a mammalian subject comprising a compound or salt of this invention in an amount effective to reduce the uric acid concentration in blood of, or increase uric acid excretion from, the subject. This disclosure provides a kit comprising one or more unit oral doses of a compound or salt of this invention, and instructions for administering the compound or salt to reduce the uric acid concentration in blood of, or increasing uric acid excretion from, a mammalian subject.

Reducing uric acid as described herein can be used to treat or prevent a variety of conditions including gout (any or all of: asymptomatic gout, acute gouty arthritis, intercritical gout, and chronic tophaceous gout), hyperuricemia, elevated levels of uric acid that do not meet the levels customarily justifying a diagnosis of hyperuricemia, renal dysfunction, kidney stones, cardiovascular disease, risk for developing cardiovascular disease and other consequences of hyperuricemia, cognitive impairment, early-onset essential hypertension, and *Plasmodium falciparum*-induced inflammation.

This invention is based on the observation that compounds of this invention inhibited URAT1 *in vitro,* as shown in Example 6. Inhibition of URAT1 is an established *in vitro* model for lowering uric acid *in vivo.*

DETAILED DESCRIPTION OF THE INVENTION

DEFINITIONS

A 1*H*-tetrazolyl-5-yl moiety and the corresponding 2*H*-tetrazolyl-5-yl moiety can exist as tautomers. In this document compounds are named and structural formulas are written with reference to the 1*H*-tautomer. All such references are to be understood as including both tautomeric forms. Thus, for example, "3-((3-(1*H*-tetrazol-5-yl)phenoxy)methyl)-2,4-dimethylphenol" includes both 3-((3-(1*H*-tetrazol-5-yl)phenoxy)methyl)-2,4-dimethylphenol and 3-((3-(2*H*-tetrazol-5-yl)phenoxy)methyl)-2,4-dimethylphenol.

As used herein the term "alkyl" means a linear or branched-chain alkyl group. An alkyl group identified as having a certain number of carbon atoms means any alkyl group having the specified number of carbons. For example, an alkyl having three carbon atoms can be propyl or isopropyl; and an alkyl having four carbon atoms can be n-butyl, 1-methylpropyl, 2-methylpropyl or t-butyl.

As used herein the term "halo" refers to one or more of fluoro, chloro, bromo and iodo.

As used herein the term "perfluoro" as in perfluoromethyl or perfluoromethoxy, means that the group in question has fluorine atoms in place of all of the hydrogen atoms.

Certain chemical Compounds are referred to herein by their chemical name or by the two-letter code shown

below. Compounds EJ, EK, EM, and EN are included within the scope of Formula I shown above.

**EJ** 3-((3-(1*H*-tetrazol-5-yl)phenoxy)methyl)-2,4-dimethylphenol
**EK** 2-(3-(3-hydroxy-2,6-dimethylbenzyloxy)-4-methylphenyl)acetic acid
**EL** 3-((5-(carboxymethyl)-2-methylphenoxy)methyl)-2,4-dimethylphenyl sulfate
**EM** 2-(3-(3-methoxy-2,6-dimethylbenzyloxy)-4-methylphenyl)acetic acid
**EN** 2-(3-(2,6-dimethyl-3-nitrobenzyloxy)phenyl)acetic acid
**EB** 5-(3-(2,6-Dimethylbenzyloxy)phenyl)-1*H*-tetrazole
**EH** 2-(3-(2,6-Dimethylbenzyloxy)-4-methylphenyl)acetic acid

As used herein the transitional term "comprising" is open-ended. A claim utilizing this term can contain elements in addition to those recited in such claim.
As used in the claims the word "or" means "and/or" unless such reading does not make sense in context. So for example the phrase "reducing the uric acid concentration in blood of or increasing uric acid excretion from, a mammalian subject" is equivalent to "reducing the uric acid concentration in blood of and/or increasing uric acid excretion from, a mammalian subject.

COMPOUNDS OF THE INVENTION

When $R^8$ is hydroxyl and $R^9$ is hydrogen, the carbon to which both are bonded is a chiral center. This invention provides the racemate, the (R) enantiomer, and the (S) enantiomer, of the compounds of Formula I, all of which are active. Mixtures of these enantiomers can be separated by using HPLC, for example as described in Chirality 11:420-425 (1999).
In embodiments of the compound, method, use or pharmaceutical composition described in the Summary above, the compound is represented by Formula IA or IB.

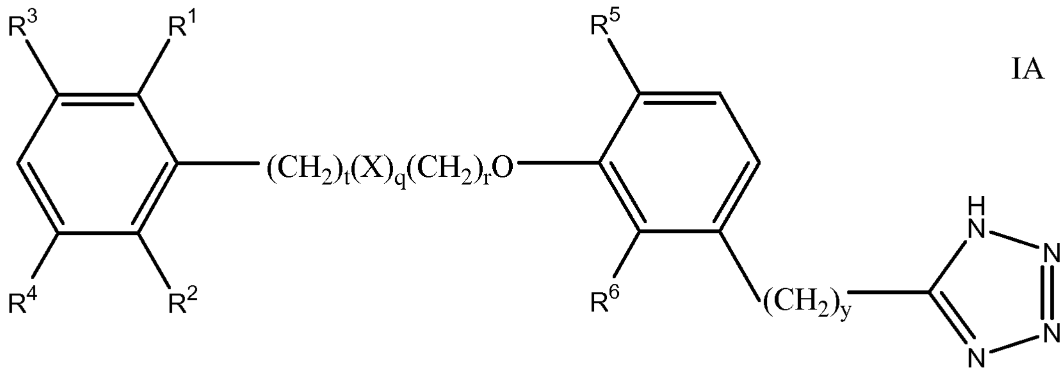

IA

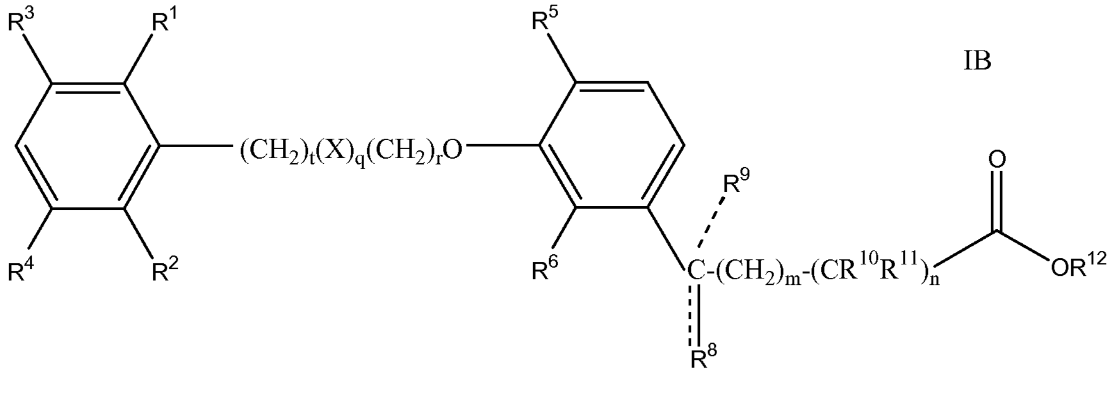

IB

According to the invention, $R^1$ is methyl, $R^2$ is methyl, t is 0, q is 0, r is 1, $R^5$ is hydrogen or methyl, $R^6$ is hydrogen. In further embodiments of each of Formulas I and IA, y is 0 or 1. In further embodiments of each of Formulas I and IB, one or more of the variables have the following values: $R^8$ is hydrogen, $R^9$ is hydrogen, m is 1, n is 0.
In specific embodiments of this invention the compound is selected from the group consisting of:

3-((3-(1*H*--tetrazol-5-yl)phenoxy)methyl)-2,4-dimethylphenol;

2-(3-(3-hydroxy-2,6-dimethylbenzyloxy)-4-methylphenyl)acetic acid;
2-(3-(3-methoxy-2,6-dimethylbenzyloxy)-4-methylphenyl)acetic acid; and
2-(3-(2,6-dimethyl-3-nitrobenzyloxy)phenyl)acetic acid.

In an embodiment of the compound of this invention, the compound is in substantially (at least 98%) pure form.

REACTION SCHEMES

The compound of formula I where m is 0, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydrogen, methyl, or ethyl and $R^9$ is hydrogen or methyl or $R^8$ and $R^9$ together are $-CH_2CH_2-$. One of $R^{10}$ and $R^{11}$ is alkyl having 1 to 3 carbon atoms, and the other is hydrogen or alkyl having from 1 to 3 carbon atoms, X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

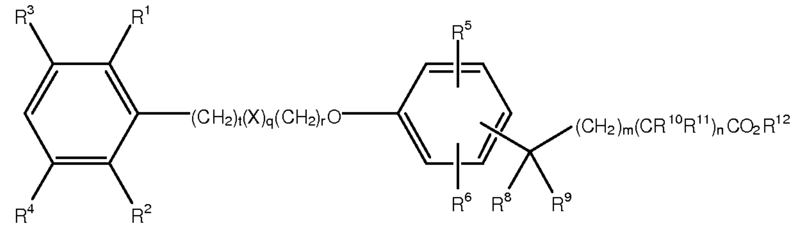

(I)

wherein $R^1$, $R^2$, $R^3$ and $R^4$ are described as above, can be prepared via reaction scheme of Scheme 1.

In the reaction scheme of Scheme 1,

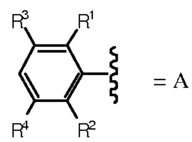

and q, t, m, n, r, $R^5$, $R^6$, $R^8$ and $R^9$ are as above. $R^{16}$ is alkyl group having from 1 to 2 carbon atoms. $R^{15}$ is alkyl group having from 1 to 3 carbon atoms or benzyl. $R^{14}$ is chloro or bromo and Y is halide or leaving group.

The compound of formula II can be alkylated with the compound of formula III or with the compound of formula (IV) via reaction of step (1-1). The reaction can be carried out in a suitable solvent, such as tetrahydrofuran, tetrahydrofuran/ 1,3-dimethyl-3,4,5,6-tetrahydro-2 (1H)-pyrimidinone, toluene, dimethylformamide, tetrahydrofuran/hexamethylphosphoramide and the like. Generally, the reaction is carried out in the presence of 2 to 3 molar equivalents of base to produce the compound of formula V where $R^8$ is alkyl having 1 to 2 carbon atoms and $R^9$ is hydrogen or 4 to 6 molar equivalents of base to produce the compounds of formula V where $R^8$ and $R^9$ are alkyl having from 1 to 2 carbon atoms or together are $-CH_2CH_2-$. The conventional base for this purpose can be sodium hydride, potassium hydride, sodium hydroxide and tetrabutylammonium hydroxide, potassium bis(trimethylsilyl)amide, lithium bis(trimethylsilyl)amide, lithium diisopropylamide and the like. In carrying out this reaction it is generally preferred to utilize aq. solution of tetrabutylammonium hydroxide and aq. sodium hydroxide. The reaction can be carried out at temperatures from -78°C to $25^0$C for 6 to 72 hours. The conventional techniques such as extraction, evaporation, chromatography and recrystallization can be utilized to purify the product.

The compound of formula V can be converted to the compound of formula VI via reaction step (1-2) by hydrolysis. In carrying out this reaction it is generally preferred to utilize, for example aqueous sodium hydroxide: ethanol. Any of the conditions conventionally used in hydrolysis of nitrile to produce carboxylic acid can be utilized to carry out the reaction of step (1-2).

The compound of formula VI can be converted to the compound of formula VII by esterification of the compound of formula VI with LV. The reaction can be carried out either by using catalyst for example $H_2SO_4$, TsOH and the like or by using dehydrating agent for example dicyclohexylcarbodiimide and the like. Any of the conditions conventional in such esterification reactions can be utilized to carry out the reaction of step (1-3).

The compound of formula VI can also be converted to the compound of formula VII by alkylation of compound

of formula VI with $R^{15}Y$ in the presence of base for example pyridine, triethylamine, potassium carbonate, cesium carbonate. The reaction can be carried out at temperatures from $25^0C$ -100°C for 6 to 72 hours in the solvent for example, dimethylformamide, dimethyl sulfoxide, dichloromethane, tetrahydrofuran.

In the case where X is C(O), the compound of formula VI can be reacted with the benzyl bromide in the presence of base for example, pyridine, triethylamine, potassium carbonate, cesium carbonate. The reaction can be carried out at temperatures from 25°C-100°C for 6 to 72 hours in the solvent for example, dimethylformamide, dimethyl sulfoxide, dichloromethane, tetrahydrofuran to produce the compound of formula VII. Any conditions conventional in such alkylation reactions can be utilized to carry out the reaction of step (1-3).

The compound of formula VII can be demethylation by utilizing lewis acid for example $BBr_3$ or $BCl_3$ in dichloromethane or chloroform at temperature from $-78^0C$ to 25°C. Any of the conditions conventional in such demethylation reactions can be utilized to carry out the reaction via the reaction of step (1-4).

In the second step, the product of reaction step (1-4) can be converted to the compound of formula XI via reaction of step (1-5) using Mitsunobu condensation with IX utilizing triphenylphosphine and diethyl azodicarboxylate or diisopropyl azodicarboxylate. The reaction is carried out in a suitable solvent for example tetrahydrofuran. Any of the conditions conventionally used in Mitsunobu reactions can be utilized to carry out the reaction of step (1-5).

In the case where X is C(O), the compound of formula VII can be reacted with the compound of formula IX in the presence of dehydrating agent for example dicyclohexylcarbodiimide, alkyl chloroformate and triethylamine, DCC and an aminopyridine, triethylamine and N,N'-carbonyldiimidazole and the like. Any conditions conventional in such esterification reactions can be utilized to carry out the reaction of step (1-5).

The compound of formula XI can also be prepared by etherifying or alkylating the hydroxyl from step (1-4) with the compound of formula X via reaction of step (1-5). In the compound of formula X, Y, include but are not limited to mesyloxy, tosyloxy, chloro, bromo, iodo, and the like. Any conventional method of etherifying of a hydroxyl group by reaction with a halide or leaving group can be utilized to carry out the reaction of step (1-5).

In the case where X is C(O), the compound of formula VII can be reacted with the compound of formula X where Y is chloro. Generally, the reaction is carried out in the presence of base for example pyridine, triethylamine and the like. Any conditions conventional in such reactions can be utilized to carry out the reaction of step (1-5).

The compound of formula XI is the compound of formula I where m is 0, n is 0 and $R^{12}$ is alkyl having from 1 to 3 carbon atoms.

The compound of formula XI can be converted to the compound of formula XII via reaction of step (1-6) where m is 0, n is 0 and $R^{12}$ is H by ester hydrolysis. Any conventional method of ester hydrolysis will produce the compound of formula XII where $R^{12}$ is H.

In the case where X is C(O), the benzyl group can be removed by catalytic hydrogenation to give the compound of formula XII where $R^{12}$ is H. Any conditions conventional for catalytic hydrogenation reactions can be utilized to produce the compound of formula XII.

The compound of formula XII is the compound of formula I where m is 0, n is 0 and $R^{12}$ is H.

The products in all the steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 1

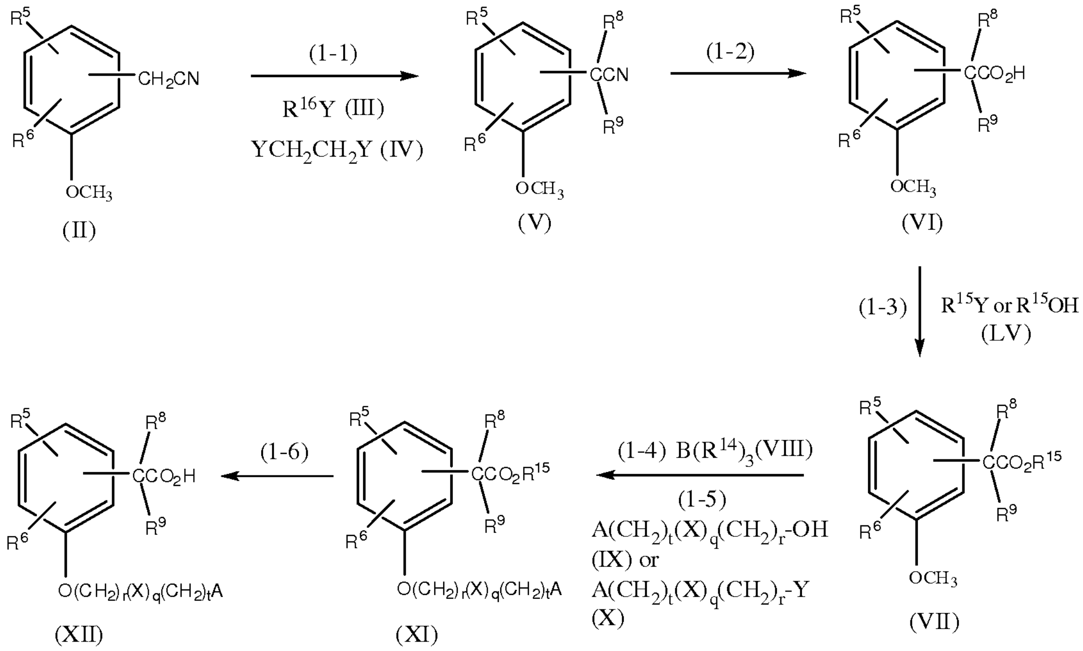


(II) (V) (VI) (VII) (XI) (XII)

The compound of formula I where m is 1 to 4, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydrogen, methyl, or ethyl and $R^9$ is hydrogen or methyl or $R^8$ and $R^9$ together are $-CH_2CH_2-$. One of $R^{10}$ and $R^{11}$ is alkyl having 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

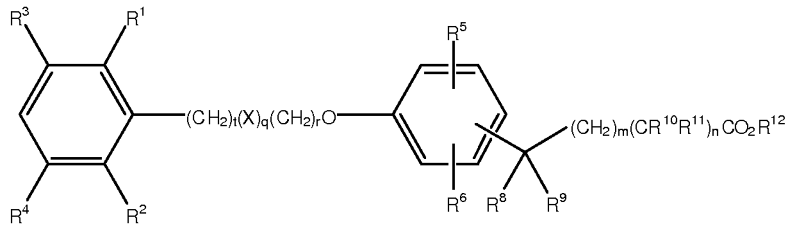

(I)

wherein $R^1$, $R^2$, $R^3$ and $R^4$ are described as above, can be prepared via reaction scheme of

Scheme 2.

In the reaction scheme of Scheme 2,

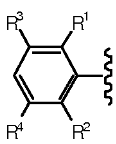

= A

and q, t, m, r, $R^5$, $R^6$, $R^8$, $R^9$ and $R^{12}$ are as above, and Y is a halide. $R^{15}$ is alkyl group having from 1 to 3 carbon atoms or benzyl.

The compound of formula VII can be reduced to the compound of formula XIII via reaction of step (2-1). The reaction is carried out utilizing a conventional reducing agent for example alkali metal hydride such as lithium aluminum

hydride, sodium borohydride, and diisobutyl aluminum hydride. The reaction is carried out in a suitable solvent, such as tetrahydrofuran, ether. Any of the conditions conventional in such reduction reactions can be utilized to carry out the reaction of step (2-1).

The compound of formula XIII can be converted to the compound of formula XIV by displacing hydroxyl group with a halogen group preferred halogen being bromo or chloro. Appropriate halogenating reagents include but are not limited to thionyl chloride, oxalyl chloride, bromine, phosphorous tribromide, carbon tetrabromide and the like. Any conditions conventional in such halogenation reactions can be utilized to carry out the reaction of step (2-2).

The compound of formula XIV can be converted to the compound of formula XV by reacting the compound of formula XIV with alkali metal cyanide for example sodium, potassium or copper cyanide. The reaction is carried out in a suitable solvent, such as ethanol, dimethyl sulfoxide, dimethylformamide and the like. Any of the conditions conventionally used in the preparation of nitriles can be utilized to carry out the reaction of step (2-3).

The compound of formula XV can be converted to the compound of formula XVI via reaction step (2-4) by base hydrolysis. In carrying out this reaction it is generally preferred to utilize, for example aqueous sodium hydroxide in ethanol, tetrahydrofuran: water and the like. Any of the conditions conventionally used in hydrolysis of nitrile can be utilized to carry out the reaction of step (2-4).

The compound of formula XVI can be converted to the compound of formula XVII via reaction of step (2-5) in the same manner as described hereinbefore in connection with the reaction of step (1-3).

The compound of formula XVII can be converted to the compound of formula XVIII via reaction of step (2-6) in the same manner as described hereinbefore in connection with the reaction of step (1-4), and reaction of step (1-5).

The compound of formula XVIII is the compound of formula I where m is 1, n is 0 and $R^{12}$ is alkyl group having 1 to 3 carbon atoms.

The compound of formula XVIII can be converted to the compound of formula I where m is 1, n is 0 and $R^{12}$ is H in the same manner as described hereinbefore in connection with the reaction of step (1-6).

The compound of formula XIV can be reacted with diethyl malonate utilizing a suitable base for example sodium hydride to give the compound of formula XIX. The reaction is carried out in suitable solvent, such as dimethylformamide, tetrahydrofuran and the like. Any of the conditions conventional in such alkylation reactions can be utilized to carry out the reaction of step (2-7).

The compound of formula XIX can be hydrolyzed and decarboxylated utilizing sodium hydroxide in suitable solvent, such as ethanol-water to give the compound of formula XX. Any of the conditions conventional in such reactions can be utilized to carry out the reaction of step (2-8).

The compound of formula XX can be converted to the compound of formula XXI via reaction of step (2-9) in the same manner as described hereinbefore in connection with the reaction of step (1-3).

The compound of formula XXI can be converted to the compound of formula XXII via reaction of step (2-10) in the same manner as described hereinbefore in connection with the reaction of step (1-4) and reaction of step (1-5).

The compound of formula XXII is the compound of formula I where m is 2, n is 0 and $R^{12}$ is alkyl group having 1 to 3 carbon atoms.

The compound of formula XXII can be converted to the compound of formula I where m is 2, n is 0 and $R^{12}$ is H in the same manner as described hereinbefore in connection with the reaction of step (1-6).

The compound of formula XX can be reduced to give the compound of formula XXIII via reaction of step (2-11). This reaction can be carried out in the same manner as described hereinbefore in the reaction of step (2-1).

The compound of formula XXIII can be converted to the compound of formula XXIV via reaction of step (2-12) in the same manner as described hereinbefore in connection with the reaction of step (2-2).

The compound of formula XXIV can be converted to the compound of formula XXV via reaction of step (2-13) in the same manner as described hereinbefore in connection with the reaction of step (2-3).

The compound of formula XXV can be converted to the compound of formula XXVI via reaction of step (2-14) in the same manner as described hereinbefore in connection with the reaction of step (2-4).

The compound of formula XXVI can be converted to the compound of formula XXVII via reaction of step (2-15) in the same manner as described hereinbefore in connection with the reaction of step (1-3).

The compound of formula XXVII can be converted to the compound of formula XXVIII via reaction of step (2-16) in the same manner as described hereinbefore in connection with the reaction of step (1-4) and reaction of step (1-5).

The compound of formula XXVIII is the compound of formula I where m is 3, n is 0 and $R^{12}$ is alkyl group having 1 to 3 carbon atoms.

The compound of formula XXVIII can be converted to the compound of formula I where m is 3, n is 0 and $R^{12}$ is H in the same manner as described hereinbefore in connection with the reaction of step (1-6).

The compound of formula XXIV can be converted to the compound of formula XXIX via reaction of step (2-17) in the same manner as described hereinbefore in connection with the reaction of step (2-7).

The compound of formula XXIX can be converted to the compound of formula XXX via reaction of step (2-18) in the same manner as described hereinbefore in connection with the reaction of step (2-8).

The compound of formula XXX can be converted to the compound of formula XXXI via reaction of step (2-19) in the same manner as described hereinbefore in connection with the reaction of step (1-3).

The compound of formula XXXI can be converted to the compound of formula XXXII via reaction of step (2-20) in the same manner as described hereinbefore in connection with the reaction of step (1-4) and reaction of step (1-5).

The compound of formula XXXII is the compound of formula I where m is 4, n is 0 and $R^{12}$ is alkyl group having 1 to 3 carbon atoms.

The compound of formula XXXII can be converted to the compound of formula I where m is 4, n is 0 and $R^{12}$ is H in the same manner as described hereinbefore in connection with the reaction of step (1-6).

The products in all the steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 2

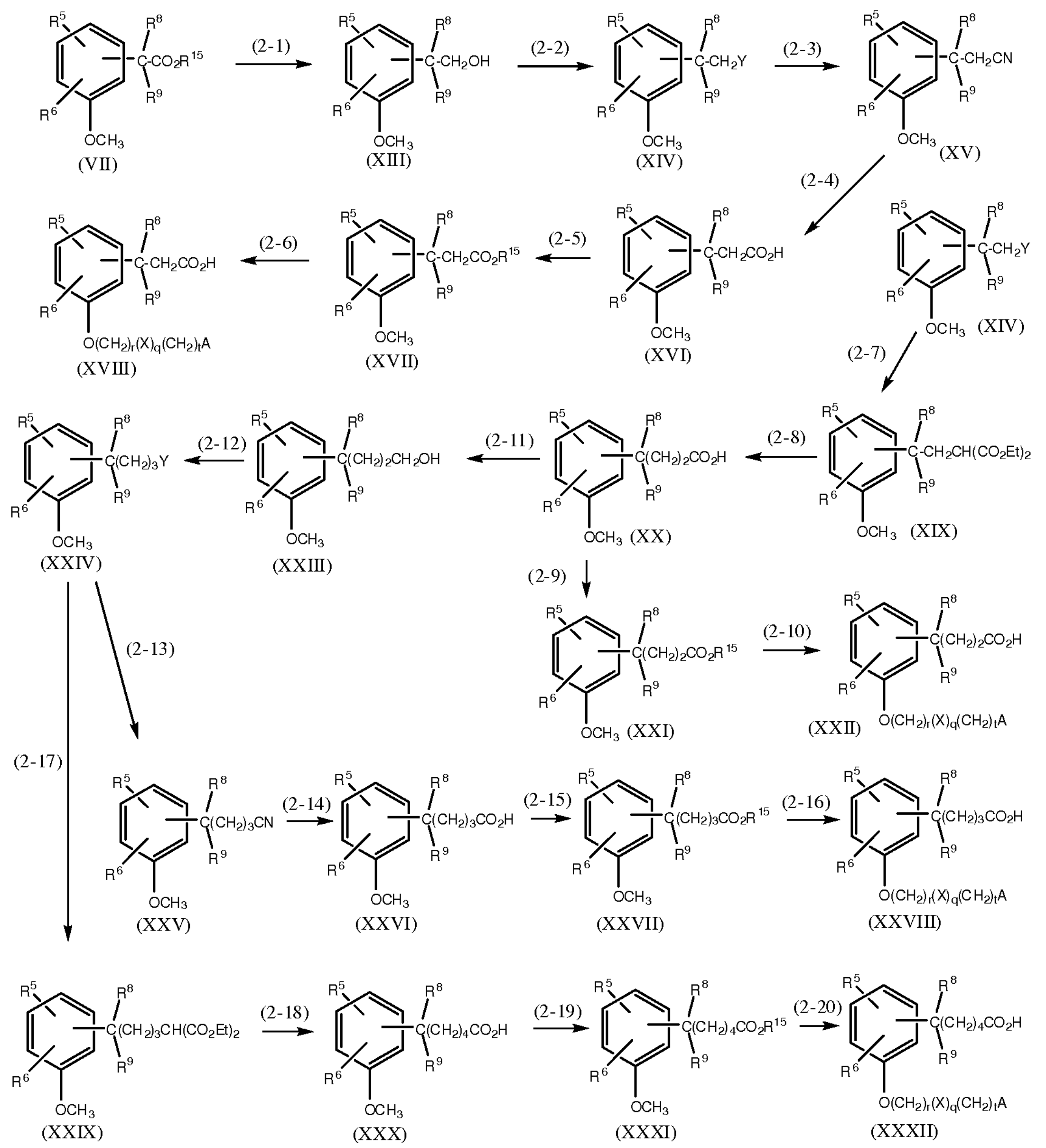

The compound of formula I where m is 1 to 4, q is 0 or 1, t is 0 or 1, and r is 0, 1 or 2, n is 1, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydrogen, methyl, or ethyl and $R^9$ is hydrogen or methyl or $R^8$ and $R^9$ together are $-CH_2CH_2-$. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

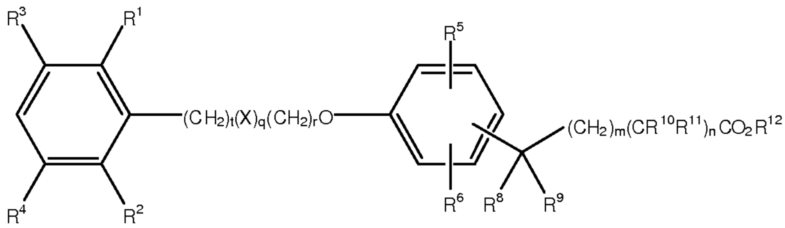

(I)

wherein $R^1$, $R^2$, $R^3$ and $R^4$ are described as above, can be prepared via reaction scheme of Scheme 3.

In the reaction scheme of Scheme 3,

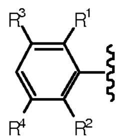
= A

and q, t, m, r, $R^5$, $R^6$, $R^8$, $R^9$ and $R^{12}$ are as above, p is 2 to 5, s is 1 to 4 and Y is halide. $R^{17}$ is alkyl group having from 1 to 3 carbon atoms. $R^{15}$ is alkyl group having from 1 to 3 carbon atoms or benzyl group.

The compound of formula XXXIII can be converted to the compound of formula XXXV via reaction of step (3-1) using Wittig reaction by treating the compound of formula XXXIII with the compound of formula XXXIV. Any conventional method of reaction of an aldehyde with a triarylphosphine hydrohalide can be utilized to carry out the reaction of step (3-1). Any of the conditions conventional in Wittig reactions can be utilized to carry out the reaction of step (3-1).

The compound of formula XXXV can be converted to the compound of formula XXXVI by reduction of alkene via catalytic hydrogenation in the presence of transition metal catalyst for example, raney nickel, palladium-on-charcoal, platinum metal or its oxide, tris(triphenylphosphine)chlororhodium(I) (Wilkinson's catalyst) under hydrogen atmosphere. The reaction can be carried in a suitable solvent, such as toluene, ethyl acetate, ethanol and the like. Any of the conditions conventional in such catalytic hydrogenation can be utilized to carry out the reaction of step (3-2).

The compound of formula XXXVI can be alkylated with the compound of formula LVII to produce the compound of formula XXXVII via reaction of step (3-3). The reaction is carried out in a suitable solvent, such as tetrahydrofuran, tetrahydrofuran/ 1,3-dimethyl-3, 4,5,6-tetrahydro-2 (1H)-pyrimidinone, terahydrofuran/hexamethylphosphoramide and the like. Generally, the reaction is carried out in the presence of 2 to 3 molar equivalents of base to produce the compound of formula XXXVII where one of $R^{10}$ and $R^{11}$ is alkyl having 1 to 3 carbon atoms and other is hydrogen or 4 to 6 molar equivalents of base to produce the compound of formula XXXVII where $R^{10}$ and $R^{11}$ are alkyl having 1 to 3 carbon atoms. The conventional base can be potassium bis(trimethylsilyl)amide, lithium bis(trimethylsilyl)amide, lithium diisopropylamide and the like. Generally, the reaction is carried out at temperatures from $-78^0C$ to $25^0C$ for 6 to 72 hours. The conventional techniques such as extraction, evaporation, chromatography and recrystallization can be utilized to purify the product.

In the compound of formula XXXVII, m is 1 to 4 and n is 1.

The compound of formula XXXVII can be converted to the compound of formula XXXVIII by demethylation utilizing lewis acid for example $BBr_3$ or $BCl_3$ in dichloromethane or chloroform at temperature for example $-78^0C$ to 25°C. Any of the conditions conventional in such demethylation reactions can be utilized to carry out the reaction of step (3-4).

The compound of formula XXXVIII can be converted to the compound of formula XXXIX via reaction of step (3-5) in the same manner as described hereinbefore in connection with the reaction of step (1-5).

The compound of formula XXXIX is the compound of formula I where m is 1 to 4, n is 1 and $R^{12}$ is alkyl group having 1 to 3 carbon atoms.

The compound of formula XXXIX can be converted to the compound of formula XL where $R^{12}$ is H via reaction of step (3-6) in the same manner as described hereinbefore in connection with the reaction of step (1-6).

The compound of XL is the compound of formula I where m is 1 to 4, n is 1 and $R^{12}$ is H.

The conventional techniques such as extraction, evaporation, chromatography and recrystallization can be utilized to purify the product of the all steps.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected after the reaction of step (e') utilizing suitable deprotecting reagents such as those described in Protective Groups in Organic Synthesis by T. Greene.

Reaction Scheme 3

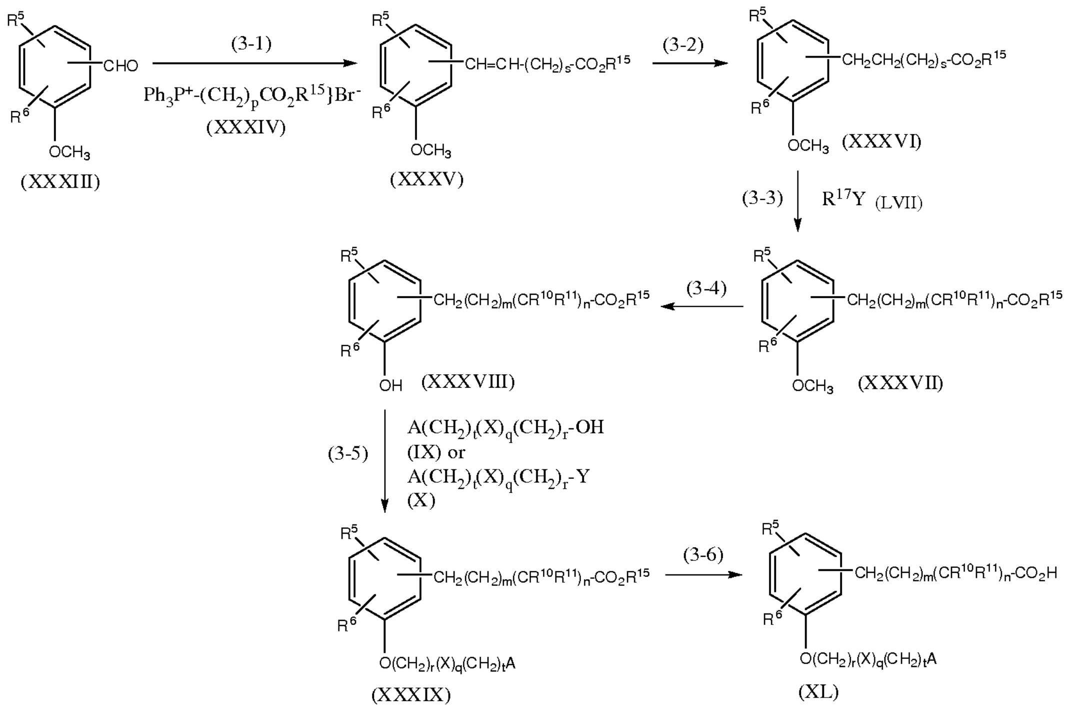


The compound of formula I where m is 0, q is 0 or 1, t is 0 or 1, and r is 0, 1 or 2, n is 1, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydrogen, methyl, or ethyl and $R^9$ is hydrogen or methyl or $R^8$ and $R^9$ together are $-CH_2CH_2-$. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

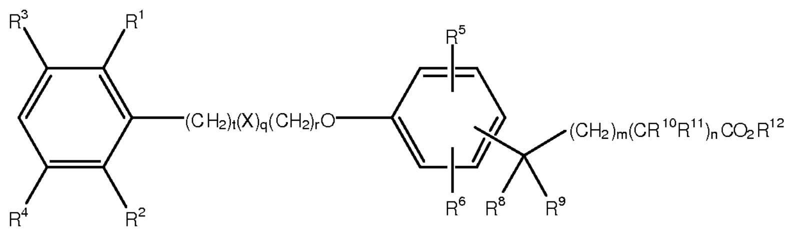

(I)

wherein $R^1$, $R^2$, $R^3$ and $R^4$ are described as above, can be prepared via reaction scheme of Scheme 4.

In the reaction scheme of Scheme 4,

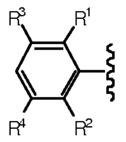
= A

and q, t, r, $R^5$, $R^6$, $R^8$, $R^9$ and $R^{12}$ are as above, and Y is halide. $R^{17}$ is alkyl group having from 1 to 3 carbon atoms. $R^{15}$ is alkyl group having from 1 to 3 carbon atoms or benzyl group.

The compound of formula XVIII can be converted to the compound of formula XLI via reaction of step (4-1) in the same manner as described hereinbefore in connection with the reaction of step (1-3).

The compound of formula XLI can be alkylated with the compound of formula LVII via reaction of step (4-2). The reaction is carried out in a suitable solvent, such as tetrahydrofuran, tetrahydrofuran/ 1,3-dimethyl-3,4,5,6-tetrahydro-2 (1H)-pyrimidinone, toluene, dimethylformamide, tetrahydrofuran/hexamethylphosphoramide and the like. Generally, the reaction is carried out in the presence of 2 to 3 molar equivalents of base to produce the compound of formula XLII where $R^{10}$ is alkyl having 1 to 2 carbon atoms and $R^{11}$ is hydrogen or 4 to 6 molar equivalents of base to produce the compounds of formula XLII where $R^{10}$ and $R^{11}$ are alkyl having 1 to 2 carbon atoms. The conventional base for this purpose can be sodium hydride, potassium hydride, sodium hydroxide and tetrabutylammonium hydroxide, potassium bis(trimethylsilyl)amide, lithium bis(trimethylsilyl)amide, lithium diisopropylamide and the like. In carrying out this reaction it is generally preferred to utilize aq. solution of tetrabutylammonium hydroxide and aq. sodium hydroxide. The reaction can be carried out at temperatures from -78$^0$C to 25$^0$C for 6 to 72 hours. Any conventional techniques for such alkylation can be utilized to produce compound of formula XLII.

The compound of formula XLII is the compound of formula I where m is 0, n is 1 and $R^{12}$ is alkyl group having from 1 to 3 carbon atoms.

The compound of formula XLII can be converted to the compound of formula XLIII via reaction of (4-3) in the same manner as described hereinbefore in connection with the reaction of step (1-6).

The compound of formula XLIII is the compound of formula I where m is 0, n is 1 and $R^{12}$ is H.

The conventional techniques such as extraction, evaporation, chromatography and recrystallization can be utilized to purify the product of all the steps.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 4

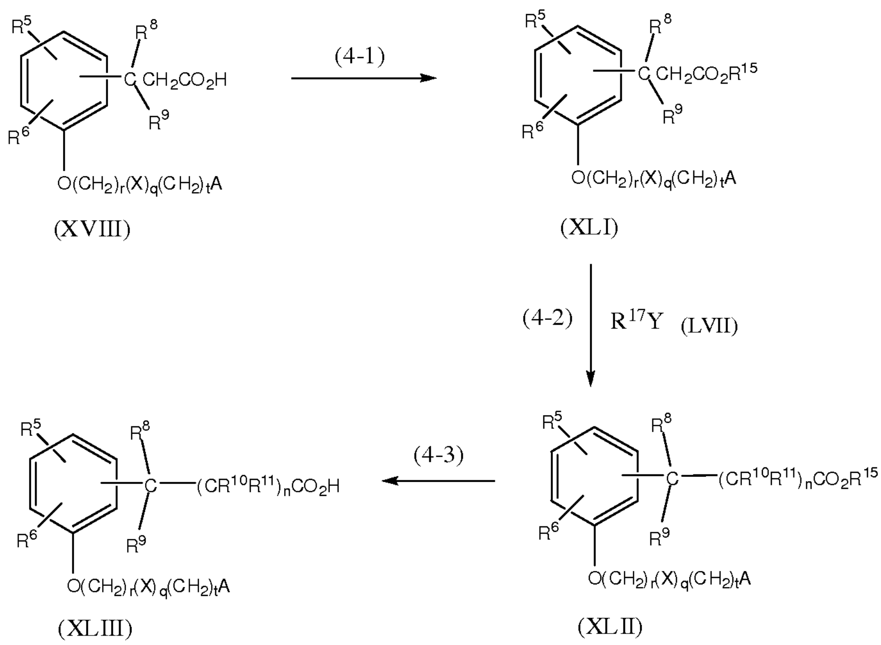

(XVIII) (XLI) (XLII) (XLIII)

The compound of formula I where m is 0, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is =O and $R^9$ is absent. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

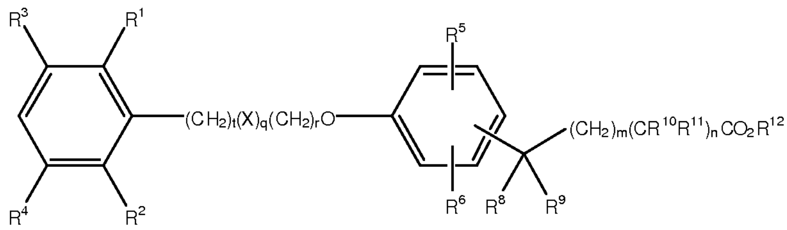

(I)

wherein $R^1$, $R^2$, $R^3$ and $R^4$ are described as above, can be prepared via reaction scheme of Scheme 5.

In the reaction scheme of Scheme 5,

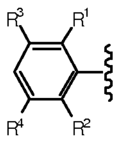

= A

and q, t, r, $R^5$, $R^6$, $R^8$, $R^9$ and $R^{12}$ are as above, and Y is halide or leaving group. $R^{17}$ is alkyl group having from 1 to 3 carbon atoms.

The compound of formula XLIV can be converted to the compound of formula XLV via reaction of step (5-1) using Mitsunobu condensation of XLIV with IX using triphenylphosphine and diethyl azodicarboxylate or diisopropyl azodicarboxylate. The reaction is carried out in a suitable solvent for example tetrahydrofuran. Any of the conditions conventionally used in Mitsunobu reactions can be utilized to carry out the reaction of step (5-1).

The compound of formula XLV can also be prepared by etherifying or alkylating the compound of formula XLIV with the compound of formula X via the reaction of step (5-2) by using suitable base such as potassium carbonate, cesium carbonate, sodium hydride, triethylamine, pyridine and the like. In the compound of formula X, Y, include but are not limited to mesyloxy, tosyloxy, chloro, bromo, iodo, and the like. Any conventional conditions to alkylate a hydroxyl group with a leaving group can be utilized to carry out the reaction of step (5-2). The reaction of step (5-2) is preferred over step (5-1) if compound of formula X is readily available.

The compound of formula XLV can be converted to the compound of formula XLVII via reaction of step (5-3) by oxidation of keto methyl group with selenium dioxide (XLV) in the presence of pyridine. Generally the reaction is carried out at temperatures of from 25°C-100°C from 2 to 24 hours. Any conventional conditions for such oxidations with can be utilized to carry out the reaction of step (5-3).

The compound of formula XLVII is the compound of formula I where m is 0, n is 0, $R^8$ is =O, $R^9$ is absent and $R^{12}$ is H.

The compound of formula XLVII can be converted to the compound of formula XLVIII by esterification of the compound of formula XLVII with CXLIII. The reaction can be carried out either by using catalyst for example $H_2SO_4$, TsOH and the like or by using dehydrating agent for example dicyclohexylcarbodiimide and the like. Any of the conditions conventional in such esterification reactions can be utilized to carry out the reaction of step (5-4).

The compound of formula XLVII can also be converted to the compound of formula XLVIII by alkylation of compound of formula XLVII with LVII in the presence of base for example pyridine, triethylamine, potassium carbonate, cesium carbonate, sodium hydride. The reaction can be carried out at temperatures from $25^0$C -100°C for 6 to 72 hours in the solvent for example, dimethylformamide, dimethyl sulfoxide, dichloromethane, tetrahydrofuran. Any of the conditions conventional in such esterification reactions can be utilized to carry out the reaction of step (5-4).

The compound of formula XLVIII is the compound of formula I where m is 0, n is 0 and $R^{12}$ is alkyl having 1 to 3 carbon atoms.

The product of the all steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable

protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 5

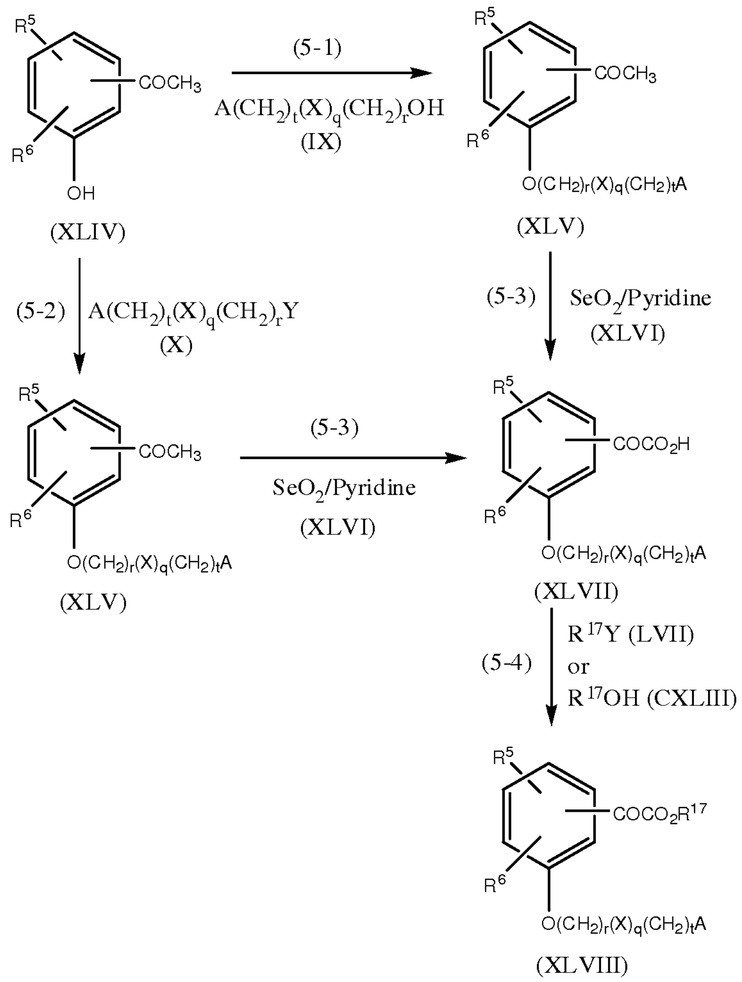


The compound of formula I where m is 1, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is =O and $R^9$ is absent. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

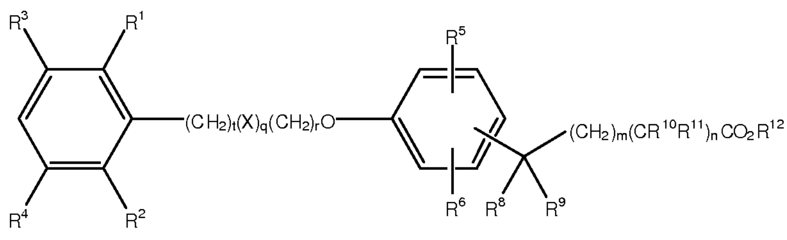

(I)

wherein $R^1$, $R^2$, $R^3$ and $R^4$ are described as above, can be prepared via reaction scheme of Scheme 6.

In the reaction scheme of Scheme 6,

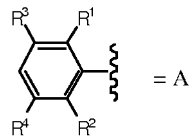

and q, t, r, $R^5$, $R^6$ are as above, $R^{17}$ is alkyl group having from 1 to 3 carbon atoms.

The compound of formula XLV (prepared in the same manner as described in the reaction of scheme 5) can be reacted with the compound of formula XLIX via reaction of step (6-1) in the presence of a suitable base such as sodium hydride and the like. The reaction can be carried out in conventional solvents such as dimethylformamide, tetrahydrofuran, and the like followed by addition of dialkyl carbonate such as dimethyl or diethyl or dipropyl carbonate to produce the corresponding compound of formula L. Any conditions conventional in such alkylation reactions can be utilized to carry out the reaction of step (6-1).

The compound of formula L is the compound of formula I where m is 1, n is 0, $R^8$ is =O, $R^9$ is absent and $R^{12}$ is alkyl having 1 to 3 carbon atoms.

The compound of formula L can be converted to the compound of formula LI via reaction step (6-2) in the same manner as described hereinbefore in connection with the reaction of step (1-6).

The compound of LI is the compound of formula I where m is 1, n is 0, $R^8$ is =O, $R^9$ is absent and $R^{12}$ is H.

The conventional techniques such as extraction, evaporation, chromatography and recrystallization can be utilized to purify the product of the all steps.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 6

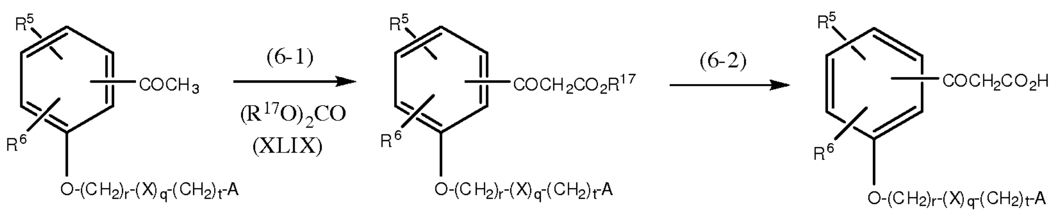


(XLV) (L) (LI)

The compound of formula I where m is 2 to 4, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is =O and $R^9$ is absent. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

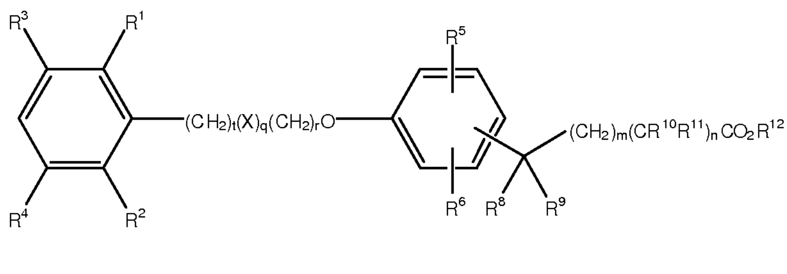

(I)

wherein $R^1$, $R^2$, $R^3$ and $R^4$ are described as above, can be prepared via reaction scheme of Scheme 7.

In the reaction scheme of Scheme 7,

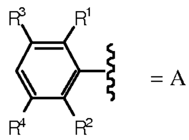

and q, t, m, r, $R^5$, and $R^6$ are as above. $R^{15}$ is alkyl group having 1 to 2 carbon atoms or benzyl group and p is 1 to 3.

The compound of formula XLV (prepared in the same manner as described in the reaction of scheme 5) can be converted to the compound of formula LIII via the reaction of step (7-1) by alkylating the compound of formula XLV with the compound of formula LII. This reaction can be carried out in the presence of approximately a molar equivalent of a conventional base that converts acetophenone to 3-keto ester (i.e. gamma-keto ester). In carrying out this reaction it is generally preferred but not limited to utilize alkali metal salts of hexamethyldisilane such as lithium bis-(trimethylsilyl) amide and the like. Generally this reaction is carried out in inert solvents such as tetrahydrofuran: 1,3-Dimethyl-3,4,5,6-tetrahydro-2 (1H)-pyrimidinone. Generally the reaction is carried out at temperatures of from -65°C to 25°C. Any of the conditions conventional in such alkylation reactions can be utilized to carry out the reaction of step (7-1).

The compound of formula LIII is the compound of formula I where m is 2 to 4, n is 0, $R^8$ is =O, $R^9$ is absent and $R^{12}$ is alkyl group having from 1 to 3 carbon atoms.

The compound of formula LIII can be converted to the compound of formula LIV via reaction of step (7-2) where X is $N(R^{13})$ wherein $R^{13}$ is hydrogen or alkyl having 1 to 3 carbon atoms and $R^{12}$ is H by ester hydrolysis or compound of formula LIV where X is C(O) and r is 0 and t is 0 and $R^{12}$ is H by catalytic hydrogenation. Any conventional methods of catalytic hydrogenation to remove benzyl group or ester hydrolysis can be utilized to produce the compound of formula LIV.

The compound of formula LIV is the compound of formula I where m is 2 to 4, n is 0, $R^8$ is =O, $R^9$ is absent and $R^{12}$ is H.

The conventional techniques such as extraction, evaporation, chromatography and recrystallization can be utilized to purify the product of all steps.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 7

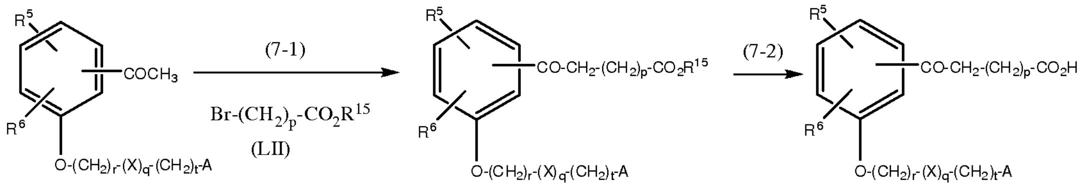


(XLV) (LIII) (LIV)

The compound of formula I where m is 0 to 4, q is 0 or 1, t is 0 or 1, and r is 0, 1 or 2, n is 1, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is =O and $R^9$ is absent. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

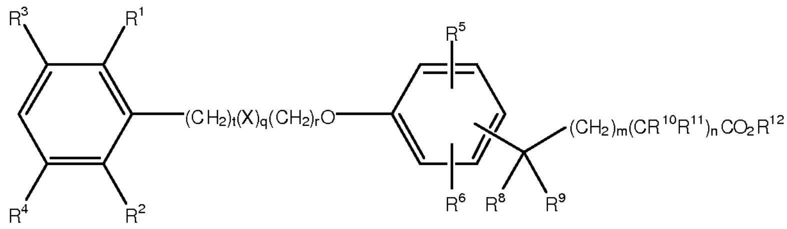

(I)

wherein $R^1$, $R^2$, $R^3$ and $R^4$ are described as above, can be prepared via reaction scheme of Scheme 8.

In the reaction scheme of Scheme 8,

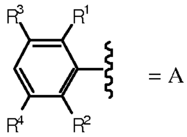

and q, t, m, n, r, $R^5$, $R^6$, $R^{10}$, $R^{11}$ are as above, and u is 1 to 5. $R^{15}$ is alkyl group having from 1 to 3 carbon atoms or benzyl group. $R^{17}$ is alkyl group having from 1 to 3 carbon atoms, and Y is halide.

The compound of formula LVI can be converted to the compound of formula LVIII via reaction of step (8-1) in the same manner as described hereinbefore in the reaction of (3-3).

The compound of LVIII is the compound of formula I where m is 0 to 4, n is 1, $R^8$ is =O, $R^9$ is absent and $R^{12}$ is alkyl group having 1 to 3 carbon atoms.

The compound of formula LVIII can be converted to the compound of formula LIX via reaction of step (8-2) where X is $N(R^{13})$ wherein $R^{13}$ is hydrogen or alkyl having 1 to 3 carbon atoms and $R^{12}$ is H by ester hydrolysis or compound of formula LIX where X is C(O) and r is 0 and t is 0 and $R^{12}$ is H by catalytic hydrogenation. Any conventional methods of ester hydrolysis and catalytic hydrogenation can be utilized to produce the compound of formula LIX.

The compound of formula LIX is the compound of formula I where m is 0 to 4, n is 1, $R^8$ is =O, $R^9$ is absent and $R^{12}$ is H.

The conventional techniques such as extraction, evaporation, chromatography and recrystallization can be utilized to purify the product of all the steps.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 8

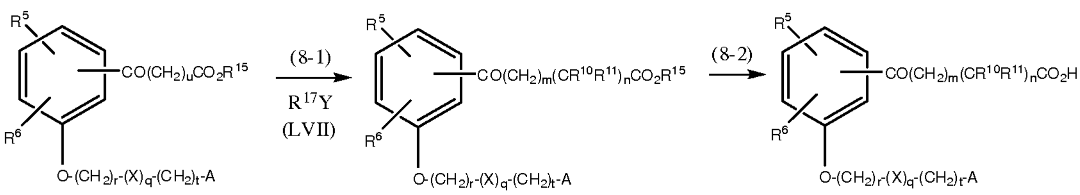


(LVI) (LVIII) (LIX)

The compound of formula I where m is 0, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydroxy and $R^9$ is hydrogen. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

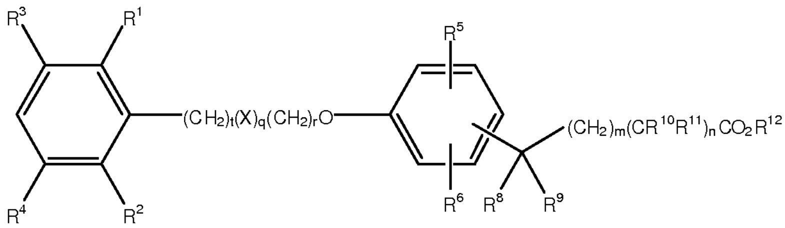

(I)

wherein $R^1$, $R^2$, $R^3$ and $R^4$ are described as above, can be prepared via reaction scheme of Scheme 9.

In the reaction scheme of Scheme 9,

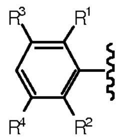

= A

and q, t, r, $R^5$, and $R^6$ are as above. $R^{17}$ is alkyl group having from 1 to 3 carbon atoms.

The compound of formula XLVIII (prepared in the same manner as described in the reaction of scheme 5) can be converted to the compound of formula LX via reaction of step (9-1) by hydrogenation of alpha-keto acid using catalyst for example rhodium-{amidophosphine-phosphinite} (Tetrahedron: Asymmetry, Vol 8, No. 7, 1083-1099, 1997), $[Ru_2Cl_4(BINAP)_2](NEt_3)$ (EP-A-0 295 890) and the like. Any conditions conventional in such hydrogenations can be utilized to carry out the reaction of step (9-1). Using HPLC can separate racemic mixtures of formula LX. (Chirality 11:420-425 (1999)

The compound of formula LX is the compound of formula I where m is 0, n is 0, $R^8$ is hydroxy, $R^9$ is hydrogen and $R^{12}$ is alkyl group having from 1 to 3 carbon atoms.

The compound of formula LX can be converted to the compound of formula LXI where $R^{12}$ is H in the same manner as described hereinbefore in connection with the reaction of step (1-6).

The compound of formula LXI is the compound of formula I where m is 0, n is 0, $R^8$ is hydroxy, $R^9$ is hydrogen and $R^{12}$ is H.

The product of all the steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 9

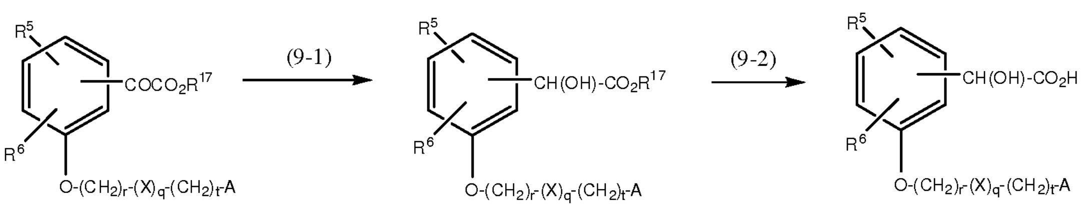


(XLVIII) (LX) (LXI)

The compound of formula I where m is 1, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydroxy and $R^9$ is hydrogen. One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

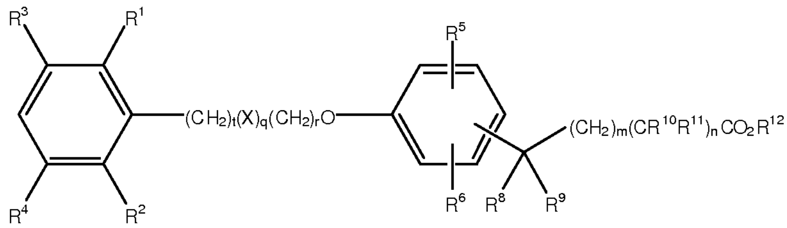

(I)

wherein $R^1$, $R^2$, $R^3$ and $R^4$ are described as above, can be prepared via reaction scheme of Scheme 10.

In the reaction scheme of Scheme 10,

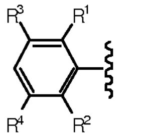 = A

and q, t, r, $R^5$, and $R^6$ are as above. $R^{17}$ is alkyl group having from 1 to 3 carbon atoms.

The compound of formula L (prepared in the same manner as described in the reaction of scheme 6) can be converted the compound of formula LXII via reaction of step (10-1) by reducing the beta -keto group to an alcohol group. The reaction can be carried out by utilizing a conventional reducing agent that converts ketone to an alcohol for example, by hydrogenation using a Raney nickel catalyst that had been treated with tartaric acid (Harada, T.; Izumi, Y. Chem. Lett. 1978, 1195-1196) or hydrogenation with a chiral homogeneous ruthenium catalyst (Akutagawa, S.; Kitamura, M.; Kumobayashi, H.; Noyori, R.; Ohkuma, T.; Sayo, N.; Takaya, M. J. Am. Chem. Soc. 1987, 109, 5856-5858). Utilizing sodium borohydride in solvents such as methanol, ethanol and the like can also carry out the reduction. Generally the reaction is carried out at temperatures from 0°C to 25°C for 2 to 72 hours.

Racemic mixtures of formula LXII can be separated by using HPLC. (Chirality 11:420-425 (1999).

The compound of formula LXII is the compound of formula I where m is 1, n is 0, $R^8$ is hydroxy, $R^9$ is hydrogen and $R^{12}$ is alkyl having from 1 to 3 carbon atoms.

The compound of formula LXII can be converted to compound of formula LXIII via reaction of step (10-2) where $R^{12}$ is H in the same manner as described hereinbefore in connection of the reaction of step (1-6).

The compound of formula LXIII is the compound of formula I where m is 1, n is 0, $R^8$ is hydroxy, $R^9$ is hydrogen and $R^{12}$ is H.

The product of all steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 10

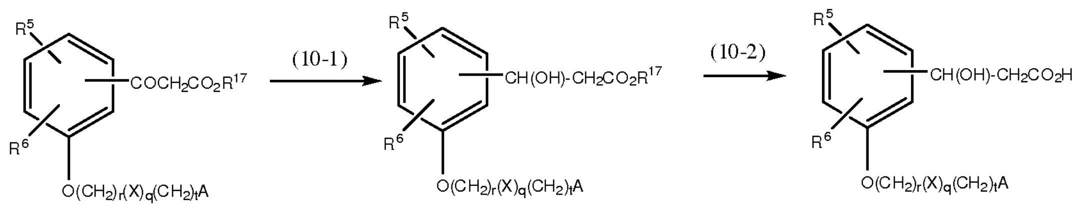


(L) (LXII) (LXIII)

The compound of formula I where m is 2 to 4, q is 0 or 1, t is 0 or 1, and r is 0, 1 or 2, n is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydroxy and $R^9$ is hydrogen. One of $R^{10}$ and $R^{11}$ is alkyl having from 1 to 3 carbon atoms, and the other is hydrogen or alkyl having from 1 to 3 carbon

atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

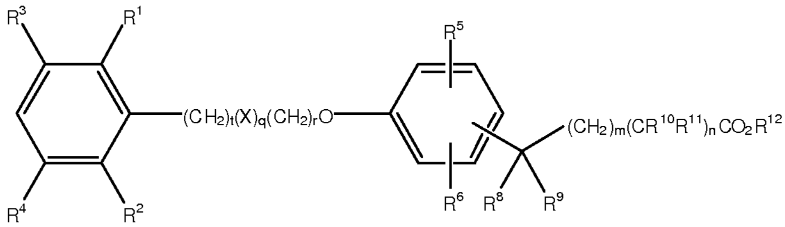

(I)

wherein $R^1$, $R^2$, $R^3$ and $R^4$ are described as above, can be prepared via reaction scheme of Scheme 11.

In the reaction scheme of Scheme 11,

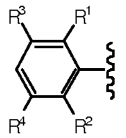 = A

and q, t, r, $R^5$, and $R^6$ are as above. $R^{15}$ is alkyl group having from 1 to 3 carbon atoms or benzyl group and p is 1 to 3.

The compound of formula LIII (prepared in the same manner as described in the reaction of scheme 7) can be converted to the compound of formula LXIV via reaction of step (11-1) by reducing the ketone group to an alcohol group. The reaction can be carried out by utilizing a conventional reducing agent, which converts ketone to alcohol. In carrying out this reaction it is generally preferred but not limited to utilize sodium borohydride as the reducing agent. Generally this reaction is carried out in solvents such as methanol, ethanol and the like. Generally the reaction is carried out at temperatures of from 0°C to 25°C.

Racemic mixtures of formula LXIV can be separated by using HPLC. (Chirality 11:420-425 (1999).

The compound of formula LXIV is the compound of formula I where m is 2 to 4, n is 0, $R^8$ is hydroxy, $R^9$ is hydrogen and $R^{12}$ is alkyl group having from 1 to 3 carbon atoms. The compound of formula LXIV can be converted to the compound of formula LXV where $R^{12}$ is H by ester hydrolysis or catalytic hydrogenation via reaction of step (11-2) in the same manner as described hereinbefore in connection with the reaction of step (1-6). Any conventional methods of ester hydrolysis or catalytic hydrogenation will produce the compound of formula LXV.

The compound of formula LXV is the compound of formula I where m is 2 to 4, n is 0, $R^8$ is hydroxy, $R^9$ is hydrogen and $R^{12}$ is H.

The product of all the steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 11

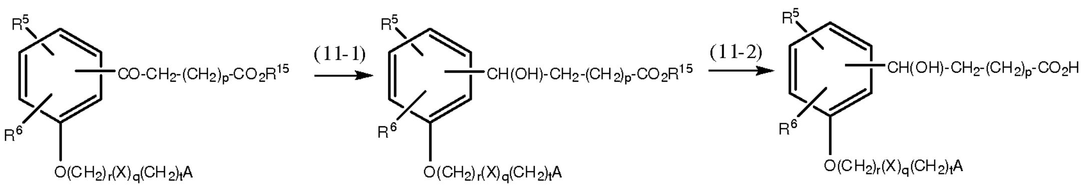


(LIII) (LXIV) (LXV)

The compound of formula I where m is 0 to 4, q is 0 or 1, t is 0 or 1, and r is 0, 1 or 2, n is 1, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^8$ is hydroxy and $R^9$ is hydrogen.

One of $R^{10}$ and $R^{11}$ is alkyl having from 1 to 3 carbon atoms, and the other is hydrogen or alkyl having from 1 to 3 carbon atoms, and X is C(O), r is 0 and t is 0; X is $N(R^{13})$ wherein $R^{13}$ is hydrogen or alkyl having from 1 to 3 carbon atoms. $R^{12}$ is hydrogen or alkyl having from 1 to 3 carbon atoms, i.e. compounds of formula:

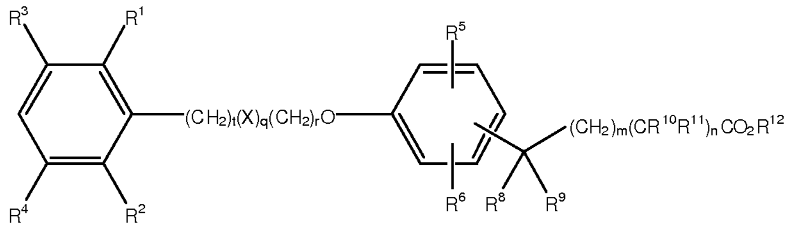

(I)

wherein $R^1$, $R^2$, $R^3$ and $R^4$ are described as above, can be prepared via reaction scheme of Scheme 12.

In the reaction scheme of Scheme 12,

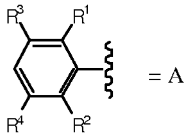

and q, t, r, m, n, $R^5$, $R^6$, $R^{10}$, and $R^{11}$ are as above. $R^{15}$ is alkyl group having from 1 to 3 carbon atoms or benzyl group.

The compound of formula LVIII (prepared in the same manner as described in the reaction of scheme 8) can be converted to the compound of formula LXVI via reaction of step (12-1) in the same manner as described hereinbefore in the reaction of step (11-1). Racemic mixtures of formula LXVI can be separated by using HPLC. (Chirality 11:420-425 (1999).

The compound of formula LXVI is the compound of formula I where m is 0 to 4, n is 1, $R^8$ is hydroxy, $R^9$ is hydrogen and $R^{12}$ is alkyl group having from 1 to 3 carbon atoms.

The compound of formula LXVI can be converted to the compound of formula LXVII where $R^{12}$ is H via reaction of step (12-2) in the same manner as described hereinbefore in the reaction of step (1-6).

The compound of formula LXVII is the compound of formula I where m is 0 to 4, n is 1, $R^8$ is hydroxy, $R^9$ is hydrogen and $R^{12}$ is H.

The product of all the steps can be isolated and purified by techniques such as extraction, evaporation, chromatography, and recrystallization.

If A is phenyl substituted by hydroxy or amino groups, or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 12

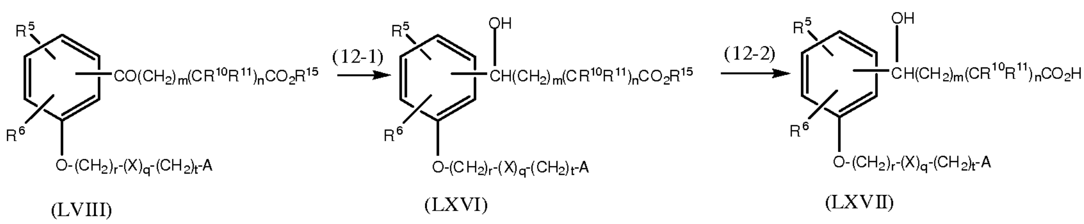


(LVIII) (LXVI) (LXVII)

The compound of formula I where q is 0 or 1, t is 0 or 1, and r is 0, 1 or 2, y is 0, 1, 2 or 3, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms. X is C(O), r is 0 and t is 0; X is $N(R^{13})$ wherein $R^{13}$ is hydrogen or alkyl having from 1 to 3 carbon atoms, i.e. compounds of formula:

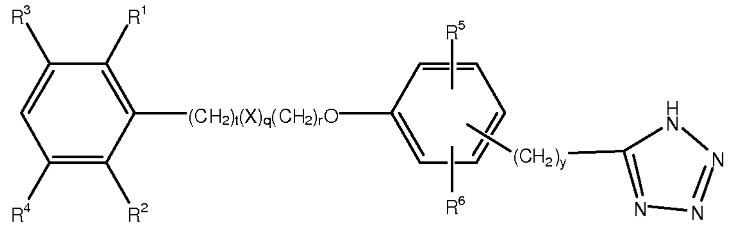

(I)

wherein $R^1$, $R^2$, $R^3$ and $R^4$ are described as above, can be prepared via reaction scheme of Scheme 13.

In the reaction scheme of Scheme 13,

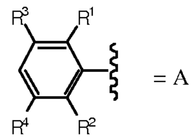

and q, t, r, y, $R^5$, and $R^6$ are as above. Y is halide or leaving group.

The compound of formula LXVIII can be converted to the compound of formula LXIX via reaction of step (13-1) using Mitsunobu condensation of LXVIII with IX using triphenylphosphine and diethyl azodicarboxylate or diisopropyl azodicarboxylate. The reaction is carried out in a suitable solvent for example tetrahydrofuran. Any of the conditions conventionally used in Mitsunobu reactions can be utilized to carry out the reaction of step (13-1).

The compound of formula LXIX can also be prepared by etherifying or alkylating the compound of formula LXVIII with the compound of formula X as in reaction of step (13-1) by using suitable base for example potassium carbonate, cesium carbonate, triethylamine, pyridine and the like. The reaction is carried out in solvents for example dimethylformamide, acetonitrile, dichloromethane and the like. In the compound of formula X, Y, include but are not limited to mesyloxy, tosyloxy, chloro, bromo, iodo, and the like. Any conventional method of etherifying of a hydroxyl group by reaction with a halide or leaving group can be utilized to carry out the reaction of step (13-1).

The compound of formula LXIX can be converted to the compound of formula LXX via reaction of step (13-2) by reacting the nitrile with an azide for example trimethylsilyl azide or with metal azide for example sodium azide, potassium azide, lithium azide preferred azide being sodium azide in the presence of lewis acid for example zinc chloride, magnesium chloride, ammoinum chloride, tin tetrachloride and the like. The reaction is carried out in the solvent for example dimethylformamide at the temperature of 80°C to 145°C from 6 to 60 hours. The ideal reaction utilizes reacting nitrile with sodium azide/ammonium chloride/dimethylformamide at 120°C for 24 hours.

The product of all the steps can be isolated and purified by techniques such as extraction, evaporation, chromatography, and recrystallization.

If A is phenyl substituted by hydroxy or amino groups or one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 13

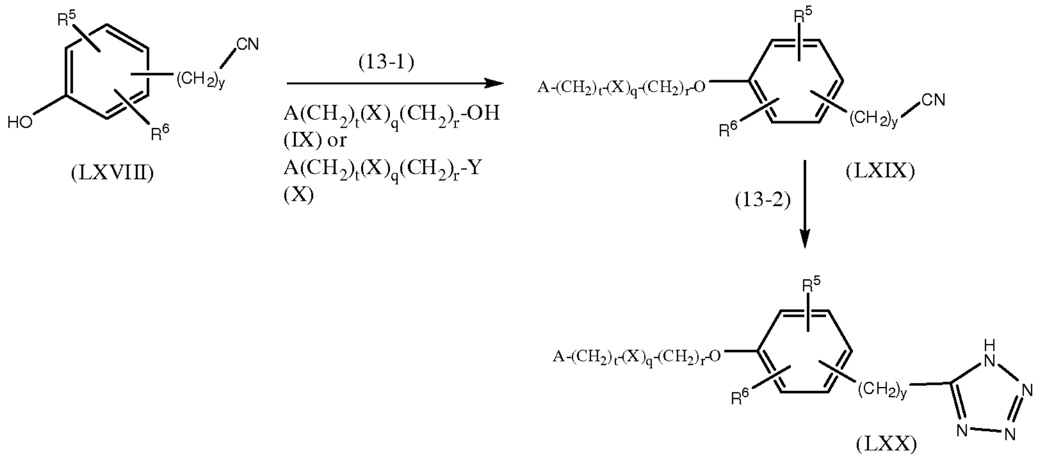


(LXVIII) (LXIX) (LXX)

The compound of formula LXVIII where y is 1 to 3, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, i.e. compounds of formula:

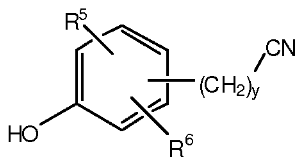

(LXVIII)

wherein $R^5$, $R^6$ and y is described as above, can be prepared via reaction of scheme 14. The compound of formula LXXI can be alkylated to the compound of formula LXXII via reaction of step (14-1) by methyl halide in the presence of 3 equivalent molar base for example potassium carbonate, cesium carbonate, sodium hydride and the like in solvent for example dimethylformamide, tetrahydrofuran, acetone to give the compound of formula LXXII. Any conventional conditions of such alkylation can be utilized to carry out the reaction of step (14-1).

The compound of formula LXXII can be reduced to the compound of formula LXXIII by utilizing conventional reducing reagent that converts ester group to alcohol via reaction of step (14-2). In carrying out this reaction it is generally preferred but not limited to utilize lithium aluminum hydride. The reaction is carried out in a suitable solvent such as tetrahydrofuran, ether and the like. Any of the conditions conventional in such reduction reactions can be utilized to carry out the reaction of step (14-2).

The compound of formula LXXIII can be converted to the compound of formula LXXIV by displacing hydroxy group with a halogen preferred halogen being bromo or chloro. Appropriate halogenating reagents include but are not limited to thionyl chloride, oxalyl chloride, bromine, phosphorous tribromide, carbon tetrabromide and the like. Any conditions conventional in such halogenation reactions can be utilized to carry out the reaction of step (14-3).

The compound of formula LXXIV can be converted to the compound of formula LXXV by reacting LXXIV with alkali metal cyanide for example sodium or potassium cyanide or copper cyanide. The reaction can be carried out in a suitable solvent for example dimethyl sulfoxide, dimethylformamide and the like. Any of the conditions conventionally used in the preparation of nitriles from halides can be utilized to carry out the reaction of step (14-4).

The compound of formula LXXV can be demethylated to the compound of formula LXXVI by utilizing lewis acid for example $BBr_3$ or $BCl_3$ in dichloromethane or chloroform at temperature for example -78$^0$C to 25°C. Any of the conditions conventional in such demethylation reactions can be utilized to carry out the reaction via the reaction of step (14-5).

The compound of formula LXXVI is the compound of formula LXVIII where y is 1.

The compound of formula LXXV can be converted to the compound of formula LXXVII via reaction step (14-6) by hydrolysis. In carrying out this reaction, it is generally preferred to utilize basic hydrolysis, for example aqueous sodium hydroxide in ethanol and the like. Any of the conditions conventional in the hydrolysis of nitriles to carboxylic acids can be utilized to carry out the reaction of step (14-6).

The compound of formula LXXVII can be reduced to give the compound of formula LXXVIII via reaction of step

(14-7). This reaction can be carried out in the same manner as described hereinbefore in the reaction of step (14-2).

The compound of formula LXXVIII can be converted to the compound of formula LXXIX via reaction of step (14-8) in the same manner as described hereinbefore in the reaction of step (14-3).

The compound of formula LXXIX can be converted to the compound of formula LXXX via reaction of step (14-9) in the same manner as described hereinbefore in the reaction of step (14-4).

The compound of formula LXXX can be converted to the compound of formula LXXXI via reaction of step (14-10) in the same manner as described hereinbefore in the reaction of step (14-5).

The compound of formula LXXXI is the compound of formula LXVIII where y is 2.

The compound of formula LXXX can be hydrolyzed in the same manner as described hereinbefore in the reaction of step (14-6) to give the compound of formula LXXXII via the reaction of step (14-11).

The compound of formula LXXXII can be reduced to give the compound of formula LXXXIII via reaction of step (14-12). This reaction can be carried out in the same manner as described hereinbefore in the reaction of step (14-2). The compound of formula LXXXIII can be converted to the compound of formula LXXXIV via reaction of step (14-13) in the same manner as described hereinbefore in the reaction of step (14-3).

The compound of formula LXXXIV can be converted to the compound of formula LXXXV via reaction of step (14-14) in the same manner as described hereinbefore in the reaction of step (14-4).

The compound of formula LXXXV can be converted to the compound of formula LXXXVI via reaction of step (14-15) in the same manner as described hereinbefore in the reaction of step (14-5).

The compound of formula LXXXVI is the compound of formula LXVIII where y is 3.

The product of all the steps can be isolated and purified by techniques such as extraction, evaporation, chromatography, and recrystallization.

If one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 14

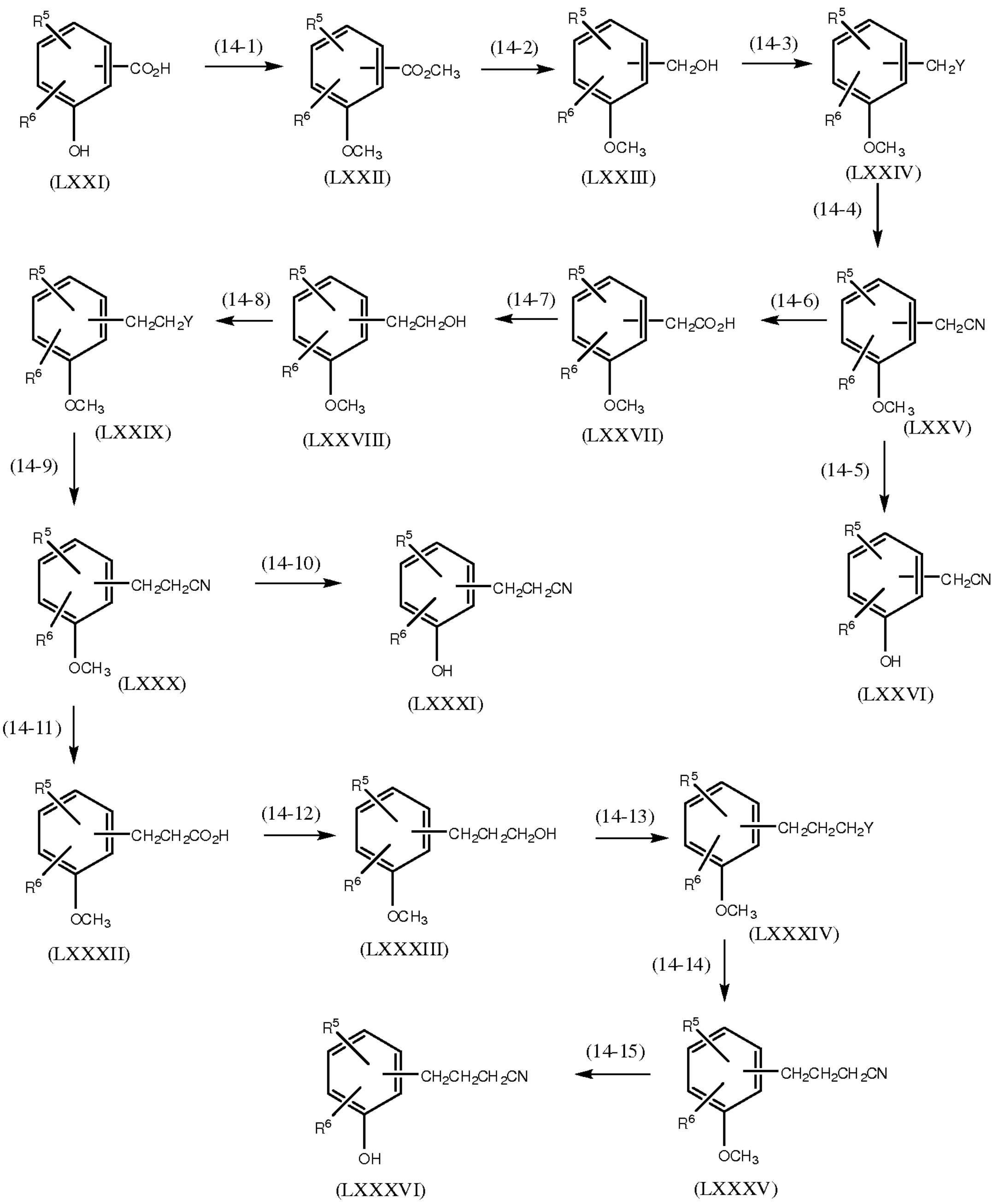


The compound of formula LXVIII where y is 0, one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, i.e. compounds of formula:

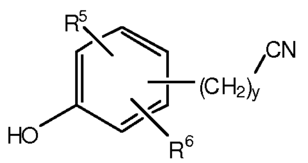

(LXVIII)

wherein $R^5$, $R^6$ and y is described as above, can be prepared via reaction scheme of Scheme 15.

In the reaction of Scheme 15, $R^{16}$ is alkyl group having from 1 to 2 carbon atoms. P is a hydroxy protecting group. Y is chloro or bromo.

The compound of formula LXXXVII can be converted to the compound of formula LXXXVIII via two steps; first protecting the hydroxy group by utilizing suitable protecting groups such as those described in Protective Groups in Organic Synthesis by T. Greene and then hydrolyzing ester to give carboxylic acid via the reaction step of (15-1).

The intermediate from the reaction step (15-1) can be converted to the compound of formula LXXXVIII by reacting with halogenating reagent for example thionyl chloride, oxalyl chloride, phosphorous pentachloride, phosphorous trichloride, bromine, carbon tetrabromide and the like. The reaction is carried out either neat or in suitable solvent for example dichloromethane. Any of the conditions conventional in such halogenation reactions of carboxylic acids can be utilized to carry out the reaction of step (15-2).

The compound of formula LXXXVIII can be converted to the compound of formula LXXXIX via reaction of (15-3) by reacting with ammonia directly or by first treating the compound of formula LXXXVIII with coupling reagent for example dicyclohexylcarbodiimide, benzotriazolyloxy)tris(dimethylamino)phosphonium hexafluorophosphate and then reacting with ammonia. Any of the conditions conventional in acylation of ammonia can be utilized to carry out the reaction of step (15-3).

The compound of formula LXXXIX can be converted to the compound of formula XC via reaction of step (15-4) by dehydration utilizing reagents for example thionyl chloride, phosphorous pentoxide, phosphorous pentachloride, phosphorous oxychoride, carbon tetrachloride-triphenylphosphine, cyanuric chloride, and the like. The reaction is carried out either neat or in suitable solvent for example dimethylformamide. Any of the conditions conventional in such dehydration reaction can be utilized to carry out the reaction of step (15-4).

The compound of formula XC can be converted to the compound of formula XCI via reaction of step (15-5) by removal of hydroxy protecting group utilizing suitable deprotecting reagents such as those described in Protective Groups in Organic Synthesis by T. Greene.

The compound of formula XCI is the compound of formula LXVIII where y is 0.

The product of all the steps can be isolated and purified by techniques such as extraction, evaporation, chromatography, and recrystallization.

If one of $R^5$ and $R^6$ is hydrogen and the other is selected from the group consisting of hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 15

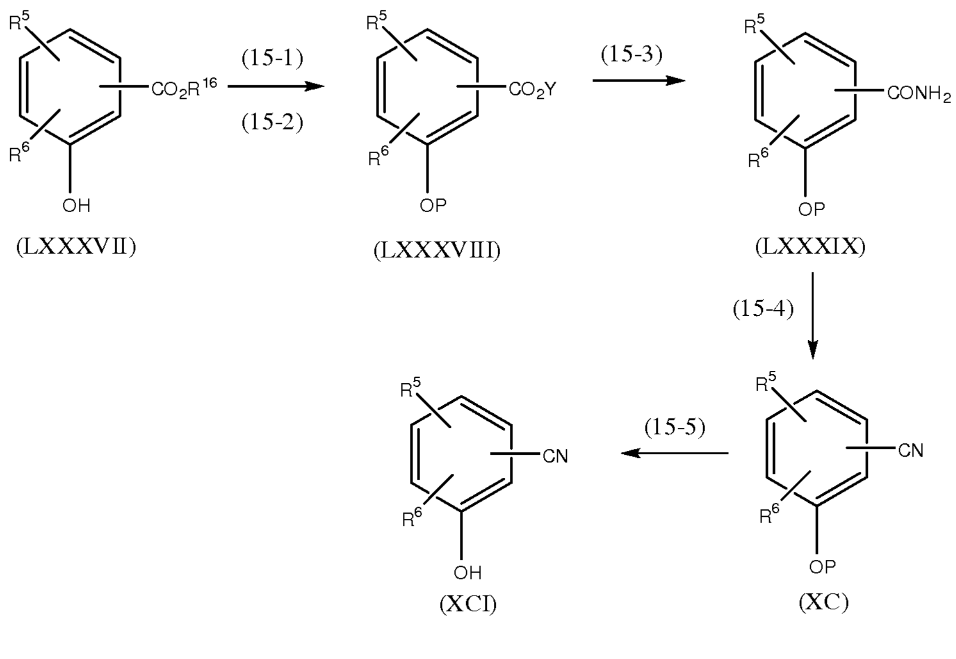


The compound of formula IX, where t is 0 or 1, r is 0, 1 or 2 and q is 0, i.e. compounds of formula:

$$A\text{-}(CH_2)_t(X)_q(CH_2)_r\text{-}OH \qquad (IX)$$

and the compound of formula X, where t is 0 or 1, r is 0, 1 or 2 and q is 0, i.e. compounds of formula:

$$A\text{-}(CH_2)_t(X)_q(CH_2)_r\text{-}Y \qquad (X)$$

can be prepared via reaction scheme of Scheme 16.

In the reaction scheme of Scheme 16,

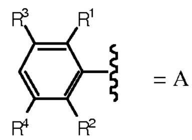

wherein $R^1$, $R^2$, $R^3$, and $R^4$ are described as above and Y is chloro or bromo.

The compound of formula XCII can be reduced to the compound of formula XCIII via reaction of step (16-1). The reaction is carried out utilizing a conventional reducing agent for example alkali metal hydride such as lithium aluminum hydride, borane dimethyl sulfide complex. The reaction is carried out in a suitable solvent, such as tetrahydrofuran, ether, dichloromethane and the like. Any of the conditions conventional in such reduction reactions can be utilized to carry out the reaction of step (16-1).

The compound of formula XCIII is the compound of formula IX where t is 0 and r is 1.

The compound of formula XCIII can be converted to the compound of formula XCIV by displacing hydroxyl group with a halogen group preferred halogen being bromo or chloro. Appropriate halogenating reagents include but are not limited to thionyl chloride, oxalyl chloride, bromine, phosphorous tribromide, carbon tetrabromide and the like. Any conditions conventional in such halogenation reactions can be utilized to carry out the reaction of step (16-2).

The compound of formula XCIV is the compound of formula X where t is 0 and r is 1.

The compound of formula XCIV can be converted to the compound of formula XCV by reacting XCIV with alkali metal cyanide for example sodium or potassium cyanide. The reaction is carried out in a suitable solvent, such as ethanol, dimethyl sulfoxide, and dimethylformamide. Any of the conditions conventionally used in the preparation of nitrile can be utilized to carry out the reaction of step (16-3).

The compound of formula XCV can be converted to the compound of formula XCVI via reaction step (16-4) by hydrolysis. In carrying out this reaction it is generally preferred to utilize basic hydrolysis, for example aqueous sodium hydroxide. Any of the conditions conventionally used in hydrolysis of nitrile can be utilized to carry out the reaction of step (16-4).

The compound of formula XCVI can be reduced to give the compound of formula XCVII via reaction of step (16-5). This reaction can be carried out in the same manner as described hereinbefore in the reaction of step (16-1).

The compound of formula XCVII is the compound of formula IX where t is 1 and r is 1.

The compound of formula XCVII can be converted to the compound of formula XCVIII via reaction of step (16-6) in the same manner as described hereinbefore in connection with the reaction of step (16-2).

The compound of formula XCVIII is the compound of formula X where t is 1 and r is 1.

The compound of formula XCVIII can be converted to the compound of formula XCIX via reaction of step (16-7) in the same manner as described hereinbefore in connection with the reaction of step (16-3).

The compound of formula XCIX can be hydrolyzed by base in the same manner as described hereinbefore in connection with the reaction of step (16-4) to give the compound of formula C via reaction of step (16-8).

The compound of formula C can be converted to the compound of formula CI via reaction of step (16-9) in the same manner as described hereinbefore in connection with the reaction of step (16-1).

The compound of formula CI is the compound of formula IX where t is 1 and r is 2.

The compound of formula CI can be converted to the compound of formula CII via reaction of step (16-10) in the same manner as described hereinbefore in connection with the reaction of step (16-2).

The compound of formula CII is the compound of formula X where t is 1 and r is 2.

The product of the each step can be isolated and purified by techniques such as extraction, evaporation, chromatography, and recrystallization.

If A is phenyl substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 16

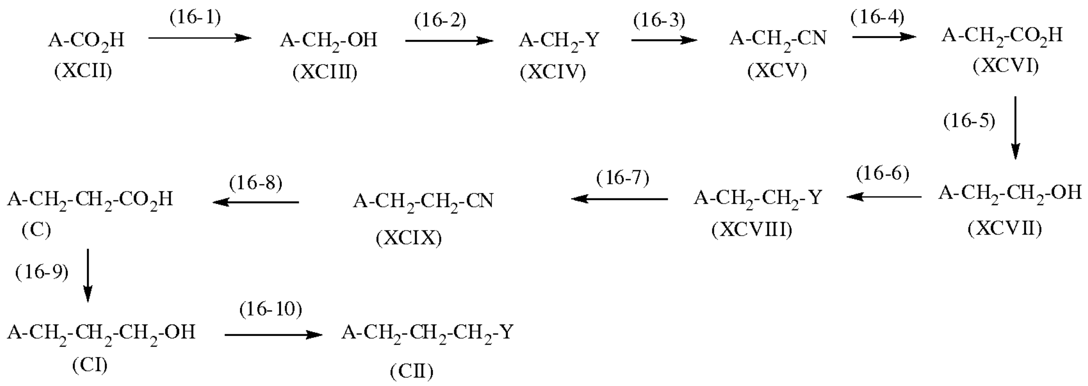


The compound of formula IX, where t is 0 or 1, r is 0, 1 or 2 and q is 1 and X is $N(R^{13})$ wherein $R^{13}$ is hydrogen or alkyl having from 1 to 3 carbon atoms, i.e. compounds of formula:

$$A\text{-}(CH_2)_t(X)_q(CH_2)_r\text{-}OH \qquad (IX)$$

and the compound of formula X, where t is 0 or 1, r is 0, 1 or 2 and q is 1 and X is $N(R^{13})$ wherein $R^{13}$ is hydrogen or alkyl having from 1 to 3 carbon atoms, i.e. compounds of formula:

$$A\text{-}(CH_2)_t(X)_q(CH_2)_r\text{-}Y \qquad (X)$$

can be prepared via reaction scheme of Scheme 17.

In the reaction scheme of Scheme 17,

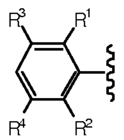 = A

wherein $R^1$, $R^2$, $R^3$, and $R^4$ are described as above and Y is chloro or bromo.

The compound of formula CIII can be mesylated to furnish the compound of formula CIV via the reaction of step (17-1). Any conventional conditions to carry out the mesylation reaction of a hydroxyl group can be utilized to carry out the step (17-1). The compound of formula CIV is then heated with the compound of formula CV to produce the compound of formula CVI. Any of the conditions conventional in producing amino alcohols can be utilized to carry out the reaction of step (17-2).

The compound of formula CV is the compound of formula IX.

In the compound of formula CVI, alcohol can be displaced by chloro or bromo by treating the compound of formula CVI with thionyl chloride, bromine, phosphorus tribromide, oxalyl chloride, carbon tetrabromide and the like to produce the compound of formula CVII. Any conventional method to displace alcohol with chloro or bromo can be utilized to carry out the reaction of step (17-3).

The compound of formula CVII is the compound of formula X.

The product of each step can be isolated and purified by techniques such as extraction, evaporation, chromatography, and recrystallization.

If A is phenyl substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 17

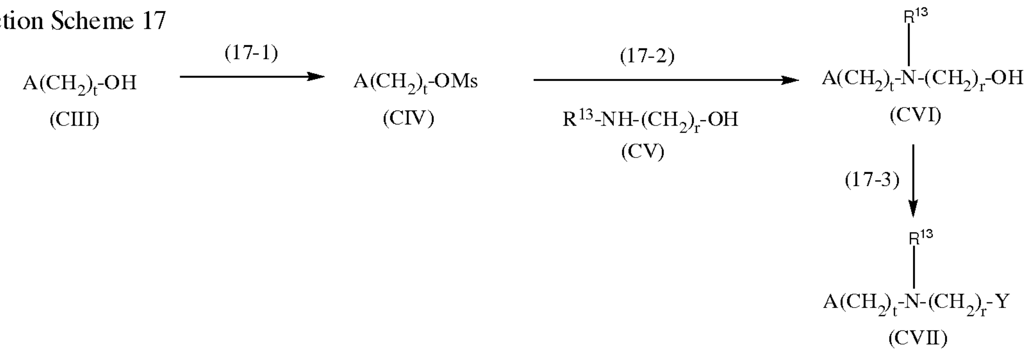

(CIII) (CIV) (CVI) (CVII)

The compound of formula CVIII where one of $R^3$ and $R^4$ is hydrogen, and the other is hydroxy, amino, nitro and alkoxy having from 1 to 2 carbon atoms, $R^{16}$ is alkyl group having from 1 to 2 carbon atoms, i.e. compounds of formula:

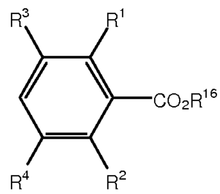

(CVIII)

wherein, $R^1$ and $R^2$ are described as above, can be prepared via reaction scheme of Scheme 18.

In the reaction of scheme 18, $R^1$, $R^2$, $R^4$ and $R^{16}$ are as above.

The compound of formula CIX can be nitrated via reaction of step (18-1) by using mixture of concentrated nitric and sulfuric acid or by using neat nitric acid or nitric acid in water or acetic acid to give the compound of formula CX. The reaction is carried out from 0°C to 100°C for 4 to 72 hours.

The compound of formula CX can be converted to the compound of formula CXI via the reaction of step (18-2) in the same manner as described hereinbefore in connection with the reaction of step (5-4).

The compound of formula CXI is the compound of formula CVIII where $R^3$ is nitro.

The compound of formula CXI can be reduced to the compound of formula CXII via reaction of step (18-3) by utilizing metals for example Zn, Sn, or Fe and acid, or by catalytic hydrogenation. Any conventional conditions in such reductions can be utilized to carry out the reaction of step (18-2).

The compound of formula CXII is the compound of formula CVIII where $R^3$ is amino.

The compound of formula CXII can be converted to the compound of formula CXIII via reaction of step (18-4) by first diazotization of amine using aqueous sulfuric acid at higher temperatures and then by adding sodium nitrite in water at 0-5°C.

The intermediate diazonium solution was further heated with aqueous sulfuric acid at 100-110°C to give the compound of formula CXIII. Any conventional conditions in such conversion of amino to phenol can be utilized to carry out the reaction of step (18-4).

The compound of formula CXIII is the compound of formula CVIII where $R^3$ is hydroxy.

The compound of formula CXIII can be converted to the compound of CXIV via reaction of step (18-5) by reacting hydroxy group with methyl or ethyl iodide in the presence of base for example potassium carbonate, cesium carbonate in solvent for example dimethylformamide. Any conventional conditions in such alkylation can be utilized to carry out the reaction of step (18-5).

The compound of formula CXIV is the compound of formula CVIII where $R^3$ is alkoxy having from 1 to 2 carbon atoms.

The product of each step can be isolated and purified by techniques such as extraction, evaporation, chromatography, and recrystallization.

If $R^1$ and $R^2$ are substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 18

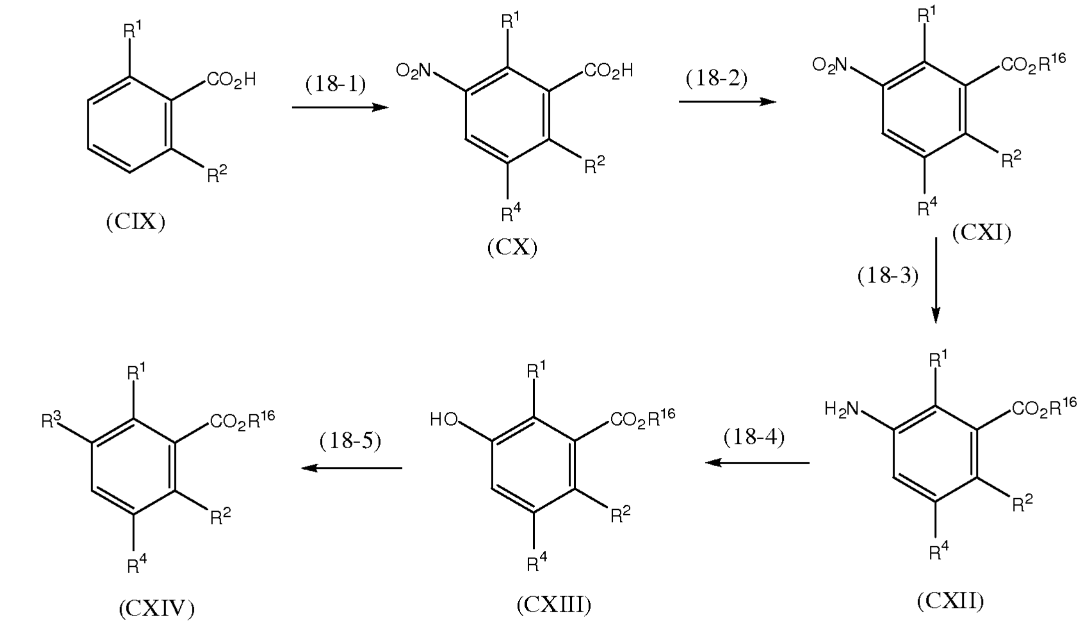


The compound of formula CVIII where one of $R^3$ and $R^4$ is hydrogen and the other is halo, or cyano, and $R^{16}$ is alkyl group having from 1 to 2 carbon atoms, i.e. compounds of formula:

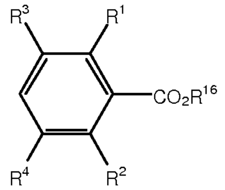

(CVIII)

wherein $R^1$ and $R^2$ are described as above, can be prepared via reaction scheme of Scheme 19.

In the reaction of scheme 19, $R^1$, $R^2$, $R^4$ and $R^{16}$ are as above.

The compound of formula CXII can be converted to the compound of formula CXV via reaction of step (19-1) by first diazotization of amine using aqueous sulfuric acid at higher temperatures and then by adding sodium nitrite in water at 0-5°C.

The intermediate diazonium solution was further heated with aqueous sulfuric acid in the presence of iodide ions for example potassium iodide and copper powder at 50°C-100°C to give the compound of formula CXVIII. Any conventional conditions in such iodo-de-diazoniation can be utilized to carry out the reaction of step (19-1).

The compound of formula CXV is the compound of formula CVIII where $R^3$ is iodo.

The compound of formula CXII can be converted to the compound of formula CXVI or compound of formula CXVIII via reaction of step (19-2) or step (19-4) by first diazotization as in reaction of step (19-1) and then intermediate diazonium solution was further heated with aqueous sulfuric acid in the presence of cuprous chloride and copper powder or copper with HCl at temperature range from 50°C-100°C to give the compound of formula CXVI where $R^3$ is chloro or intermediate diazonium solution was heated with aqueous sulfuric acid in the presence of cuprous bromide and copper powder or copper with HBr at temperature range from 50°C-100°C to give the compound of formula CXVIII. Any conventional conditions in such chloro-de-diazoniation or bromo-de-diazoniation can be utilized to carry out the reaction of step (19-2) or step (19-4).

The compound of formula CXVI is the compound of formula CVIII where $R^3$ is chloro. The compound of formula CXVIIII is the compound of formula CVIII where $R^3$ is bromo.

The compound of formula CXII can be converted to the compound of formula CXVII via reaction of step (19-3) by first diazotization as in reaction of step (19-1) and then intermediate diazonium solution was further treated with cold aqueous solution of $NaBF_4$, $HBF_4$ or $NH_4BF_4$ to give fluoroborate salts which can be heated to give the compound of formula CXVII. Any conventional conditions of such fluoro-de-diazoniation can be utilized to carry out the reaction of

step (19-3).

The compound of formula CXVII is the compound of formula CVIII where $R^3$ is fluoro.

The compound of formula CXVIII can be converted to the compound of formula CXIX by reaction of the compound of formula CXVIII with cuprous cyanide in the presence of solvent such as dimethylformamide at 75°C to 150°C for 6 to 72 hours. Any conventional conditions in such cyano-de-halogenation can be utilized to carry out the reaction of step (19-5).

The compound of formula CXIX is the compound of formula CVIII where $R^3$ is cyano.

The product of each step can be isolated and purified by techniques such as extraction, evaporation, chromatography, and recrystallization.

If $R^1$ and $R^2$ are substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 19

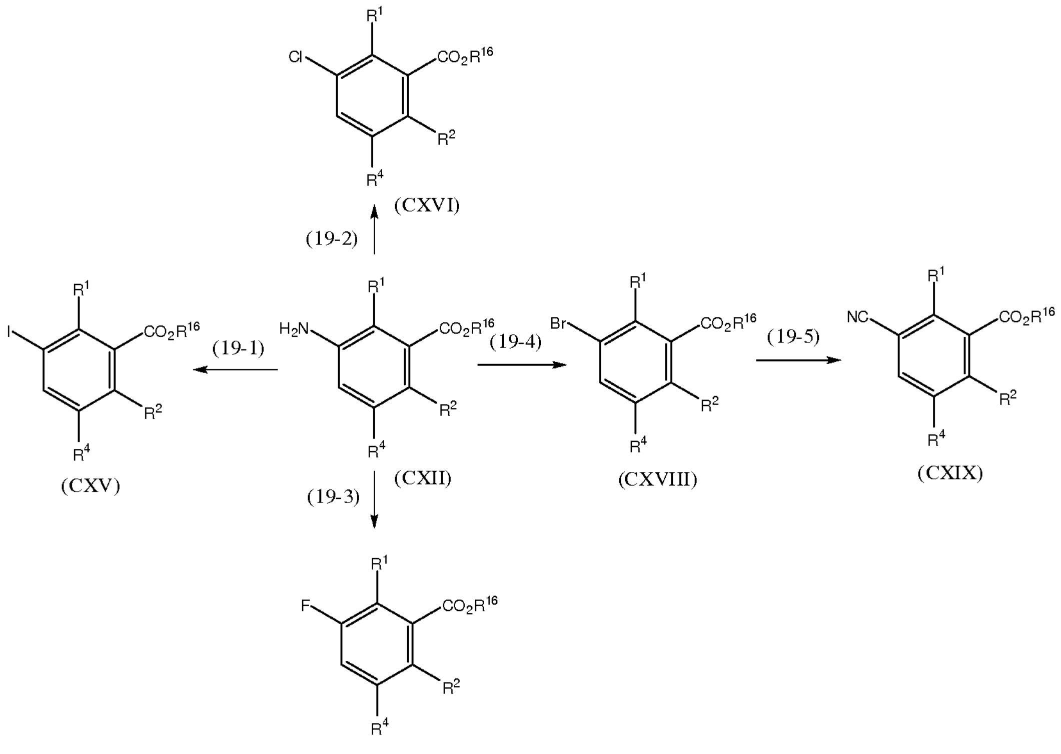

(CXVI) (CXV) (CXII) (CXVIII) (CXIX) (CXVII)

The compound of formula CVIII where one of $R^3$ and $R^4$ is hydrogen and the other is alkyl having from 1 to 2 carbon atoms and $R^{16}$ is alkyl group having from 1 to 2 carbon atoms, i.e. compounds of formula:

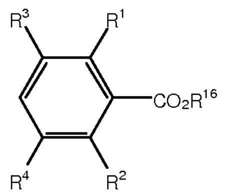

(CVIII)

wherein $R^1$ and $R^2$ are described as above, can be prepared via reaction scheme of Scheme 20.

In the reaction of scheme 20, $R^1$, $R^2$, $R^4$ and $R^{16}$ are as above. $R^{18}$ is alkyl group having from 1 to 2 carbon atoms. The compound of formulas CXV or CXVIII can be converted to the compound of formula CXXII via reaction of step (20-1)

by using Suzuki cross-coupling reaction of the compound of formula CXX with the compound of formula CXV or CXVIII in the presence of base for example $K_2CO_3$, $Cs_2CO_3$, $NaHCO_3$, $Na_2CO_3$, $Et_3N$, and the like. The reaction is catalyzed by palladium complexes for example $PdCl_2(dppf).CH_2Cl_2$, $PdCl_2(dppf)$, $Pd(OAc)_2$, $[Pd(C_3H_5)Cl]_2$ , $PdCl(C_3H_5)(dppb)$ and the like. The reaction is carried out in a suitable solvent, such as toluene, tetrahydrofuran, tetrahydrofuran: water at 25°C to reflux temperature. Any of the conditions conventionally used in the Suzuki couplings can be utilized to carry out the reaction of step (20-1).

The compound of formulas CXV or CXVIII can also be converted to the compound of formula CXXII via reaction of step (20-1) by using Stille cross-coupling reaction of the compound of formula CXXI with the compound of formula CXV or CXVIII catalyzed by palladium for example $Pd(PPh_3)_4$, $Pd_2dba_3$, $Pd(PPh_3)_2Cl_2$ in a suitable solvent such as dioxane, DMF, or HMTP under inert atmosphere at temperature from 25°C to 100°C for 6 to 72 hours. Any of the conditions conventionally used in the Stille couplings can be utilized to carry out the reaction of step (20-1).

The compound of formula CXXII is the compound of formula CVIII where $R^3$ is alkyl group having from 1 to 2 carbon atoms.

The product of each step can be isolated and purified by techniques such as extraction, evaporation, chromatography, and recrystallization.

If $R^1$ and $R^2$ are substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 20

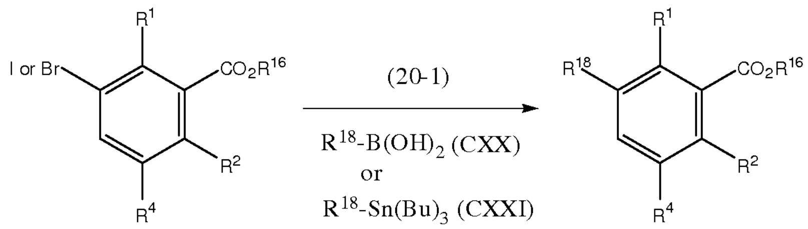


(CXV) or (CXVIII) (CXXII)

The compound of formula XXXIII where one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, i.e. compounds of formula:

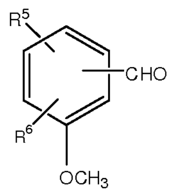

(XXXIII)

wherein $R^5$ and $R^6$ are described as above, can be prepared via reaction scheme of Scheme 21.

The compound of formula LXXIII can be converted to the compound of formula XXXIII via reaction of step (21-1) by oxidation of alcohol to the aldehyde. The reaction can be carried out utilizing a suitable oxidizing agent for example pyridinium chlorochromate, or dimethyl sulfoxide activated by 2,4,6-trichloro[1,3,5]-triazine (cyanuric chloride, TCT) under Swern oxidation conditions (J.O.C. 2001, 66, 7907-7909) and the like. Any of the conditions conventional in such oxidation reactions can be utilized to carry out the reaction of step (21-1).

The product can be isolated and purified by techniques such as extraction, evaporation, chromatography, and recrystallization which are known in the art.

If one of $R^5$ and $R^6$ are substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in

Protective Groups in Organic Synthesis by T. Greene.

Reaction Scheme 21

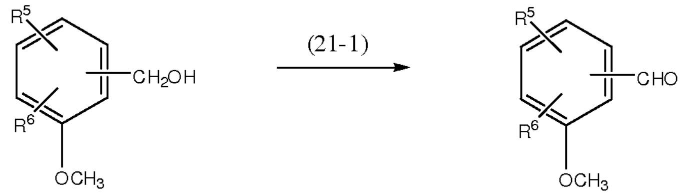


(LXXIII) (XXXIII)

The compound of formula XXXIV where p is 2 to 5 and $R^{15}$ is alkyl group having 1 to 3 carbon atoms or benzyl group, i.e. compounds of formula:

$Ph_3P^+\text{-}(CH_2)_pCO_2R^{15}\}Br^-$ (XXXIV)

can be prepared via reaction of scheme 22.

In the reaction scheme of Scheme 22, $R^{15}$ and p are as above.

The compound of formula CXXIII can be reacted with the compound of formula CXXIV via the reaction of step (22-1) to give compound of formula XXXIV. Any of the conditions conventionally used in reacting triphenylphosphine with hydrohalide can be utilized to carry out the reaction of step (22-1).

The product can be isolated and purified by techniques such as extraction, evaporation, chromatography, and recrystallization which are known in the art.

Reaction Scheme 22

$$(C_6H_5)_3P + Br\text{-}(CH_2)_pCO_2R^{15} \xrightarrow{(22\text{-}1)} Ph_3P^+\text{-}(CH_2)_pCO_2R^{15}\}Br^-$$

(CXXIII) (CXXIV) (XXXIV)

The compound of formula XLIV where one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, i.e. compounds of formula:

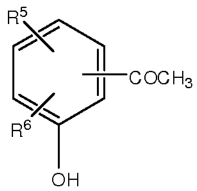

(XLIV)

wherein $R^5$ and $R^6$ are described as above, can be prepared via reaction scheme of Scheme 23.

The compound of formula XLIV can be synthesized according to the method of George M Rubottom et al., J. Org. Chem. 1983, 48, 1550-1552.

The product can be isolated and purified by techniques such as extraction, evaporation, chromatography, and recrystallization.

If one of $R^5$ and $R^6$ are substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected utilizing suitable deprotecting reagents such as those described in Protective Groups in Organic Synthesis by T. Greene.

Reaction Scheme 23

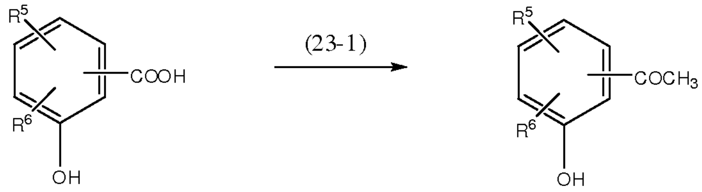


(LXXI) (XLIV)

The compound of formula LXXI where one of $R^5$ and $R^6$ is hydrogen, and the other is halo, i.e. compounds of formula:

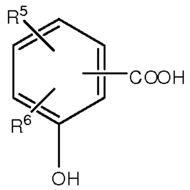

(LXXI)

are either commercially available or can be prepared according to the methods described in the literature as follows:

1. 3-Br or F-2-$OHC_6H_3CO_2H$
Canadian Journal of Chemistry (2001), 79(11) 1541-1545.
2. 4-Br-2-$OHC_6H_3CO_2H$
WO 9916747 or JP 04154773.
3. 2-Br-6-$OHC_6H_3CO_2H$
JP 47039101.
4. 2-Br-3-$OHC_6H_3CO_2H$
WO 9628423.
5. 4-Br-3-$OHC_6H_3CO_2H$
WO 2001002388.
6. 3-Br-5-$OHC_6H_3CO_2H$
Journal of labeled Compounds and Radiopharmaceuticals (1992), 31 (3), 175-82.
7. 2-Br-5-$OHC_6H_3CO_2H$ and 3-Cl-4-$OHC_6H_3CO_2H$
WO 9405153 and US 5519133.
8. 2-Br-4-$OHC_6H_3CO_2H$ and 3-Br-4-$OHC_6H_3CO_2H$
WO 20022018323
9. 2-Cl-6-$OHC_6H_3CO_2H$
JP 06293700
10. 2-Cl-3-$OHC_6H_3CO_2H$
Proceedings of the Indiana Academy of Science (1983), Volume date 1982, 92, 145-51.
11. 3-Cl-5-$OHC_6H_3CO_2H$
WO 2002000633 and WO 2002044145.
12. 2-Cl-5-$OHC_6H_3CO_2H$
WO 9745400.
13. 5-I-2-$OHC_6H_3CO_2H$ and 3-I, 2-$OHC_6H_3CO_2H$
Z. Chem. (1976), 16(8), 319-320.
14. 4-I-2-$OHC_6H_3CO_2H$
Journal of Chemical Research, Synopses (1994), (11), 405.
15. 6-I-2-$OHC_6H_3CO_2H$
US 4932999.
16. 2-1-3-$OHC_6H_3CO_2H$ and 4-1-3-$OHC_6H_3CO_2H$
WO 9912928.

17. 5-1-3-$OHC_6H_3CO_2H$
J. Med. Chem. (1973), 16(6), 684-7.
18. 2-I-4-$OHC_6H_3CO_2H$
Collection of Czechoslovak Chemical Communications, (1991), 56(2), 459-77. 19. 3-I-4-$OHC_6H_3CO_2$,
J.O.C. (1990), 55(18), 5287-91.

The compound of formula LXXI where one of $R^5$ and $R^6$ is hydrogen and the other is alkoxy having from 1 to 3 carbon atoms, i.e. compounds of formula:

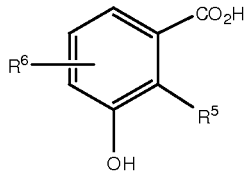

(LXXI)

can be synthesized via the reaction of Scheme 24.

In the reaction of Scheme 24, $R^{16}$ is alkyl group having from 1 to 2 carbon atoms. P is a hydroxy protecting group. The compound of formula CXXV can be converted to the compound of formula CXXVI via reaction of step (24-1) by protecting phenol by suitable protecting group. The suitable protecting group can be described in the Protective Groups in Organic Synthesis by T. Greene.

The compound of formula CXXVI can be converted to the compound of formula CXXVII by oxidation of aldehyde to carboxylic acid. The reaction can be carried out by utilizing suitable oxidizing reagents for example, pyridinium chlorochromate, potassium permanganate, sodium permanganate and the like. Any of the conditions suitable in such oxidation reactions can be utilized to carry out the reaction of step (24-2).

The compound of formula CXXVII can be converted to the compound of formula CXXVIII via reaction of step (24-3) where $R^5$ is alkoxy having 1 carbon atom by deprotecting the protecting group. The suitable deprotecting conditions can be described in the Protective Groups in Organic Synthesis by T Greene.

The compound of formula CXXVII can be converted to the compound of formula CXXIX by treating the compound of formula CXXVII with $BBr_3$ or $BCl_3$ using solvent for example dichloromethane, chloroform for 4 to 48 hours at the temperature from -72°C to 0°C. Any of the conditions conventional in such demethylation reactions can be utilized to carry out the reaction of step (24-4).

The compound of formula CXXIX can be converted to the compound of formula CXXX by esterification of compound of formula CXXIX with ethanol or propanol. The reaction can be carried out either by using catalysts for example $H_2SO_4$, TsOH and the like or by using dehydrating agent for example dicyclohexylcarbodiimide and the like. Any of the conditions conventional in such esterification reactions can be utilized to carry out the reaction of step (24-5).

The compound of formula CXXX can be converted to the compound of formula CXXXI by etherifying or alkylating the compound of formula CXXX with alkyl halide having 2 to 3 carbon atoms by using suitable base for example $K_2CO_3$, $Cs_2CO_3$, NaH, pyridine, $Et_3N$ and the like. The reaction can be carried out in conventional solvents, such as tetrahydrofuran, dimethylformamide, and dichloromethane. The reaction is generally carried out at temperatures from 0°C to 60°C for 4 to 48 hours. Any of the conditions suitable in such alkylation reactions can be utilized to carry out the reaction of step (24-6).

The compound of formula CXXXI can be converted to the compound of formula CXXXII via reaction of step (24-7) where $R^5$ is alkoxy having 2 to 3 carbon atoms by deprotection of the hydroxy protecting group. The suitable deprotecting conditions can be described in the Protective Groups in Organic Synthesis by T Greene and hydrolyzing the ester group by using aqueous sodium or lithium hydroxide to give carboxylic group.

The compound of formula CXXXII is the compound of formula LXXI where $R^5$ is alkoxy having 2 to 3 carbon atoms and $R^{12}$ is H.

The product of each step can be isolated and purified by techniques such as extraction, evaporation, chromatography, and recrystallization.

Reaction Scheme 24

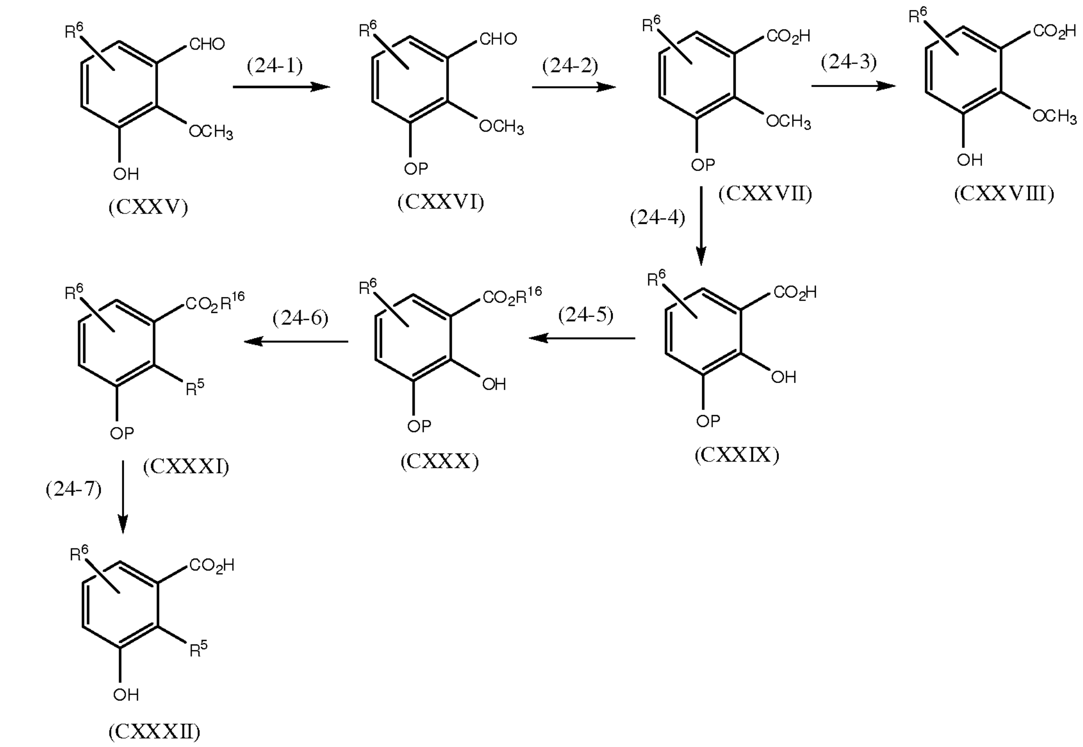


(CXXV) (CXXVI) (CXXVII) (CXXVIII) (CXXIX) (CXXX) (CXXXI) (CXXXII)

The compound of formula LXXI where one of $R^5$ and $R^6$ is hydrogen and the other is alkoxy having from 1 to 3 carbon atoms, i.e. compounds of formula:

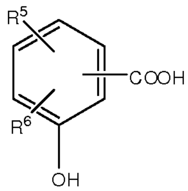

(LXXI)

are either commercially available or can be prepared according to the methods described in the literature as follows:

1. 2-OMe-4-$OHC_6H_3CO_2H$
US 2001034343 or WO 9725992.
2. 5-OMe-3-$OHC_6H_3CO_2H$
J.O.C (2001), 66(23), 7883-88.
3. 2-OMe-5-$OHC_6H_3CO_2H$
US 6194406 (Page 96) and Journal of the American Chemical Society (1985), 107(8), 2571-3.
4. 3-OEt-5-$OHC_6H_3CO_2H$
Taiwan Kexue (1996), 49(1), 51-56.
5. 4-OEt-3-$OHC_6H_3CO_2H$
WO 9626176
6. 2-OEt-4-$OHC_6H_3CO_2H$
Takeda Kenkyusho Nempo (1965), 24,221-8.
JP 07070025.
7. 3-OEt-4-$OHC_6H_3CO_2H$
WO 9626176.
8. 3-OPr-2-$OHC_6H_3CO_2H$
JP 07206658, DE 2749518.

9. 4-OPr-2-OH$C_6H_3CO_2H$
Farmacia (Bucharest) (1970), 18(8), 461-6.
JP 08119959.
10. 2-OPr-5-OH$C_6H_3CO_2H$ and 2-OEt-5-OH$C_6H_3CO_2H$
Adapt synthesis from US 6194406 (Page 96) by using propyl iodide and ethyl iodide.
11. 4--OPr-3-OH$C_6H_3CO_2H$
Adapt synthesis from WO 9626176
12. 2-OPr-4-OH$C_6H_3CO_2H$
Adapt synthesis from Takeda Kenkyusho Nempo (1965), 24,221-8 by using propyl halide.
13. 4-OEt-3-OH$C_6H_3CO_2H$
Biomedical Mass Spectrometry (1985), 12(4), 163-9.
14. 3-OPr-5-OH$C_6H_3CO_2H$
Adapt synthesis from Taiwan Kexue (1996), 49(1), 51-56 by using propyl halide.

The compound of formula LXXI where one of $R^5$ and $R^6$ is hydrogen and the other is alkyl having from 1 to 3 carbon atoms, i.e. compounds of formula:

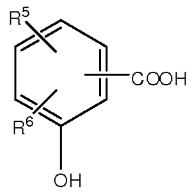

(LXXI)

are either commercially available or can be prepared according to the methods described in the literature as follows:

1. 5-Me-3-OH$C_6H_3CO_2H$ and 2-Me-5-OH$C_6H_3CO_2H$ WO 9619437.
J.O.C. 2001, 66, 7883-88.
2. 2-Me-4-OH$C_6H_3CO_2H$
WO 8503701.
3. 3-Et-2-OH$C_6H_3CO_2H$ and 5-Et-2-OH$C_6H_3CO_2H$ J. Med. Chem. (1971), 14(3), 265.
4. 4-Et-2-OH$C_6H_3CO_2H$
Yaoxue Xuebao (1998), 33(1), 67-71.
5. 2-Et-6-OH$C_6H_3CO_2H$ and 2-n-Pr-6-OH$C_6H_3CO_2H$
J. Chem. Soc., Perkin Trans 1 (1979), (8), 2069-78.
6. 2-Et-3-OH$C_6H_3CO_2H$
JP 10087489 and WO 9628423.
7. 4-Et-3-OH$C_6H_3CO_2H$
J.O.C. 2001, 66, 7883-88.
WO 9504046.
8. 2-Et-5-OH$C_6H_3CO_2H$
J.A.C.S. (1974), 96(7), 2121-9.
9. 2-Et-4-OH$C_6H_3CO_2H$ and 3-Et-4-OH$C_6H_3CO_2H$ JP 04282345.
10. 3-n-Pr-2-OH$C_6H_3CO_2H$
J.O.C. (1991), 56(14), 4525-29.
11. 4-n-Pr-2-OH$C_6H_3CO_2H$
EP 279630.
12. 5-n-Pr-2-OH$C_6H_3CO_2H$
J. Med. Chem. (1981), 24(10), 1245-49.
13. 2-n-Pr-3-OH$C_6H_3CO_2H$
WO 9509843 and WO 9628423.
14. 4-n-Pr-3-OH$C_6H_3CO_2H$
WO 9504046.
15. 2-n-Pr-5-OH$C_6H_3CO_2H$

Synthesis can be adapted from J.A.C.S (1974), 96(7), 2121-9 by using ethyl alpha formylvalerate.

16. 3-n-Pr-4-$OHC_6H_3CO_2H$

Polymer (1991), 32(11) 2096-105.
17. 2-n-Pr-4-$OHC_6H_3CO_2H$
3-Propylphenol can be methylated to 3-Propylanisole, which was then formylated to 4-Methoxy-3-benzaldehyde. The aldehyde can be oxidized by Jones's reagent to give corresponding acid and deprotection of methyl group by $BBr_3$ will give the title compound.

18. 1.3-Et-5-$OHC_6H_3CO_2H$ and 3-Pr-n-5-$OHC_6H_3CO_2H$

Adapt synthesis from J.O.C. 2001, 66, 7883-88 by using 2-Ethylacrolein and 2-Propylacrolein.

The compound of formula I where q is 0 or 1, t is 0 or 1, and r is 0, 1 or 2, y is 0, 1, 2 or 3, one of $R^5$ and $R^6$ is hydrogen, and the other is selected from the group consisting of hydroxy. X is C(O), r is 0 and t is 0; X is $N(R^{13})$ wherein $R^{13}$ is hydrogen or alkyl having from 1 to 3 carbon atoms, i.e. compounds of formula:

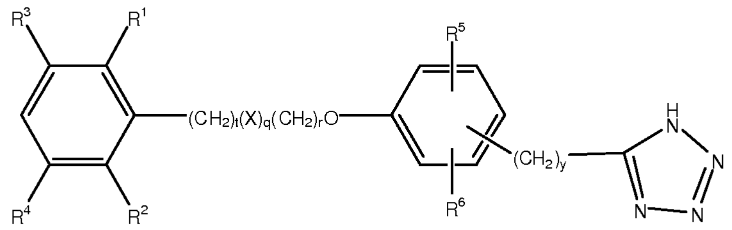

(I)

wherein $R^1$, $R^2$, $R^3$ and $R^4$ are described as above, can be prepared via reaction scheme of Scheme 25.
In the reaction scheme of Scheme 25,

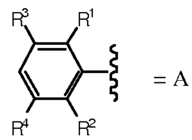

and q, t, r, y, and $R^6$, are as above.
The compound of formula LXIX can be converted to the compound of formula CXXXIII via reaction of step (25-1) in the same manner as described hereinbefore in connection with the reaction of step (1-4). Any of the conditions conventional in such demethylation reactions can be utilized to carry out the reaction of step (25-1).
The compound of formula CXXXIII can be converted to the compound of formula CXXXIV where $R^5$ is hydroxy via reaction of step (25-2) in the same manner as described hereinbefore in the reaction of step (13-2).
The compound of formula CXXXIV is the compound of formula I where one of $R^5$ and $R^6$ is hydrogen and the other is hydroxy.
The conventional techniques such as extraction, evaporation, chromatography and recrystallization can be utilized to purify the product of the all steps.
If A is phenyl substituted by hydroxy or amino groups, it is generally preferred to protect those groups by utilizing suitable protecting groups, which are known in the art. The suitable protecting group can be described in the Protective Groups in Organic Synthesis by T. Greene.
The protecting group can be deprotected by utilizing suitable deprotecting reagents such as those described in Protective Groups in Organic Synthesis by T. Greene.

Reaction Scheme 25

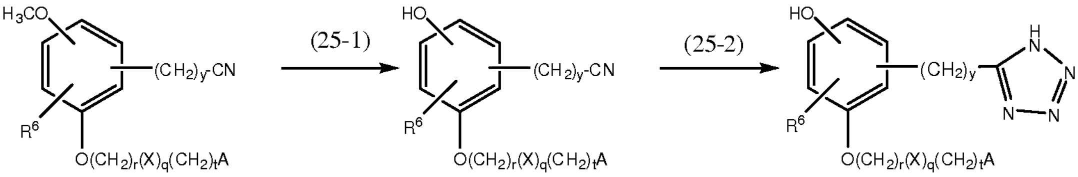


(LXIX) (CXXXIII) (CXXXIV)

The compound of formula I where q is 0 or 1, t is 0 or 1, and r is 0, 1 or 2, y is 0, 1, 2 or 3, one of $R^5$ and $R^6$ is hydrogen, and the other is selected from the group consisting of amino. X is C(O), r is 0 and t is 0; X is $N(R^{13})$ wherein $R^{13}$ is hydrogen or alkyl having from 1 to 3 carbon atoms, i.e. compounds of formula:

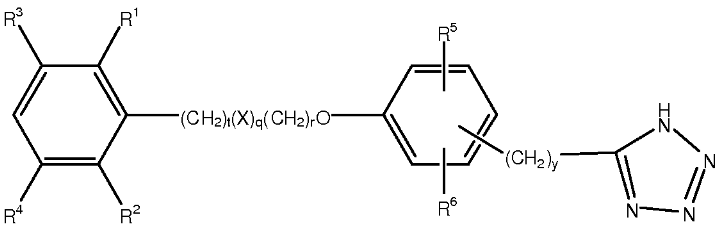

(I)

wherein $R^1$, $R^2$, $R^3$ and $R^4$ are described as above, can be prepared via reaction scheme of Scheme 26.

In the reaction scheme of Scheme 26,

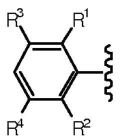 = A

and q, t, r, y, and $R^6$, are as above.

The compound of formula CXXXV can be converted to the compound of formula CXXXVI via the reaction of step (26-1) in the same manner as described hereinbefore in the reaction of step (13-1).

The compound of CXXXVI can be converted to the compound of formula CXXXVII via reaction of step (26-2) by reducing nitro to amine. The reducing agents can be metals for example Zn, Sn, Fe and the like and acid. The nitro group can also be reduced by catalytic hydrogenation to give amine. Any of the conditions conventional in such reduction of nitro to amino group can be utilized to carry out the reaction of step (26-2).

The compound of formula CXXXVII can be converted to the compound of formula CXXXVIII via reaction of step (26-3) in the same manner as described hereinbefore in the reaction of step (13-2).

The compound of formula CXXXVIII is the compound of formula I where one of $R^5$ and $R^6$ is hydrogen and other is amino.

The conventional techniques such as extraction, evaporation, chromatography and recrystallization can be utilized to purify the product of the all steps.

If A is phenyl substituted by hydroxy or amino groups, it is generally preferred to protect those groups by utilizing suitable protecting groups, which are known in the art. The suitable protecting group can be described in the Protective Groups in Organic Synthesis by T. Greene.

The protecting group can be deprotected by utilizing suitable deprotecting reagents such as those described in Protective Groups in Organic Synthesis by T. Greene.

Reaction Scheme 26

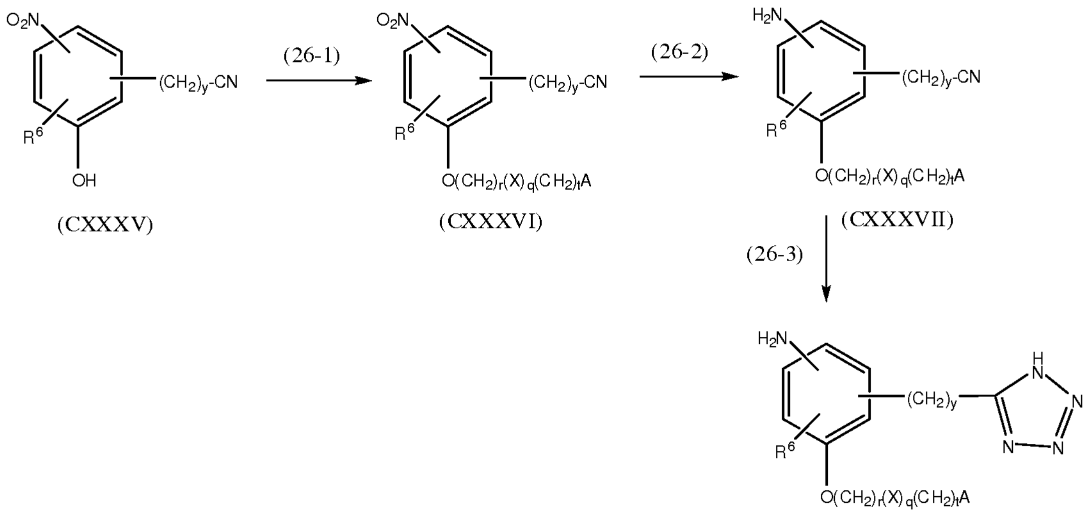


(CXXXV) (CXXXVI) (CXXXVII) (CXXXVIII)

The compound of formula LXXI where one of $R^5$ and $R^6$ is hydrogen and other is amino i.e. compounds of formula:

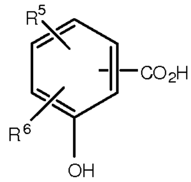

(LXXI)

wherein $R^5$ and $R^6$ are described as above, can be synthesized via the reaction of Scheme 27.

In the reaction scheme of Scheme 27, P is a protecting group.

The compound of formula CXXXIX can be converted to the compound of formula CXL via reaction of step (27-1) in the same manner as described hereinbefore in the reaction of step (14-1).

The compound of formula CXL can be converted to the compound of formula CXLI via reaction of step (27-2) in the same manner as described hereinbefore in the reaction of step (26-2).

The compound of formula CXLI can be converted to the compound of formula CXLII via reaction of scheme (27-3) in the same manner as described hereinbefore in the reactions of demethylation via the step (1-4) and by ester hydrolysis via the step (1-6) to produce phenol and carboxylic acid.

The compound of formula CXLII is the compound of formula LXXI where one of $R^5$ and $R^6$ is hydrogen and other is amino.

The conventional techniques such as extraction, evaporation, chromatography and recrystallization can be utilized to purify the product of the all steps.

If one of $R^5$ and $R^6$ is substituted by amino groups, it is generally preferred to protect those groups by utilizing suitable protecting groups, which are known in the art. The suitable protecting group can be described in the Protective Groups in Organic Synthesis by T. Greene.

Reaction Scheme 27

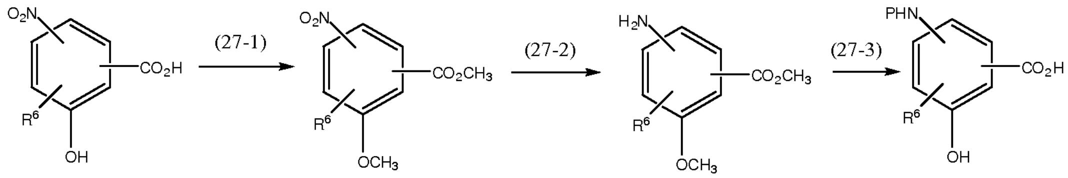


(CXXXIX) (CXL) (CXLI) (CXLII)

The compound of formula LXXI where one of $R^5$ and $R^6$ is hydrogen and other is hydroxy i.e. compounds of formula:

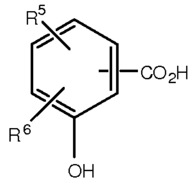

(LXXI)

wherein $R^5$ and $R^6$ are described as above, can be synthesized via the reaction of Scheme 28.

In the reaction scheme of Scheme 28, P is a protecting group.

The compound of formula CXLI can be converted to the compound of formula CXLIV via reaction of step (28-1) in the same manner as described hereinbefore in the reactions of step (18-4).

The compound of formula CXLIV can be converted to the compound of formula CXLV via reaction of step (28-2) by protecting phenol by suitable protecting group. The suitable protecting group can be described in the Protective Groups in Organic Synthesis by T. Greene.

The compound of formula CXLV can be converted to the compound of formula CXLVI via reaction of step (28-3) by ester hydrolysis to produce carboxylic acid in the same manner as described hereinbefore in the reaction of step (1-6) and then by demethylation in the same manner as described hereinbefore in the reaction of step (1-4).

The compound of formula CXLVI is the compound of formula LXXI where one of $R^5$ and $R^6$ is hydrogen and other is hydroxy.

The conventional techniques such as extraction, evaporation, chromatography and recrystallization can be utilized to purify the product of the all steps.

If one of $R^5$ and $R^6$ is substituted by hydroxy, it is generally preferred to protect those groups by utilizing suitable protecting groups, which are known in the art. The suitable protecting group can be described in the Protective Groups in Organic Synthesis by T. Greene.

Reaction Scheme 28

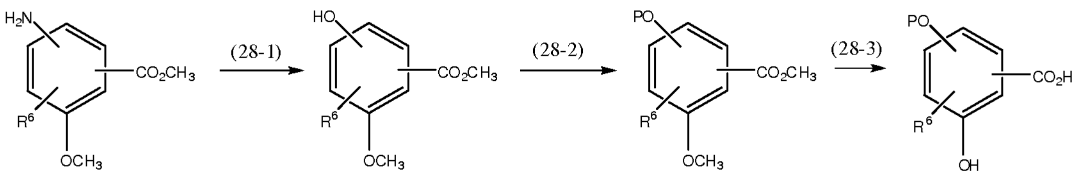


(CXLI) (CXLIV) (CXLV) (CXLVI)

The compound of formula LXXXVII where one of $R^5$ and $R^6$ is hydrogen, and the other is selected from the group consisting of hydrogen, amino, hydroxy, fluoro, chloro, bromo, alkoxy having from 1 to 2 carbon atoms or alkyl having from 1 to 2 carbon atoms, $R^{16}$ is alkyl group having 1 to 2 carbon atoms i.e. compounds of formula:

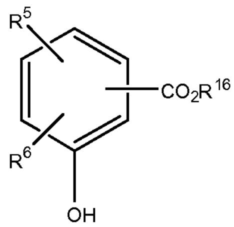

(LXXXVII)

wherein $R^5$, $R^6$ and $R^{16}$ are described as above, can be prepared by reaction scheme of Scheme 29.

The compound of formula LXXI can be converted to the compound of formula LXXXVII via reaction of step (29-1) by esterification of the compound of formula LXXI with CXLVII. The reaction can be carried out either by using catalysts for example $H_2SO_4$, TsOH and the like or by using dehydrating agent for example dicyclohexylcarbodiimide and the like. Any of the conditions conventional in such esterification reactions can be utilized to carry out the reaction of step (29-1).

Reaction Scheme 29

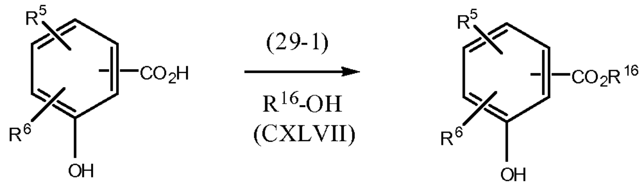


(LXXI) (LXXXVII)

USE IN METHODS OF TREATMENT

This invention provides a compound or salt of this invention, or a pharmaceutical composition of this invention, for use in reducing the uric acid concentration in blood of, or increasing uric acid excretion from, a mammalian subject; preferably wherein the compound or salt is administered orally. This invention also provides the use of a compound or salt of this invention or a pharmaceutical composition of this invention in the manufacture of a medicament for reducing the uric acid concentration in blood of, or increasing uric acid excretion from, a mammalian subject; preferably wherein the medicament is formulated for oral administration.

This disclosure provides a method for reducing uric acid levels in a mammalian subject or increasing uric acid excretion from a mammalian subject. The level of uric acid in a mammal can be determined using any conventional measure. Typically the level of uric acid in the blood is determined. Uric acid can also be deposited or precipitated in tissues, resulting in depots (e.g. tophi) that can be affected by raising or lowering blood uric acid concentrations, and which conversely can contribute to circulating uric acid. Reducing uric acid can be used to treat or prevent a variety of conditions including gout, hyperuricemia, elevated levels of uric acid that do not meet the levels customarily justifying a diagnosis of hyperuricemia, kidney stones, renal dysfunction, cardiovascular disease, cardiovascular risk factor, and cognitive impairment. By lowering uric acid levels, administration of the compounds of this invention slows progression of kidney disease. An elevated uric acid level has been identified as a risk factor for cardiovascular disease. A significant correlation has been shown between elevated uric acid and cognitive impairment in older adults. (Schretlen, D.J. et al., "Serum Uric Acid and Cognitive Function in Community-Dwelling Older Adults", Neuropsychology (Jan. 2007) 21(1): 136-140). Accordingly reducing uric acid can be used to treat or prevent cognitive impairment, including cognitive impairment in elderly adults. It is well known that people with Lesch-Nyhan Syndrome have elevated levels of uric acid and suffer the numerous consequences of this hyperuricemia, including gout. Thus, this invention for reducing blood levels and increasing elimination of uric acid can be used to treat people with Lesch-Nyhan Syndrome.

The normal range of uric acid in blood is between 3.4 mg/dL and 7.0 mg/dL in men, between 2.4 mg/dL and 6.0 mg/dL in premenopausal women, and from 2.5 mg/dL to 5.5 mg/dL in children. Urate crystal formation/precipitation typically occurs in men at levels of 6.6 mg/dL or higher and in women at levels of 6.0 mg/dL or higher. This illustrates that levels of uric acid that are within the so-called normal range can have undesirable health consequences, even producing gout. Also, what may be in the normal range for the population as a whole may be elevated for the individual. Cardiovascular and other consequences of elevated uric acid can occur with blood levels well within these "normal" ranges. Therefore, a diagnosis of hyperuricemia is not necessarily a prerequisite for the beneficial effects of the compounds of the invention.

This invention is useful in the treatment of hyperuricemia associated with gout, hypertension, vascular inflammation, heart failure, arterio-venous disorders, myocardial infarct, stroke, pre-eclampsia, eclampsia, sleep apnea, renal dysfunction (including renal failure, end stage renal disease [ESRD]), organ transplant, diuretics, thiazides, cyclosporine, aspirin, vitamin C, nicotinic acid, levodopa (L-DOPA), cytotosic drugs, and certain antibacterial agents (such as pyrozinamide), cirrhosis, thyroid dysfunction, parathyroid dysfunction, lung cancer, anemia, leukemia, lymphoma, multiple myeloma, tumor-lysis syndrome, thyroid or parathyroid dysfunction, Lesch-Nyhan Syndrome, smoking, alcohol consumption, and psoriasis. This invention includes the treatment of hyperuricemia that can lead to gout, formation of urate crystals, renal dysfunction, graft or organ failure following transplant, endothelial disorders (such as inflammation), chronic heart failure, arterio-venous disorders, pre-eclampsia, eclampsia, hypertension, and cognitive impairment. In embodiments relating to the treatment of gout, tissue deposits of uric acid, including but not limited to tophi, are reduced, and the incidence and severity of gout flares are also reduced.

The compounds of this invention can be administered by any conventional route of systemic administration. Preferably they are administered orally. Accordingly, it is preferred for the medicament to be formulated for oral administration. Other routes of administration that can be used in accordance with this invention include rectally, parenterally, by injection (e.g. intravenous, subcutaneous, intramuscular or intraperitioneal injection), or nasally.

Further embodiments of each of the uses and methods of treatment comprise administering any one of the embodiments of the compounds described above. In the interest of avoiding unnecessary redundancy, each such compound and group of compounds is not being repeated, but they are incorporated into this description of uses and methods of treatment as if they were repeated.

Both human and non-human mammalian subjects can be treated in accordance with the invention. The optimal dose of a particular compound of the invention for a particular subject can be determined in the clinical setting by a skilled clinician. In the case of oral administration the compound of this invention is generally administered to adults in a daily dose of from 1 mg to 2500 mg, more preferably from 1 mg to 1200 mg. In other embodiments of this invention the compound is administered in a dose of from 400 mg to 1000 mg, from 600 mg to 800 mg, from 600 mg to 1000 mg, or from 100 to 300 mg, administered once or twice per day. The average body weight of a typical adult is 60 to 70 kilograms, so that appropriate dose ranges expressed as mg/kg are approximately from 0.015 to 42 mg/kg, from 0.015 to 20 mg/kg, from 6.6 to 13 mg/kg, from 10 to 13 mg/kg, from 10 to 16 mg/kg, or from 1.67 to 4.3 mg/kg, administered once or twice per day. When treating children the optimal dose is determined by the patient's physician. In the case of oral administration to a mouse the compound of this invention is generally administered in a daily dose from 1 to 300 mg of the compound per kilogram of body weight.

The compound of this invention can be administered in combination with other uric acid lowering drugs. In such cases the dose of the compound of this invention is as described above. Any conventional or investigational uric acid lowering drug can be utilized in combination with the compound of this invention. Examples of such drugs include xanthine oxidase inhibitors such as allopurinol (from 100 mg/day to 1000 mg/day; more typically from 100 mg/day to 300 mg/day) febuxostat (from 40 mg/day to 120 mg/day; more specifically from 60 mg/day to 80 mg/day) and oxypurinol; Puricase / PEG-uricase (from 4 mg to 12 mg every two weeks by infusion); uricosuric agents such as sulfinpyrazone (from 100 mg/day to 800 mg/day), probenecid (500 mg/day), losartan (from 25 mg/day to 200 mg/day, more typically from 50 mg/day to 100 mg/day), fenofibrate, JTT-552 (a URAT-1 inhibitor), benzbromarone (from 70mg/day to 150 mg/day), and statins such as atorvastatin (LIPITOR®). The other uric acid lowering drug can be administered in its usual amount or in an amount that is less than the usual amount, whether by administering lower doses of such other drug or by less frequent dosing with such other drug.

The compounds of this invention can be administered together with other drugs used to decrease the pain associated with gouty attacks, for example nonsteroidal antiinflammatory drugs (NSAIDs), colchicine, corticosteroids, and other analgesics.

In the course of lowering uric acid levels in the blood it is expected that the compounds of this invention will increase the levels of uric acid in the urine. To increase the pH of the urine and thereby improve solubility of the uric acid, citrate or bicarbonate, for example, can be administered in conjunction with the compound of this invention.

An admixture of the compound or salt of this invention with one or more other uric acid lowering drugs, analgesics, and pH increasing agents, can be administered to the subject. Alternatively the compound or salt of this invention and the one or more other uric acid lowering drugs, analgesics, and pH increasing agents are not mixed together to form an admixture but are administered independently to the subject. When the active ingredients are not mixed together to form a single admixture or composition it is convenient to provide them in the form of a kit comprising one or more unit oral doses of a compound of this invention, one or more unit oral doses of one or more other uric acid lowering drugs, analgesics, and pH increasing agents, and instructions for administering the compound of this invention in combination with the other active ingredients. Preferably the components of the kit are packaged together, such as in a box or a blister pack.

PHARMACEUTICAL COMPOSITIONS

This invention provides a pharmaceutical composition comprising a compound of this invention, and a pharmaceutically acceptable carrier. Further embodiments of the pharmaceutical composition of this invention comprise any one of the embodiments of the compounds described above. In the interest of avoiding unnecessary redundancy, each such compound and group of compounds is not being repeated, but they are incorporated into this description of pharmaceutical compositions as if they were repeated.

Preferably the composition is adapted for oral administration, e.g. in the form of a tablet, coated tablet, dragee, hard or soft gelatin capsule, solution, emulsion or suspension. In general the oral composition will comprise from 1 mg to 2500 mg, more preferably from 1 mg to 1200 mg of the compound of this invention. In more specific embodiments of this invention the oral composition will comprise from 400 mg to 1000 mg, from 600 mg to 800 mg, from 600 mg to 1000 mg, or from 100 to 300 mg, of the compound of this invention. It is convenient for the subject to swallow one or two tablets, coated tablets, dragees, or gelatin capsules per day. However the composition can also be adapted for administration by any other conventional means of systemic administration including rectally, e.g. in the form of suppositories, parenterally, e.g. in the form of injection solutions, or nasally.

The active ingredients can be processed with pharmaceutically inert, inorganic or organic carriers for the production of pharmaceutical compositions. Lactose, corn starch or derivatives thereof, talc, stearic acid or its salts and the like can be used, for example, as such carriers for tablets, coated tablets, dragees and hard gelatin capsules. Suitable carriers for soft gelatin capsules are, for example, vegetable oils, waxes, fats, semi-solid and liquid polyols and the like. Depending on the nature of the active ingredient no carriers are, however, usually required in the case of soft gelatin capsules, other than the soft gelatin itself. Suitable carriers for the production of solutions and syrups are, for example, water, polyols, glycerol, vegetable oils and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, coating agents or antioxidants.

The invention will be better understood by reference to the following examples, which illustrate but do not limit the invention described herein.

EXAMPLES

EXAMPLE 1: Synthesis of Compound EJ

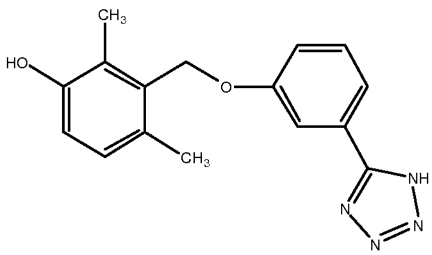

3-((3-(1*H*-tetrazol-5-yl)phenoxy)methyl)-2,4-dimethylphenol

Step A: Preparation of 3-(3-(*tert*-butyldimethylsilyloxy)-2,6-dimethylbenzyloxy)benzonitrile:

A solution of (3-(*tert*-butyldimethylsilyloxy)-2,6-dimethylphenyl)methanol (1.40g, 5.2mmol) and diisopropyl azodicarboxylate (DIAD, 1.16g, 5.7mmol) in dry THF (15ml) was added drop wise to a solution of 3-hydroxybenzonitrile (.688g, 5.7mmol) and triphenylphosphine (1.50g, 5.7mmol) in THF (30ml) at 0°C. The reaction mixture was stirred at room temperature for 4 hours or until starting material is consumed, diluted with ethyl acetate and washed with water and brine. The organic layer was dried over $Na_2SO_4$, filtered, concentrated, and purified by flash chromatography on a silica gel column (hex: ethyl acetate, 4:1) to give the title compound.
$^1$H NMR (400 MHz, $CDCl_3$): .21 (s, 3H); 1.01 (s, 9H); 2.23 (s, 3H); 2.31 (s, 3H); 5.0 (s, 2H); 6.74-6.76 (d, 1H);

6.9 (d, 1H); 7.26-7.29 (m, 3H); 7.4 (t, 1H).

Step B: Preparation of 3-((3-(1*H*-tetrazol-5-yl)phenoxy)methyl)-2,4-dimethylphenol:

A mixture of 3-(3-(*tert*-butyldimethylsilyloxy)-2,6-dimethylbenzyloxy)benzonitrile (Step A, 1.88g, 5.11mmol), sodium azide (.432g, 6.64mmol), and ammonium chloride (.355g, 6.64mmol) in dry DMF (20ml) was heated under argon at 90°C for 16 hours or until all the starting material is consumed, the reaction mixture was cooled, diluted with water and extracted with ethyl acetate (4X). The combined organic layer was washed with brine, dried over $Na_2SO_4$, filtered, concentrated, and purified by flash chromatography on a silica gel column (chloroform: methanol, 8:2) to give the title compound.
$^1H$ NMR (400 MHz, $d_6$-DMSO): 2.13 (s, 3H); 2.22 (s, 3H); 5.07 (s, 2H); 5.78 (s, 1H); 6.74-6.76 (d, 1H); 6.9 (d, 1H); 7.26-7.29 (d, 1H); 7.4 (t, 1H) 7.8 (m, 2H); 9.14 (s, 1H).

EXAMPLE 2: Synthesis of Compound EK

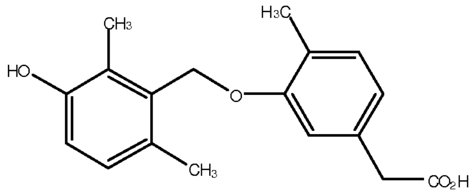

2-(3-(3-hydroxy-2,6-dimethylbenzyloxy)-4-methylphenyl)acetic acid

Step A: Preparation of 2-(3-methoxy-4-methylphenyl)acetic acid:

To a stirred solution of 2-(3-methoxy-4-methylphenyl)acetonitrile (5g, 31mmol) in abs ethanol (25ml) was added 2M NaOH (20ml) at room temperature and reaction mixture was refluxed for 16 hours or until starting material is consumed. The reaction mixture was concentrated, diluted in chloroform and pH was adjusted to 4 by addition of 1N HCl. The organic layer was washed with brine, dried over $Na_2SO_4$, filtered, concentrated, to give off white solid. The solid was washed with hexane, filtered, dried under vacuum and purified by flash chromatography on a silica gel column (chloroform: methanol, 95:5) to give the title compound.
$^1H$ NMR (400 MHz, $CDCl_3$): 2.19 (s, 3H); 3.62 (s, 2H); 3.82 (s, 3H); 6.74 (m, 2H); 7.14 (d, 1H).

Step B: Preparation of Ethyl 2-(3-methoxy-4-methylphenyl)acetate:

To a stirred solution of 2-(3-methoxy-4-methylphenyl)acetic acid (Step A, 4.64g, 25.7mmol) in ethanol (100ml) was added p-TsOH (.7g, 3.7mmol) at room temperature under argon and the reaction mixture was refluxed for 12 hours or until all the starting material is consumed, concentrated, diluted in EtOAc and washed with 1N HCl, brine, dried over $Na_2SO_4$, filtered, concentrated and purified by flash chromatography on a silica gel column (hexane: ethyl acetate, 2:1) to give the title compound.

$^1H$ NMR (400 MHz, $CDCl_3$): 1.25 (t, 3H); 2.10 (s, 3H); 3.57 (s, 2H); 3.82 (s, 3H); 4.14 (q, 2H); 6.76 (m, 2H); 7.14 (d, 1H).

Step C: Preparation of Ethyl 2-(3-hydroxy-4-methylphenyl)acetate:

To a stirred solution of Ethyl 2-(3-methoxy-4-methylphenyl)acetate (Step B, 4.12g, 19.8mmol) in methylene chloride (30ml) was added dropwise a solution of $BBr_3$ (1M in $CH_2Cl_2$, 25ml) at -78°C under argon, the cold bath was substituted by ice bath after 30 minutes and the reaction mixture was stirred at the same temperature for 2 hours and then 30 minutes at room temperature. The reaction mixture was quenched by addition of ice and worked up by washing with water and brine. The organic layer was dried over $Na_2SO_4$, filtered, concentrated, and purified by flash chromatography on a silica gel column (hexane: ethyl acetate, 2:1) to give the title compound as oil.

$^1H$ NMR (400 MHz, $CDCl_3$): 1.23 (t, 3H); 2.21 (s, 3H); 3.52 (s, 2H); 4.13 (q, 2H); 6.72 (m, 2H); 7.14 (d, 1H).

Step D: Preparation of 2,6-dimethyl-3-nitrobenzoic acid:

To a stirred solution of 2,6-dimethylbenzoic acid (15g, 99.8mmol) in nitromethane (150 ml) was added dropwise 70% $HNO_3$ (18.9ml) at 0°C followed by $H_2SO_4$ (14ml). The reaction mixture was stirred at room temperature for 21 hours, concentrated, and then diluted with ethyl acetate, washed with water (3X), dried over $Na_2SO_4$, filtered, concentrated, and purified by flash chromatography on a silica gel column (chloroform: methanol, 92.5:7.5) to give the title compound as a solid.
$^1H$ NMR (400 MHz, $d_6$-DMSO): 2.33 (s, 3H); 2.37 (s, 3H); 7.34-7.36 (d, 1H); 7.86-7.88 (d, 1H).

Step E: Preparation of methyl 2,6-dimethyl-3-nitrobenzoate:

To a stirred solution of 2,6-dimethyl-3-nitrobenzoic acid (Step D, 10g, 51.3mmol), and $K_2CO_3$ (14.16g, 102mmol) in dry DMF (50ml) was added iodomethane (33g, 231mmol) at 0°C under argon. The reaction mixture was stirred at ambient temperature for 16 hours, diluted with ethyl acetate, washed with water (2X), and brine. The organic layer was dried over $Na_2SO_4$, filtered, concentrated, and purified by flash chromatography on a silica gel column (hexane: ethyl acetate, 2:1) to give the title compound.
$^1H$ NMR (400 MHz, $d_6$-DMSO): 2.28 (s, 3H); 2.32 (s, 3H); 3.83 (s, 3H); 7.34-7.36 (d, 1H); 7.91-7.93 (d, 1H).

Step F: Preparation of methyl 3-amino-2,6-dimethylbenzoate:

To a stirred solution of methyl 2,6-dimethyl-3-nitrobenzoate (Step E, 11g, 52.6mmol) in abs ethanol (100ml) was added tin dichloride dihydrate (59.34g, 263mmol), and the reaction mixture was heated at 80°C for 2 hours or until all the starting material is consumed. The reaction mixture was cooled, and pH of reaction adjusted to 5 by addition of 5N NaOH. The solids were filtered and washed with ethyl acetate (3X). The organic layer was washed with water, brine, dried over $Na_2SO_4$, filtered, concentrated, and purified by flash chromatography on a silica gel column (chloroform: methanol, 95:5) to give the title compound as solid.
$^1H$ NMR (400 MHz, $CDCl_3$): 2.08 (s, 3H); 2.19 (s, 3H); 3.90 (s, 3H); 6.67-6.69 (d, 1H); 6.84-6.94 (d, 1H).

Step G: Preparation of methyl 3-hydroxy-2,6-dimethylbenzoate:

The solution of methyl 3-amino-2,6-dimethylbenzoate (Step F, 6.82g, 38mmol) in conc $H_2SO_4$ (10.33ml) and water (38ml) was heated to a homogenous solution, cooled to 0°C and water (96ml) was added. To the reaction mixture was added drop wise a solution of $NaNO_2$ (2.68g, 38.8mmol) in water (6ml) at 0°C and then mixture was allowed to stir at the same temperature for 30 min.
The above diazonium reaction mixture was added drop wise to a heated solution of conc $H_2SO_4$ (40ml) and water (60ml) and reaction mixture was heated to 120°C for another 20-25 min, cooled to room temperature, and then extracted with ethyl acetate (4X). The combined organic layer was washed with brine, dried over $Na_2SO_4$, filtered, concentrated, and purified by flash chromatography on a silica gel column (hexane: ethyl acetate, 2:1) to give the title compound as an oil.
$^1H$ NMR (400 MHz, $d_6$-DMSO): 1.96 (s, 3H); 2.07 (s, 3H); 3.80 (s, 3H); 6.73-6.75 (d, 1H); 6.84-6.86 (d, 1H); 9.38 (s, 1H).

Step H: Preparation of methyl 3-(*tert*-butyldimethylsilyloxy)-2,6-dimethylbenzoate:

To a stirred solution of methyl 3-hydroxy-2,6-dimethylbenzoate (Step G, 1.55g, 8.6mmol) and imidazole (.644g, 9.4mmol) in dry DMF was added *tert*-Butyldimethylsilyl chloride (1.414g, 9.4mmol) at 0°C. The reaction mixture was stirred at the same temperature for 4 hours or until all the starting material is consumed, diluted with ethyl acetate (100ml) and washed with water (2X), brine, dried over $Na_2SO_4$, filtered, concentrated, and purified by flash chromatography on a silica gel column (hexane: ether, 5:1) to give the title compound.
$^1H$ NMR (400 MHz, $d_6$-DMSO): .17 (s, 6H); .95 (s, 9H); 2.02 (s, 3H); 2.11 (s, 3H); 3.81 (s, 3H); 6.73-6.75 (d, 1H); 6.84-6.86 (d, 1H).

Step I: Preparation of (3-(*tert*-butyldimethylsilyloxy)-2,6-dimethylphenyl)methanol:

To a stirred solution of methyl 3-(*tert*-butyldimethylsilyloxy)-2,6-dimethylbenzoate (Step H, 1.63g, 5.5mmol) in dry THF (15ml) was added drop wise a solution of $LiAlH_4$ (1M in THF, 6.1ml) at 0°C. After 4 hours of stirring at

the same temperature, another portion of $LiAlH_4$ (1.5ml) was added. The reaction mixture was further stirred for 1 hour, quenched slowly with ice-cold water and filtered. The resulting solids were washed with ethyl acetate (3X) and combined organic layer was washed with brine, dried over $Na_2SO_4$, filtered, concentrated, and purified by flash chromatography on a silica gel column (hexane: ethyl acetate, 2:1) to give the title compound.
$^1H$ NMR (400 MHz, $d_6$-DMSO): .14 (s, 6H); .95 (s, 9H); 2.17 (s, 3H); 2.24 (s, 3H); 4.4 (s, 2H); 4.6 (t, 1H); 6.6 (d, 1H); 6.8 (d, 1H).

Step J: Preparation of ethyl 2-(3-(3-(*tert*-butyldimethylsilyloxy)-2,6-dimethylbenzyloxy)-4-methylphenyl)acetate:

A solution of (3-(*tert*-butyldimethylsilyloxy)-2,6-dimethylphenyl)methanol (Step I, 1.86g, 7mmol) and diisopropyl azodicarboxylate (DIAD, 1.54g, 7.6mmol) in THF (10ml) was added drop wise to a solution of Ethyl 2-(3-hydroxy-4-methylphenyl)acetate (Step C, 1.47g, 7.6mmol) and triphenylphosphine (1.99g, 7.6mmol) in THF (30ml) at 0°C. The reaction mixture was stirred at room temperature for 4 hours, diluted with ether and washed with water and brine. The organic layer was dried over $Na_2SO_4$, filtered, concentrated, and purified by flash chromatography on a silica gel column (hex: ethyl acetate, 2:1) to give the title compound.
$^1H$ NMR (400 MHz, $CDCl_3$): .21 (s, 6H); 1.0 (s, 9H); 1.27 (t, 3H); 2.12 (s, 3H); 2.24 (s, 3H); 2.32 (s, 3H); 3.61 (s, 2H); 4.3 (q, 2H); 4.99 (s, 2H); 6.7 (d, 1H); 6.8 (d, 1H); 6.9 (m, 2H); 7.1 (d, 1H).

Step K: Preparation of ethyl 2-(3-(3-hydroxy-2,6-dimethylbenzyloxy)-4-methylphenyl)acetate:

To a stirred solution of ethyl 2-(3-(3-(*tert*-butyldimethylsilyloxy)-2,6-dimethylbenzyloxy)-4-methylphenyl)acetate (Step J, 2.24g, 5.0mmol) in dry THF (20ml) was added a solution of TBAF (1M in THF, 6ml) at 0°C, after 20 min of stirring at 0°C temperature, ice bath was removed and stirring continued until all the starting material is consumed. The reaction mixture was concentrated and purified by flash chromatography on a silica gel column (hex: ethyl acetate, 2:1) to give the title compound.
$^1H$ NMR (400 MHz, $CDCl_3$): 1.27 (t, 3H); 2.13 (s, 3H); 2.26 (s, 3H); 2.32 (s, 3H); 3.61 (s, 2H); 4.3 (q, 2H); 4.99 (s, 2H); 6.7 (d, 1H); 6.8 (d, 1H); 6.9 (m, 2H); 7.1 (d, 1H).

Step L: Preparation of 2-(3-(3-hydroxy-2,6-dimethylbenzyloxy)-4-methylphenyl)acetic acid:

To a stirred solution of ethyl 2-(3-(3-hydroxy-2,6-dimethylbenzyloxy)-4-methylphenyl)acetate (Step K, 1.34g, 4mmol) in absolute ethanol (35ml) was added 1N NaOH (15ml) at room temperature. The reaction mixture was stirred for 3 hours, or until all the starting material is consumed, concentrated and diluted with chloroform and acidified by 1M HCl to bring the pH to 3.5-4. The organic layer was washed with brine, dried over $Na_2SO_4$, filtered, concentrated and purified by flash chromatography on a silica gel column (chloroform: methanol, 95:5 spiked with acetic acid) to give the title compound as a white solid.
$^1H$ NMR (400 MHz, $d_6$-DMSO): 2.0 (s, 3H); 2.19 (s, 3H); 2.27 (s, 3H); 3.52 (s, 2H); 4.94 (s, 2H); 6.69-6.73 (m, 2H); 6.75-6.85 (m, 1H); 7.03-7.05 (m, 2H); 9.11 (s, 1H); 12.2 (s, 1H).

COMPARATIVE EXAMPLE 3: Synthesis of Compound EL

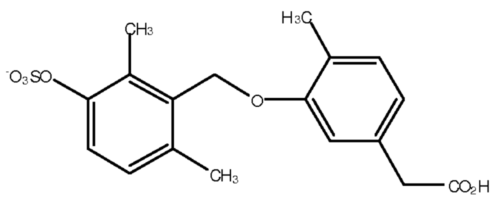

3-((5-(carboxymethyl)-2-methylphenoxy)methyl)-2,4-dimethylphenyl sulfate

Step A: Preparation of 3-((5-(carboxymethyl)-2-methylphenoxy)methyl)-2,4-dimethylphenyl sulfate:

To a stirred solution of 2-(3-(3-hydroxy-2,6-dimethylbenzyloxy)-4-methylphenyl)acetic acid (PN 2123) (.608g, 2.0mmol) in pyridine (20ml) was added $SO_3$.pyridine complex (.966g, 6.0mmol) at room temperature and the reaction mixture was stirred for 24 hours. To the reaction mixture was added solid $NaHCO_3$ (.850g, 10mmol) and stirring

continued for another 30 min. The reaction mixture was filtered, concentrated and purified by flash chromatography on a silica gel column (chloroform: methanol, 8:2 spiked with acetic acid) to give the title compound as a white solid. $^1$H NMR (400 MHz, $d_6$-DMSO): 2.0 (s, 3H); 2.11 (s, 3H); 2.21 (s, 3H); 3.46 (s, 2H); 4.95 (s, 2H); 6.73-6.75 (d, 1H); 6.95-7.0 (m, 3H); 7.21-7.24 (m, 1H); 12.2 (s, 1H).

EXAMPLE 4: Synthesis of Compound EM

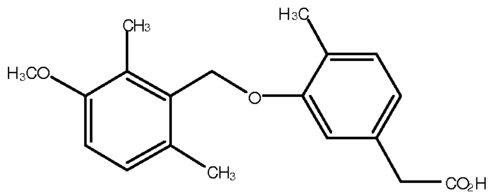

2-(3-(3-methoxy-2,6-dimethylbenzyloxy)-4-methylphenyl)acetic acid

Step A: Preparation of methyl 3-methoxy-2,6-dimethylbenzoate:

To a stirred solution of methyl 3-hydroxy-2,6-dimethylbenzoate (1.60g, 8.9mmol), and $K_2CO_3$ (2.45g, 17.7mmol) in dry DMF (10ml) was added iodomethane (5.04g, 35.5mmol) at 0°C under argon. The reaction mixture was stirred at ambient temperature for 16 hours, diluted with ethyl acetate, washed with water (2X), and brine. The organic layer was dried over $Na_2SO_4$, filtered, concentrated, and purified by flash chromatography on a silica gel column (hexane: ethyl acetate, 4:1) to give the title compound.
$^1$H NMR (400 MHz, $d_6$-DMSO): 2.15 (s, 3H); 2.22 (s, 3H); 3.80 (s, 3H); 3.90 (s, 3H); 6.78 (d, 1H); 7.0 (d, 1H).

Step B: Preparation of (3-methoxy-2,6-dimethylphenyl)methanol:

To a stirred solution of methyl 3-methoxy-2,6-dimethylbenzoate (Step A, 1.32g, 6.8mmol) in dry THF (15ml) was added drop wise a solution of $LiAlH_4$ (1M in THF, 8.16ml) at 0°C. After 4 hours of stirring at the same temperature, another portion of $LiAlH_4$ (2ml) was added. The reaction mixture was further stirred for 1 hour, quenched slowly with ice-cold water and filtered. The resulting solids were washed with ethyl acetate (3X) and organic layer was washed with brine, dried over $Na_2SO_4$, filtered, concentrated, and purified by flash chromatography on a silica gel column (hexane: ethyl acetate, 2:1) to give the title compound as solid.
$^1$H NMR (400 MHz, $CDCl_3$): 2.30 (s, 3H); 2.37 (s, 3H); 3.80 (s, 3H); 4.74 (s, 2H); 6.78 (d, 1H); 7.0 (d, 1H).

Step C: Preparation of ethyl 2-(3-(3-methoxy-2,6-dimethylbenzyloxy)-4-methylphenyl)acetate:

A solution of (3-methoxy-2,6-dimethylphenyl)methanol (Step B, .554g, 3.3mmol) and diisopropyl azodicarboxylate (DIAD, .673g, 3.3mmol) in dry THF (10ml) was added drop wise to a solution of Ethyl 2-(3-hydroxy-4-methylphenyl)acetate (.589g, 3.0mmol) and triphenylphosphine (.868g, 3.3mmol) in dry THF (20ml) at 0°C. The reaction mixture was stirred at room temperature for 4 hours, diluted with ether and washed with water and brine. The organic layer was dried over $Na_2SO_4$, filtered, concentrated, and purified by flash chromatography on a silica gel column (hex: ethyl acetate, 2:1) to give the title compound.
$^1$H NMR (400 MHz, $CDCl_3$): 1.27 (t, 3H); 2.12 (s, 3H); 2.26 (s, 3H); 2.34 (s, 3H); 3.61 (s, 2H); 3.82 (s, 3H); 4.17 (q, 2H); 5.0 (s, 2H); 6.7 (m, 1H); 6.8 (d, 1H); 6.9 (m, 2H); 7.1 (d, 1H).

Step D: Preparation of 2-(3-(3-methoxy-2,6-dimethylbenzyloxy)-4-methylphenyl)acetic acid:

To a stirred solution of ethyl 2-(3-(3-methoxy-2,6-dimethylbenzyloxy)-4-methylphenyl)acetate (Step C, .578g, 1.7mmol) in absolute ethanol (15ml) was added 1N NaOH (5ml) at room temperature. The reaction mixture was stirred for 3 hours, or until all the starting material is consumed, concentrated and diluted with chloroform and acidified by 1M HCl to bring the pH to 3.5-4. The organic layer was washed with brine, dried over $Na_2SO_4$, filtered, concentrated and purified by flash chromatography on a silica gel column (chloroform:

methanol, 95:5 spiked with acetic acid) to give the title compound as a white solid.
$^1$H NMR (400 MHz, $CDCl_3$): 2.13 (s, 3H); 2.25 (s, 3H); 2.39 (s, 3H); 3.67 (s, 2H); 3.82 (s, 3H); 5.0 (s, 2H); 6.8 (m, 2H); 6.94 (m, 1H); 7.1-7.15 (m, 2H).

EXAMPLE 5: Synthesis of Compound EN

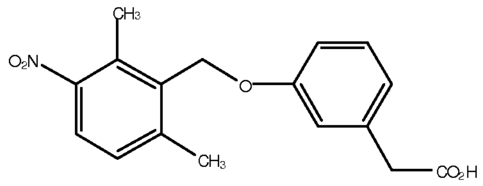

2-(3-(2,6-dimethyl-3-nitrobenzyloxy)phenyl)acetic acid

Step A: Preparation of (2,6-dimethyl-3-nitrophenyl)methanol:

To a stirred solution of 2,6-dimethyl-3-nitrobenzoic acid (2g, 10.2mmol) in dry THF (20ml) was added a solution of 1M $BH_3$ in THF (33ml). The reaction mixture was heated to 75°C for 4 hours or until all the starting material is consumed, then quenched very slowly with ice- cold water, concentrated and diluted with ethyl acetate. The organic layer was washed with 1M HCl, water, brine and dried over $Na_2SO_4$, filtered, concentrated and purified by flash chromatography on a silica gel column (chloroform: methanol, 95:5) to give the title compound as a white solid.
$^1$H NMR (400 MHz, $d_6$-DMSO): 2.40 (s, 3H); 2.42 (s, 3H); 4.54 (s, 2H); 7.21 (d, 1H); 7.65 (d, 1H).

Step B: Preparation of ethyl 2-(3-(2,6-dimethyl-3-nitrobenzyloxy)phenyl)acetate:

A solution of (2,6-dimethyl-3-nitrophenyl)methanol (Step A, .174g, .1mmol and diisopropyl azodicarboxylate (DIAD, .211g, 1mmol) in dry THF (5ml) was added drop wise to a solution of ethyl 2-(3-hydroxyphenyl)acetate (.190g, 10.5mmol) and triphenylphosphine (.274g, 10mmol in THF (10ml) at 0°C. The reaction mixture was stirred at room temperature for 4 hours, diluted with ether and washed with water and brine. The organic layer was dried over $Na_2SO_4$, filtered, concentrated, and purified by flash chromatography on a silica gel column (hex: ethyl acetate, 2:1) to give the title compound.

Step C: Preparation of 2-(3-(2,6-dimethyl-3-nitrobenzyloxy)phenyl)acetic acid:

To a stirred solution of ethyl 2-(3-(2,6-dimethyl-3-nitrobenzyloxy)phenyl)acetate (Step B, .180g, .5mmol) in absolute ethanol (15ml) was added 1N NaOH (5ml) at room temperature. The reaction mixture was stirred for 3 hours, or until all the starting material is consumed, concentrated and diluted with chloroform and acidified by 1M HCl to bring the pH to 3.5-4. The organic layer was washed with brine, dried over $Na_2SO_4$, filtered, concentrated and purified by flash chromatography on a silica gel column (chloroform: methanol, 95:5 spiked with acetic acid) to give the title compound as a white solid.
$^1$H NMR (400 MHz, $CDCl_3$): 2.47 (s, 3H); 2.50 (s, 3H); 3.65 (s, 2H); 5.0 (s, 2H); 6.94 (m, 3H); 7.18 (m, 1H); 7.25 (m, 1H); 7.70-7.73 (d, 1H).

EXAMPLE 6: URAT1 Inhibition Assay

URAT1 (Uric Acid Transporter 1) is expressed on the apical membrane in renal tubules. It mediates the reuptake of uric acid from the urine into the blood. Inhibition of URAT1 leads to increased excretion of uric acid in the urine, and is therefore a potential mode of action for drugs that lower serum uric acid concentrations. Probenecid and Benzbromarone, for example, have been used clinically for treatment of gout and hyperuricemia, and they both act on URAT1 to reduce uric acid reuptake. However, benzbromarone was withdrawn from the market due to liver toxicity via mechanisms independent of URAT1, and probenecid acts on numerous transporter proteins, resulting in interactions with a variety of other drugs.

An *in vitro* URAT1 assay is useful for identifying compounds with potential activity in lowering serum uric acid. A suitable assay involves transfection of cells (e.g. human embryonic kidney cells; "HEK") with a vector encoding human URAT1, followed by determination of the ability of transfected cells to take up radiolabeled uric acid. The activity of compounds as URAT1 inhibitors is evaluated by their ability to block uric acid uptake by transfected cells.

Test Compounds and Chemicals:

Benzbromarone (Sigma, Cat.No.B5774), Probenecid (Sigma, Cat.No.P8761)), DMSO (Sigma, Cat.No.D-2650), [8-$^{14}$C] Urate (50-60mCi/mmol; American Radio Chemicals, Cat. No. ARC0513).

Subcloning of hURAT1 into the expression vector:

Plasmid vector pCMV6-XL5 containing hURAT1 cDNA (Cat. No. SC125624) and the expression vector pCMV6-Neo (Cat. No.pCMVNEO) were obtained from OriGene Technologies, Inc. The full-length hURAT1 cDNA was obtained from the vector pCMV6-XL5 and subcloned into the expression vector pCMV6-Neo to create the hURAT1 expression plasmid pCMV6-hURAT1. The sequences were verified by automatic DNA sequencing.

Cell Culture, transfection of URAT1 expressing plasmids and the establishment of stably expressing HEK cells for hURAT1:

Human embryonic kidney 293 (HEK) cells (ATTCC, Cat No. CRL-1573) were cultured in EMEM supplemented with 10% FBS and 2mM L-glutamine and incubated at 37° C and 5% $CO_2$. For transfection experiments, cells were plated on 60 mm dishes in 1 ml media per dish. After an 18-24 hour incubation, cells were transfected with plasmid pCMV6-hURAT1 or the expression vector pCMV6-Neo, using the Lipofectin trasfection agent following the manufacturer's instructions (Invitrogen, Cat.No.18292). After transfection cells were grown in EMEM media for 72 hours and then by adding 1mg/ml Geneticin (GIBCO, Cat. No 10131) stable transfectants were selected. Stable transfectants expressing hURAT1 (herein after referred as hURAT1-HEK cells) or cells having only the expression vector pCMV6-Neo (herein after referred as mock-HEK cells) were verified using reverse transcription polymerase chain reaction (RT-PCR) methods.

[8-$^{14}$C] Urate Uptake Assay:

hURAT1-HEK cells and mock-HEK cells were plated in poly-D-Lysine Cell culture 24 well plates (Becton Dickinson, Cat. No.354414) at a concentration of 3X10$^5$ in EMEM medium and incubated overnight. Reaction solutions containing the [8-$^{14}$C] urate (55 mCi/mmol) at a final concentration of 50 $\mu$M were prepared with or without test compounds in Hanks' balanced salt solution (HBSS) containing 125 mM sodium gluconate, 4.8 mM potassium gluconate, 1.3 mM calcium, 5.6 mM glucose, 1.2 mM magnesium sulfate, 1.2 mM $KH_2PO_4$ and 25 mM HEPES (pH7.4). Before the uptake assay started, the culture medium was removed and the cells were incubated for 5 min in 0.6 ml of HBSS. After that HBSS was removed, the prepared reaction solutions were added into each well and incubated for 5 min at room temperature. Then the reaction solution was removed, cells were washed twice with 0.6 ml of cold HBSS and lysed with 0.2 ml of 0.1 M NaOH for 20 min. The cell lysates were transferred into the scintillation vials containing 1 ml of scintillation fluid (Opti Phase SuperMIX, PerkinElmer, Cat No. 1200-439) and the radioactivity was counted in the Microbeta counter (1450, Wallac Jet, PerkinElmer). Test compounds were dissolved in DMSO and the same concentration of DMSO was added into the wells of mock-HEK cells and the hURAT1-HEK cells that didn't contain test compounds. For each test compound, the uptake assay was performed 2 times and carried out in triplicate. Urate uptake of the cells for each test condition was presented as the average percent inhibition in comparison to the DMSO control. The radioactivity values obtained for the wells that contained DMSO were taken as 100% uptake of the cells. The observed concentration - percent inhibition data were fitted to a sigmoidal concentration-effect model, where:

$$\% \text{ Inhibition} = (100 * \text{Conc}^\wedge\text{Slope}) / (\text{IC50}^\wedge\text{Slope} + \text{Conc}^\wedge\text{Slope})$$

$IC_{50}$ and slope estimates with their 95% confidence limits were determined by a nonlinear, least-squares regression analysis using the Data Analysis Toolbox™ (MDL Information Systems, San Leandro, CA, USA).

For assessment of activity of compounds as URAT1 inhibitors, the percent inhibition of uric acid uptake was typically assessed at a drug concentration of 10 micromolar (Table 1) and 100 micromolar (Table 2). Additional drug concentrations of some compounds were tested for determination of IC-50 values (Table 3). In this example the compounds were not necessarily tested simultaneously.

Table 1: Inhibitory Effects of Various Compounds (at 10 μM) on Uric Acid Uptake in Human URAT1 -Expressing HEK293 cells

| | % Of Inhibition | St Dev |
|---|---|---|
| Cpd. EJ | 43 | 6 |
| Cpd. EK | 84 | 10 |
| Cpd. EL | -17 | 6.1 |
| Cpd. EM | 94.1 | 0.30 |
| Cpd. EN | 95.5 | 0.32 |
| Cpd. EH | 95.86 | 0.11 |
| Cpd. EB | 90 | 0.29 |
| Benzbromarone | 94.6 | 0.07 |

Table 2: Inhibitory Effects of Various Compounds (at 100 μM) on Uric Acid Uptake in Human URAT1 -Expressing HEK293 cells

| | % Of Inhibition | St Dev |
|---|---|---|
| Cpd. EJ | 85 | 1 |
| Cpd. EK | 97 | 0.9 |
| Cpd. EL | -5 | 2.4 |
| Probenecid (125 μM) | 39.9 | 0.74 |

Table 3:

| | IC50 values (μM) |
|---|---|
| Cpd. EK | 2.154 |
| Cpd. EM | 0.6707 |
| Cpd. EN | 0.4119 |
| Cpd. EB | 0.93 |
| Cpd. EH | 0.08 |
| Benzbromarone | 0.75 |
| Probenecid | 173.51 |

**Claims**

**1.** A compound represented by Formula I

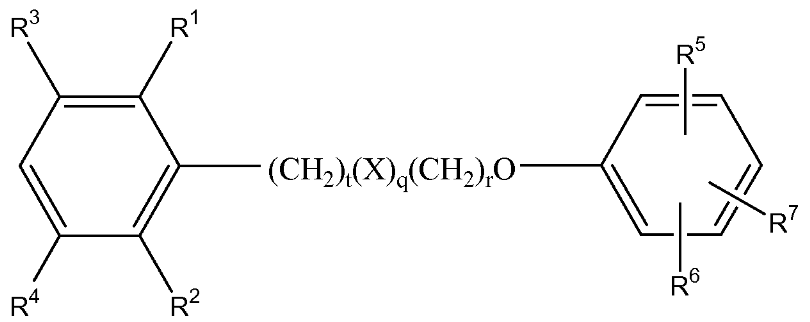

I

wherein

$R^1$ is methyl;
$R^2$ is methyl;
one of $R^3$ and $R^4$ is hydrogen, and the other is selected from the group consisting of hydroxy, alkoxy having 1 or 2 carbon atoms, and nitro;
t is 0;
q is 0;
r is 1;
$R^5$ is hydrogen or methyl;
$R^6$ is hydrogen
$R^7$ is

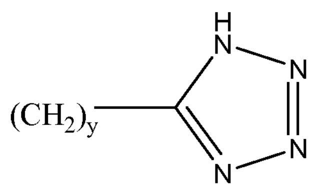

or

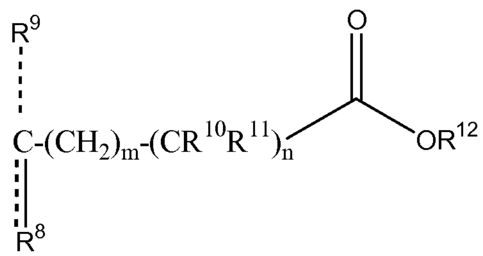

y is 0, 1, 2, or 3;
m is 0, 1, 2, 3, or 4;
n is 0;
$R^8$ is hydrogen and $R^9$ is hydrogen;
$R^{12}$ is hydrogen or alkyl having from 1 to 3 carbon atoms,
or when $R^{12}$ is hydrogen, a pharmaceutically acceptable salt of the compound.

**2.** The compound of claim 1, wherein $R^7$ is

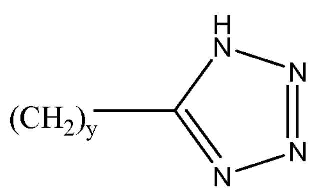

;

preferably wherein the compound is 3-((3-(1*H*-tetrazol-5-yl)phenoxy)methyl)-2,4-dimethylphenol.

**3.** The compound or salt of claim 1, wherein $R^7$ is

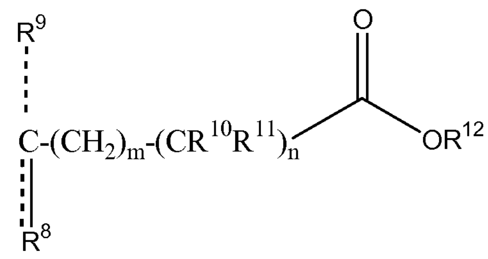

preferably wherein the compound is selected from the group consisting of:

2-(3-(3-hydroxy-2,6-dimethylbenzyloxy)-4-methylphenyl)acetic acid;
2-(3-(3-methoxy-2,6-dimethylbenzyloxy)-4-methylphenyl)acetic acid; and
2-(3-(2,6-dimethyl-3-nitrobenzyloxy)phenyl)acetic acid.

**4.** A pharmaceutical composition comprising a compound or salt according to any of claims 1 to 3 and a pharmaceutically acceptable carrier.

**5.** A compound or salt according to any of claims 1 to 3 or a pharmaceutical composition according to claim 4, for use in reducing the uric acid concentration in blood of, or increasing uric acid excretion from, a mammalian subject; preferably wherein the compound or salt is administered orally.

**6.** The compound or salt for use of claim 5, wherein the use results in treatment or prevention of a condition selected from the group consisting of gout, hyperuricemia, elevated levels of uric acid that do not meet the levels customarily justifying a diagnosis of hyperuricemia, renal dysfunction, kidney stones, cardiovascular disease, risk for developing cardiovascular disease, tumor-lysis syndrome, cognitive impairment, early-onset essential hypertension, and *Plasmodium falciparum*-induced inflammation.

**7.** The compound or salt for use of claim 5, wherein the compound or salt is administered in combination with one or more other uric acid lowering drugs in a combined amount effective to reduce the uric acid concentration in blood of, or increase uric acid excretion from, the subject;
preferably wherein the other uric acid lowering drug is selected from the group consisting of a xanthine oxidase inhibitor, a uricosuric agent, a urate transporter-1 inhibitor, a uricase, and a statin.

**8.** The compound or salt for use of claim 7 wherein the other uric acid lowering drug is administered in an amount that is less than the usual therapeutic dose when administered alone.

**9.** The compound or salt for use of claim 7, wherein the compound or salt and the one or more other uric acid lowering drugs are mixed together in the form of an admixture.

**10.** The compound or salt for use of claim 7, wherein the compound or salt and the one or more other uric acid lowering drugs are not mixed together in the form of an admixture.

**11.** Use of a compound or salt according to any one of claims 1 to 3 or a pharmaceutical composition according to claim 4 in the manufacture of a medicament for reducing the uric acid concentration in blood of, or increasing uric acid excretion from, a mammalian subject; preferably wherein the medicament is formulated for oral administration.

**12.** The use of claim 11, wherein the amount is selected so that administration of the medicament to a mammalian subject results in treatment or prevention of a condition selected from the group consisting of gout, hyperuricemia, elevated levels of uric acid that do not meet the levels customarily justifying a diagnosis of hyperuricemia, renal dysfunction, kidney stones, cardiovascular disease, risk for developing cardiovascular disease, tumor-lysis syndrome, cognitive impairment, early-onset essential hypertension, and *Plasmodium falciparum*-induced inflammation.

**13.** The use of claim 11, wherein the medicament is formulated for administration in combination with one or more other uric acid lowering drugs in a combined amount effective to reduce the uric acid concentration in blood of, or increase uric acid excretion from, the subject;
preferably wherein the other uric acid lowering drug is selected from the group consisting of a xanthine oxidase

inhibitor, a uricosuric agent, a urate transporter-1 inhibitor, a uricase, and a statin.

**14.** The use of claim 13, wherein the other uric acid lowering drug is administered in an amount that is less than the usual therapeutic dose when administered alone.

**Patentansprüche**

**1.** Verbindung gemäß Formel I:

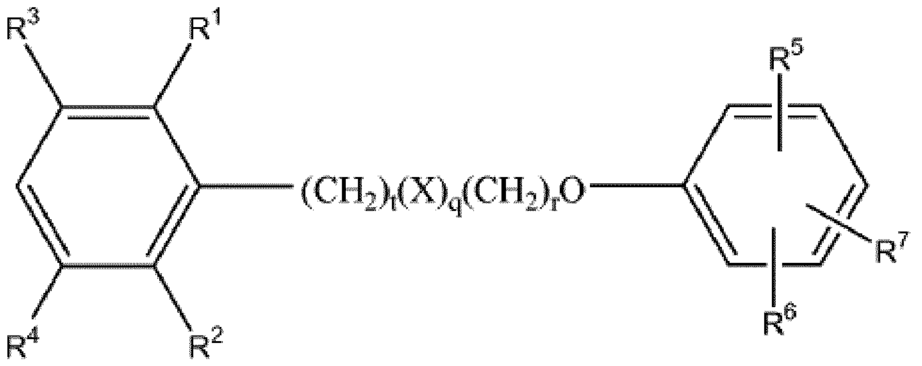

I

wobei,

$R^1$ Methyl ist;
$R^2$ Methyl ist;
eines von $R^3$ und $R^4$ Wasserstoff ist und das andere aus der Gruppe ausgewählt wird, die aus Folgendem besteht: Hydroxy, Alkoxy mit 1 oder 2 Kohlenstoffatomen und Nitro;
t 0 ist;
q 0 ist;
r 1 ist;
$R^5$ Wasserstoff oder Methyl ist;
$R^6$ Wasserstoff ist;
$R^7$ Folgendes ist:

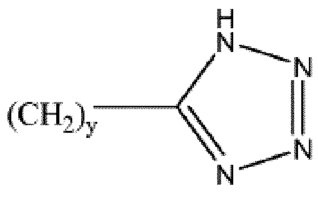

oder

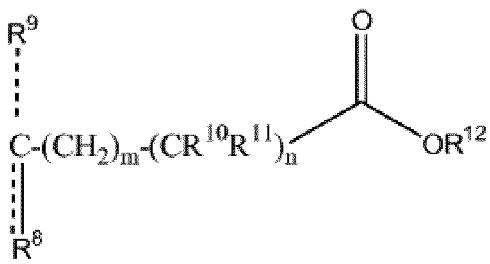

Y 0, 1, 2 oder 3 ist;
M 0, 1, 2, 3 oder 4 ist;
n 0 ist;
$R^8$ Wasserstoff ist und $R^9$ Wasserstoff ist;
$R^{12}$ Wasserstoff oder Alkyl mit 1 bis 3 Kohlenstoffatomen ist,

oder wenn $R^{12}$ Wasserstoff ist, ein pharmazeutisch verträgliches Salz der Verbindung.

**2.** Verbindung nach Anspruch 1, wobei $R^7$ Folgendes ist:

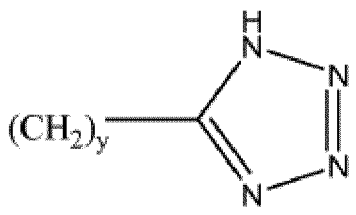

;

vorzugsweise wobei die Verbindung 3-((3-(1H-Tetrazol-5-yl)phenoxy)methyl)-2,4-dimethylphenol ist.

**3.** Verbindung oder Salz nach Anspruch 1, wobei $R^7$ Folgendes ist:

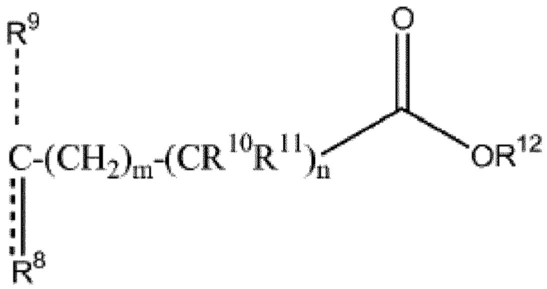

wobei die Verbindung vorzugsweise aus der Gruppe ausgewählt wird, die aus Folgendem besteht:

2-(3-(3-Hydroxy-2,6-dimethylbenzyloxy)-4-methylphenyl)essigsäure;
2-(3-(3-Methoxy-2,6-dimethylbenzyloxy)-4-methylphenyl)essigsäure; und
2-(3-(2,6-Dimethyl-3-nitrobenzyloxy)phenyl)essigsäure.

**4.** Pharmazeutische Zusammensetzung, die eine Verbindung oder ein Salz nach einem der Ansprüche 1 bis 3 und einen pharmazeutisch verträglichen Träger umfasst.

**5.** Verbindung oder Salz nach einem der Ansprüche 1 bis 3 oder pharmazeutische Zusammensetzung nach Anspruch 4, zur Verwendung bei der Verringerung der Harnsäurekonzentration im Blut oder der Erhöhung der Harnsäureausscheidung von einem Säugerpatienten;
wobei die Verbindung oder das Salz vorzugsweise oral verabreicht wird.

**6.** Verbindung oder Salz zur Verwendung nach Anspruch 5, wobei die Verwendung zur Behandlung oder Verhinderung einer Erkrankung führt, die aus der Gruppe ausgewählt wird, die aus Folgendem besteht: Gicht, Hyperurikämie, erhöhte Harnsäureniveaus, die nicht die Niveaus erfüllen, die normalerweise eine Diagnose von Hyperurikämie rechtfertigen, Nierenfunktionsstörungen, Nierensteine, Herzkreislauferkrankung, Risiko für die Entwicklung von Herz-Kreislauf-Erkrankungen, Tumor-Lyse-Syndrom, kognitive Beeinträchtigung, früh einsetzende essentielle Hypertonie und Plasmodium falciparum-induzierte Entzündung.

**7.** Verbindung oder Salz zur Verwendung nach Anspruch 5, wobei die Verbindung oder das Salz in Kombination mit einem oder mehreren anderen Harnsäure senkenden Arzneimitteln in einer kombinierten Menge verabreicht wird, um die Harnsäurekonzentration im Blut zu verringern oder die Harnsäureausscheidung aus dem Patienten zu erhöhen;
wobei das andere Harnsäure senkende Arzneimittel vorzugsweise aus der Gruppe ausgewählt wird, die aus Folgendem besteht: einem Xanthinoxidase-Inhibitor, einem urikosurischen Mittel, einem Urat-Transporter-1-Inhibitor, einer Urikase und einem Statin.

**8.** Verbindung oder Salz zur Verwendung nach Anspruch 7, wobei das andere Harnsäure senkende Arzneimittel in einer Menge verabreicht wird, die geringer ist als die übliche therapeutische Dosis, wenn sie allein verabreicht wird.

**9.** Verbindung oder Salz zur Verwendung nach Anspruch 7, wobei die Verbindung oder das Salz und das eine oder die mehreren anderen Harnsäure senkenden Arzneimittel in Form einer Beimischung miteinander vermischt werden.

**10.** Verbindung oder Salz zur Verwendung nach Anspruch 7, wobei die Verbindung oder das Salz und das eine oder die mehreren anderen Harnsäure senkenden Arzneimittel nicht in Form einer Beimischung miteinander vermischt werden.

**11.** Verwendung einer Verbindung oder eines Salzes nach einem der Ansprüche 1 bis 3 oder einer pharmazeutischen Zusammensetzung nach Anspruch 4 bei der Herstellung eines Medikaments zur Verringerung der Harnsäurekonzentration im Blut oder der Erhöhung der Harnsäureausscheidung von einem Säugerpatienten; wobei das Medikament vorzugsweise zur oralen Verabreichung formuliert ist.

**12.** Verwendung nach Anspruch 11, wobei die Menge so ausgewählt wird, dass die Verabreichung des Medikaments an einen Säugerpatienten zu einer Behandlung oder Verhinderung einer Erkrankung führt, die aus der Gruppe ausgewählt wird, die aus Folgendem besteht: Gicht, Hyperurikämie, erhöhte Harnsäureniveaus, die nicht die Niveaus erfüllen, die normalerweise eine Diagnose von Hyperurikämie rechtfertigen, Nierenfunktionsstörungen, Nierensteine, Herzkreislauferkrankung, Risiko für die Entwicklung von Herz-Kreislauf-Erkrankungen, Tumor-Lyse-Syndrom, kognitive Beeinträchtigung, früh einsetzende essentielle Hypertonie und Plasmodium falciparum-induzierte Entzündung.

**13.** Verwendung nach Anspruch 11, wobei das Medikament zur Verabreichung in Kombination mit einem oder mehreren anderen Harnsäure senkenden Arzneimitteln in einer kombinierten Menge formuliert ist, um die Harnsäurekonzentration im Blut zu verringern oder die Harnsäureausscheidung aus dem Patienten zu erhöhen;
wobei das andere Harnsäure senkende Arzneimittel vorzugsweise aus der Gruppe ausgewählt wird, die aus Folgendem besteht: einem Xanthinoxidase-Inhibitor, einem urikosurischen Mittel, einem Urat-Transporter-1-Inhibitor, einer Urikase und einem Statin.

**14.** Verwendung nach Anspruch 13, wobei das andere Harnsäure senkende Arzneimittel in einer Menge verabreicht wird, die geringer ist als die übliche therapeutische Dosis, wenn sie allein verabreicht wird.

**Revendications**

**1.** Composé représenté par la formule I

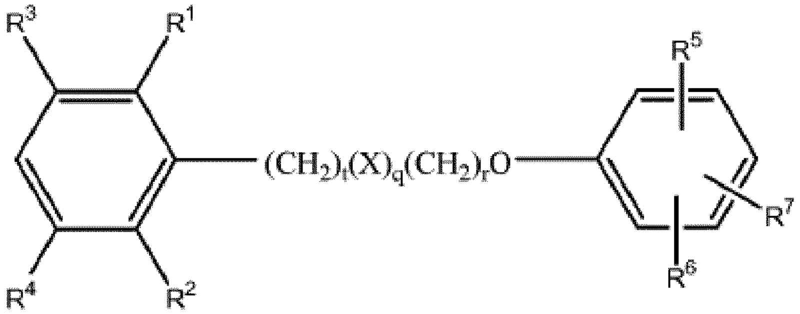

I

dans laquelle

$R^1$ est un groupe méthyle ;
$R^2$ est un groupe méthyle ;
l'un de $R^3$ et de $R^4$ est un atome d'hydrogène, et l'autre est sélectionné dans le groupe constitué de groupes hydroxy, alcoxy comprenant 1 ou 2 atomes de carbone, et nitro ;
t vaut 0 ;
q vaut 0 ;
r vaut 1 ;
$R^5$ est un atome d'hydrogène ou un groupe méthyle ;
$R^6$ est un atome d'hydrogène ;
$R^7$ est

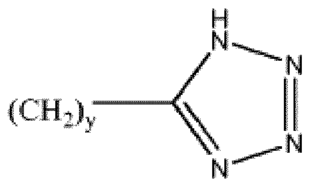

ou

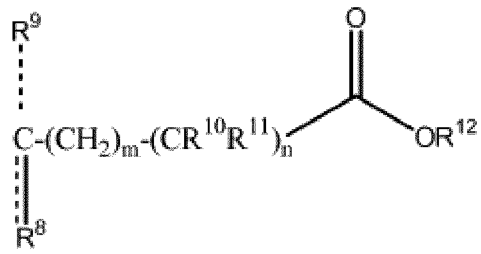

y vaut 0, 1, 2, ou 3 ;
m vaut 0, 1, 2, 3, ou 4 ;
n vaut 0 ;
$R^8$ est un atome d'hydrogène et $R^9$ est un atome d'hydrogène ;
$R^{12}$ est un atome d'hydrogène ou un groupe alkyle comprenant de 1 à 3 atomes de carbone,
ou quand $R^{12}$ est un atome d'hydrogène, un sel pharmaceutiquement acceptable du composé.

**2.** Composé selon la revendication 1, dans lequel $R^7$ est

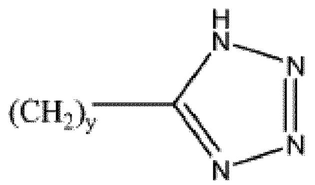

;

préférablement dans lequel le composé est le 3-((3-(1*H*-tétrazol-5-yl)phénoxy)méthyl)-2,4-diméthylphénol.

**3.** Composé ou sel selon la revendication 1, dans lequel $R^7$ est

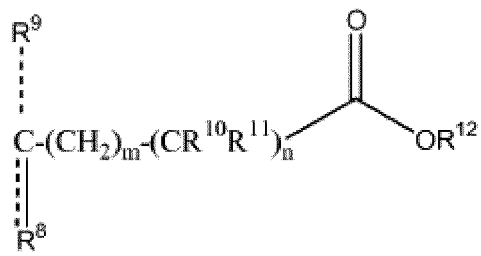

préférablement dans lequel le composé est sélectionné dans le groupe constitué de : l'acide 2-(3-(3-hydroxy-2,6-diméthylbenzyloxy)-4-méthylphényl)acétique ;
l'acide 2-(3-(3-méthoxy-2,6-diméthylbenzyloxy)-4-méthylphényl)acétique ; et
l'acide 2-(3-(2,6-diméthyl-3-nitrobenzyloxy)phényl)acétique.

**4.** Composition pharmaceutique comprenant un composé ou un sel selon l'une quelconque des revendications 1 à 3 et un support pharmaceutiquement acceptable.

**5.** Composé ou sel selon l'une quelconque des revendications 1 à 3 ou composition pharmaceutique selon la revendication 4, destiné(e) à une utilisation dans la réduction de la concentration en acide urique dans le sang de, ou dans l'augmentation de l'excrétion d'acide urique à partir de, un sujet mammifère ;
le composé ou le sel étant préférablement administré par voie orale.

**6.** Composé ou sel destiné à une utilisation selon la revendication 5, l'utilisation résultant en un traitement ou en une prévention d'une affection sélectionnée dans le groupe constitué de la goutte, de l'hyperuricémie, de niveaux élevés

d'acide urique qui n'atteignent pas les niveaux justifiant normalement un diagnostic d'hyperuricémie, d'une dysfonction rénale, de calculs rénaux, d'une maladie cardiovasculaire, d'un risque de développer une maladie cardiovasculaire, du syndrome de lyse tumorale, d'un trouble cognitif, de l'hypertension essentielle précoce, et d'une inflammation causée par *Plasmodium falciparum.*

**7.** Composé ou sel destiné à une utilisation selon la revendication 5, le composé ou le sel étant administré en combinaison avec un ou plusieurs autres médicaments abaissant la concentration en acide urique dans une quantité combinée efficace pour réduire la concentration en acide urique dans le sang, ou pour augmenter l'excrétion d'acide urique à partir, du sujet ;
l'autre médicament abaissant la concentration en acide urique étant préférablement sélectionné dans le groupe constitué d'un inhibiteur de la xanthine oxydase, d'un agent uricosurique, d'un inhibiteur du transporteur URAT-1 (urate transporter-1), d'une uricase, et d'une statine.

**8.** Composé ou sel destiné à une utilisation selon la revendication 7, dans lequel l'autre médicament abaissant la concentration en acide urique est administré dans une quantité qui est inférieure à la dose thérapeutique habituelle quand il est administré seul.

**9.** Composé ou sel destiné à une utilisation selon la revendication 7, le composé ou le sel et lesdits un ou plusieurs autres médicaments abaissant la concentration en acide urique étant mélangés les uns avec les autres sous la forme d'une admixtion.

**10.** Composé ou sel destiné à une utilisation selon la revendication 7, le composé ou le sel et lesdits un ou plusieurs autres médicaments abaissant la concentration en acide urique n'étant pas mélangés les uns avec les autres sous la forme d'une admixtion.

**11.** Utilisation d'un composé ou d'un sel selon l'une quelconque des revendications 1 à 3 ou d'une composition pharmaceutique selon la revendication 4 dans la fabrication d'un médicament pour la réduction de la concentration en acide urique dans le sang de, ou l'augmentation de l'excrétion d'acide urique à partir de, un sujet mammifère ; préférablement dans laquelle le médicament est formulé pour une administration par voie orale.

**12.** Utilisation selon la revendication 11, dans laquelle la quantité est sélectionnée de telle sorte que l'administration du médicament à un sujet mammifère résulte en un traitement ou en une prévention d'une affection sélectionnée dans le groupe constitué de la goutte, de l'hyperuricémie, de niveaux élevés d'acide urique qui n'atteignent pas les niveaux justifiant normalement un diagnostic d'hyperuricémie, d'une dysfonction rénale, de calculs rénaux, d'une maladie cardiovasculaire, d'un risque de développer une maladie cardiovasculaire, du syndrome de lyse tumorale, d'un trouble cognitif, de l'hypertension essentielle précoce, et d'une inflammation causée par *Plasmodium falciparum.*

**13.** Utilisation selon la revendication 11, dans laquelle le médicament est formulé pour une administration en combinaison avec un ou plusieurs autres médicaments abaissant la concentration en acide urique dans une quantité combinée efficace pour réduire la concentration en acide urique dans le sang, ou pour augmenter l'excrétion d'acide urique à partir, du sujet ;
l'autre médicament abaissant la concentration en acide urique étant préférablement sélectionné dans le groupe constitué d'un inhibiteur de la xanthine oxydase, d'un agent uricosurique, d'un inhibiteur du transporteur URAT-1 (urate transporter-1), d'une uricase, et d'une statine.

**14.** Utilisation selon la revendication 13, dans laquelle l'autre médicament abaissant la concentration en acide urique est administré dans une quantité qui est inférieure à la dose thérapeutique habituelle quand il est administré seul.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 200937128 W
- US 200942298 W
- EP 0295890 A
- WO 9916747 A
- JP 04154773 B
- JP 47039101 B
- WO 9628423 A
- WO 2001002388 A
- WO 9405153 A
- US 5519133 A
- WO 20022018323 A
- JP 06293700 B
- WO 2002000633 A
- WO 2002044145 A
- WO 9745400 A
- US 4932999 A
- WO 9912928 A
- US 2001034343 A
- WO 9725992 A
- US 6194406 B
- WO 9626176 A
- JP 07070025 B
- JP 07206658 B
- DE 2749518
- JP 08119959 B
- WO 9619437 A
- WO 8503701 A
- JP 10087489 B
- WO 9504046 A
- JP 04282345 B
- EP 279630 A
- WO 9509843 A


### Non-patent literature cited in the description


- *Chirality,* 1999, vol. 11, 420-425
- **T. GREENE.** *the Protective Groups in Organic Synthesis*
- **T. GREENE.** Protective Groups in Organic Synthesis
- **T. GREENE.** *Protective Groups in Organic Synthesis*
- *Tetrahedron: Asymmetry,* 1997, vol. 8 (7), 1083-1099
- **HARADA, T. ; IZUMI, Y.** *Chem. Lett.,* 1978, 1195-1196
- **AKUTAGAWA, S. ; KITAMURA, M. ; KUMOBAYASHI, H. ; NOYORI, R. ; OHKUMA, T. ; SAYO, N. ; TAKAYA, M.** *J. Am. Chem. Soc.,* 1987, vol. 109, 5856-5858
- *J.O.C.,* 2001, vol. 66, 7907-7909
- **GEORGE M RUBOTTOM et al.** *J. Org. Chem.,* 1983, vol. 48, 1550-1552
- *Canadian Journal of Chemistry,* 2001, vol. 79 (11), 1541-1545
- *Journal of labeled Compounds and Radiopharmaceuticals,* 1992, vol. 31 (3), 175-82
- *Proceedings of the Indiana Academy of Science (1983),* 1982, vol. 92, 145-51
- *Z. Chem.,* 1976, vol. 16 (8), 319-320
- *Journal of Chemical Research, Synopses,* 1994, vol. 11, 405
- *J. Med. Chem.,* 1973, vol. 16 (6), 684-7
- *Collection of Czechoslovak Chemical Communications,* 1991, vol. 56 (2), 459-77
- *J.O.C.,* 1990, vol. 55 (18), 5287-91
- *J.O.C,* 2001, vol. 66 (23), 7883-88
- *Journal of the American Chemical Society,* 1985, vol. 107 (8), 2571-3
- *Taiwan Kexue,* 1996, vol. 49 (1), 51-56
- *Takeda Kenkyusho Nempo,* 1965, vol. 24, 221-8
- *Farmacia (Bucharest),* 1970, vol. 18 (8), 461-6
- *Adapt synthesis from Takeda Kenkyusho Nempo,* 1965, vol. 24, 221-8
- *Biomedical Mass Spectrometry,* 1985, vol. 12 (4), 163-9
- *Adapt synthesis from Taiwan Kexue,* 1996, vol. 49 (1), 51-56
- *J.O.C.,* 2001, vol. 66, 7883-88
- *J. Med. Chem.,* 1971, vol. 14 (3), 265
- *Yaoxue Xuebao,* 1998, vol. 33 (1), 67-71
- *J.A.C.S.,* 1974, vol. 96 (7), 2121-9
- *J.O.C.,* 1991, vol. 56 (14), 4525-29
- *J. Med. Chem.,* 1981, vol. 24 (10), 1245-49
- *J.A.C.S,* 1974, vol. 96 (7), 2121-9
- *Polymer,* 1991, vol. 32 (11), 2096-105
- *J.O.C,* 2001, vol. 66, 7883-88
- **SCHRETLEN, D.J. et al.** Serum Uric Acid and Cognitive Function in Community-Dwelling Older Adults. *Neuropsychology,* January 2007, vol. 21 (1), 136-140